# EUROPEAN PATENT APPLICATION

(11) **EP 4 674 860 A1**
(43) Date of publication of application: **07.01.2026**
(21) Application number: 24763933.9
(22) Date of filing: 27.02.2024
(51) Int. Cl.: C07K 5/00, C07K 1/13, C07K 7/00, C07K 14/00

(54) **COMPOUND AND LABELED BIOMATERIAL USING SAME**

(30) Priority: 27.02.2023 JP 2023028797
(71) Applicant: FUJIFILM Corporation, Tokyo 106-8620 (JP)
(72) Inventor: SHIROKANE, Kenji, Ashigarakami-gun, Kanagawa 258-8577 (JP); YOSHIMITSU, Yuji, Ashigarakami-gun, Kanagawa 258-8577 (JP); KANAZAWA, Yoshinori, Ashigarakami-gun, Kanagawa 258-8577 (JP)
(74) Representative: HGF
(86) International application number: PCT/JP2024/007131
(87) International publication number: WO 2024/181454

(57) **Abstract**

A compound containing two or more phosphor moieties, in which each of the phosphor moieties adjacent to each other is linked by a linking group having a Molecular Flexibility of 0.510 or less and a cLogP of 6.0 or less, and a labeled biological substance using the compound.

## Description

### BACKGROUND OF THE INVENTION

### 1. Field of the Invention

The present invention relates to a compound and a labeled biological substance using the compound.

### 2. Description of the Related Art

To observe in vivo changes in response to various stimuli (diseases, environmental changes, and the like), fluorescently labeled biological substances obtained by labeling a biological molecule (an antibody or the like) having a binding property to a target substance to be detected, with a fluorescent compound (a fluorescent dye), are often used.

For example, also in Western blotting (hereinafter, also abbreviated as WB) that detects a specific protein from a protein mixture, a fluorescence method in which the presence or absence or the abundance of the specific protein is detected using a fluorescently labeled antibody having a binding property to this protein is used.

In addition, in bioimaging technology for analyzing the dynamics and functions of biological molecules, cells, tissues, and the like in a living body, in vivo fluorescence imaging in which a specific portion of a living body visualized by fluorescence labeling is observed is used as one of the techniques for the living body observation.

In the above-described fluorescence labeling, an organic fluorescent dye molecule is generally used, and the brightness (fluorescence intensity) is increased generally by using a fluorescently labeled biological substance to which a plurality of fluorescent dye molecules are bonded. However, since most of the organic dyes exhibiting fluorescence, such as a cyanine dye and a rhodamine dye, have an aromatic chromophore having high planarity, an interaction between the dyes easily occurs, and as a result, a decrease in the fluorescence intensity after labeling due to an interaction such as self-association between the dyes easily occurs. In addition, as the number of fluorescent dye molecules (degree of fluorescence labeling: DOL) per one molecule of the biological molecule increases, the fluorescence intensity due to self-association or the like tends to further decrease.

On the other hand, in a field different from the fluorescence labeling, a coloring agent compound that utilizes a fluorescence resonance energy transfer (FRET) phenomenon is known. As a coloring agent compound that utilizes such a FRET phenomenon, for example, a compound in which a phosphor moiety I (an energy donor) that is excited with excitation light is linked, by a group containing peptide such as polyproline, to another phosphor moiety II (an energy acceptor) that receives energy from the phosphor moiety I and emits light or is quenched is described as described in Nisha Geng, "Synthesis of Polyproline Spacers between NIR Dye Pairs for FRET to Enhance Photoacoustic Imaging", Theses, RIT Scholar Works, July 2017, WO2010/117420A, JP2003-508080A, and WO99/002544A. In addition, as described in WO2017/105927A, a compound in which a phosphor moiety I (an energy donor) that is excited with excitation light is linked, by a fluorene linker, to another phosphor moiety II (an energy acceptor) that receives energy from the phosphor portion I and emits light or is quenched is described.

### SUMMARY OF THE INVENTION

As a coloring agent used for fluorescence labeling, a dye multimer having a plurality of fluorescent dyes in one molecule is known. Even in the dye multimer, the association of the fluorescent dye occurs easily both intramolecularly and intermolecularly, the decrease in fluorescence intensity occurs easily, and as the number of fluorescent dyes present in one molecule of the dye multimer increases, the association of the fluorescent dyes occurs more easily, and the fluorescence intensity tends to decrease further. As a result of the studies by the present inventors, it has been found that the linking group which links the phosphor moiety I and the phosphor moiety II specifically described in Nisha Geng, "Synthesis of Polyproline Spacers between NIR Dye Pairs for FRET to Enhance Photoacoustic Imaging", Theses, RIT Scholar Works, July 2017, WO2010/117420A, JP2003-508080A, WO99/002544A, and WO2017/105927A, has an insufficient effect in suppressing the association of the phosphor moieties.

An object of the present invention is to provide a compound excellent in suppressing association in a solution state. In addition, another object of the present invention is to provide a labeled biological substance obtained by bonding the compound to a biological substance.

That is, the above objects of the present invention have been achieved by the following means.
[1] A compound comprising: two or more phosphor moieties, in which phosphor moieties adjacent to each other are linked by a linking group having a Molecular Flexibility of 0.510 or less and a cLogP of 6.0 or less.
[2] The compound according to [1], in which the linking group is a group containing at least one of an alkynyl group, an aliphatic hydrocarbon ring, a heterocyclic ring, or a carbonyl group.
[3] The compound according to [1] or [2], in which the linking group is a group containing a structure represented by General Formula (I), 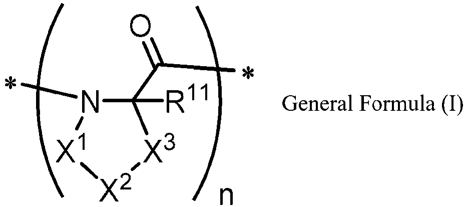 in the formula, X¹ to X³ represent -O-, -S-, >NR¹, or >CR²R³,
   R¹ to R³ and R¹¹ represent a hydrogen atom, an alkyl group, an alkenyl group, an alkynyl group, an aryl group, a heteroaryl group, an acyl group, -NR⁸R⁹, -OR¹⁰, an anionic group, a betaine residue, a polyol residue, a sugar residue, a polyamino acid residue, -(O-L)_{g}R^{E}, or -(L-O)_{g}R^{E},
   R⁸ to R¹⁰ represent a hydrogen atom, an alkyl group, an alkenyl group, an alkynyl group, an acyl group, an aryl group, a heteroaryl group, an anionic group, a betaine residue, a polyol residue, a sugar residue, a polyamino acid residue, -(O-L)_{g}R^{E}, or -(L-O)_{g}R^{E},
   L represents an alkylene group, R^{E} has the same meaning as R⁸ to R¹⁰ described above, and g represents 2 to 50, provided that R^{E} represents neither -(O-L)_{g}R^{E} nor -(L-O)_{g}R^{E},
   n represents an integer of 2 or more, and
   * represents a bonding site.
[4] The compound according to[3], in which n is an integer of 3 or more.
[5] The compound according to [3] or [4], in which the compound is represented by General Formula (II), 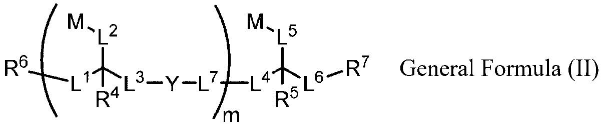
   in the formula, R⁴ and R⁵ represent a hydrogen atom, an alkyl group, an alkenyl group, an alkynyl group, an acyl group, an amino group, a hydroxy group, an alkoxy group, a sulfanyl group, an aryl group, or a heteroaryl group,
   R⁶ and R⁷ represent a hydrogen atom, a hydroxy group, a sulfanyl group, an alkyl group, an alkenyl group, an alkynyl group, an aryl group, an alkoxy group, an amino group, an acyl group, a heteroaryl group, or Q,
   Q represents an anionic group, a cationic group, a betaine residue, a polyol residue, a sugar residue, a polyamino acid residue, -(O-L)_{g}R^{E}, -(L-O)_{g}R^{E}, a substituent allowing binding to a biological substance, or a substituent allowing binding to a solid support,
   L¹ to L⁷ represent a single bond or a divalent linking group,
   M represents a phosphor moiety, a physiologically active substance moiety, a prodrug moiety, or a radioactive isotope-containing moiety,
   Y represents the structure represented by General Formula (I),
   m is an integer of 1 or more, and
   L, R^{E}, and g have the same meanings as L, R^{E}, and g, all of which are described above,
   provided that at least two of M's represent the phosphor moieties described above.
[6] The compound according to [5], in which the compound is represented by General Formula (III), 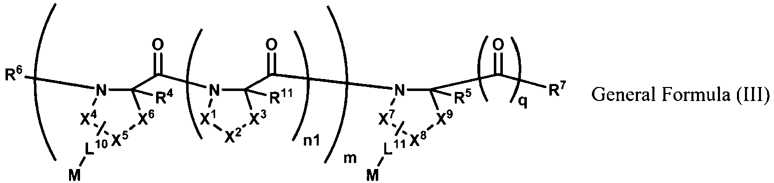 in the formula, X⁴ to X⁹ represent -O-, -S-, >NR¹⁰¹, or >CR¹⁰²R¹⁰³,
   provided that at least one of X⁴, X⁵, or X⁶ represents >N-L¹⁰-M or >C(R¹⁰²)-L¹⁰-M, and at least one of X⁷, X⁸, or X⁹ is >N-L¹¹-M or >C(R¹⁰²)-L¹¹-M,
   R¹⁰¹ to R¹⁰³ which are neither -L¹⁰-M nor -L¹¹-M represent a hydrogen atom, an alkyl group, an alkenyl group, an alkynyl group, an aryl group, a heteroaryl group, an acyl group, -NR⁸R⁹, -OR¹⁰, an anionic group, a betaine residue, a polyol residue, a sugar residue, a polyamino acid residue, -(O-L)_{g}R^{E}, or -(L-O)_{g}R^{E},
   L¹⁰ and L¹¹ represent a single bond or a divalent linking group,
   n1 represents an integer of 2 or more, and q represents an integer of 0 or 1, and
   R⁴ to R¹¹, X¹ to X³, M, L, R^{E}, g, and m have the same meanings as R⁴ to R¹¹, X¹ to X³, M, L, R^{E}, g, and m, all of which are described above.
[7] The compound according to [6], in which the compound is represented by General Formula (IV), 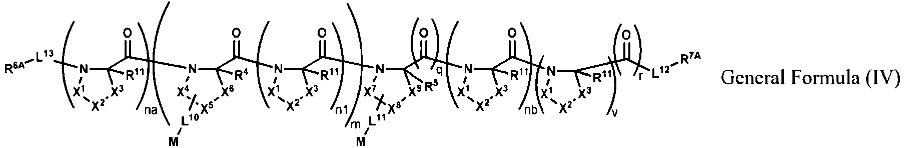
   in the formula, R^{6A} and R^{7A} represent a hydrogen atom, a hydroxy group, a sulfanyl group, an alkyl group, an alkenyl group, an alkynyl group, an aryl group, an alkoxy group, a heteroaryl group, an amino group, an acyl group, or Q, provided that at least one of R^{6A} or R^{7A} represents a carboxy group, a substituent allowing binding to a biological substance, or a substituent allowing binding to a solid support,
   L¹² and L¹³ represent a single bond or a linking group,
   na and nb represent an integer of 0 or more, and r and v represent an integer of 0 or 1, and
   R⁴, R⁵, R¹¹, L¹⁰, L¹¹, X¹ to X⁹, M, Q, q, n1, and m have the same meanings as R⁴, R⁵, R¹¹, L¹⁰, L¹¹, X¹ to X⁹, M, Q, q, nl, and m, all of which are described above.
[8] The compound according to [7], in which (A) na represents an integer of 1 or more, and R^{6A} represents the carboxy group, the substituent allowing binding to a biological substance, or the substituent allowing binding to a solid support, and/or (B) a sum of nb and v, both of which are described above, represents an integer of 1 or more, and R^{7A} represents the carboxy group, the substituent allowing binding to a biological substance, or the substituent allowing binding to a solid support,
   provided that the number of shortest linking atoms of L¹³ is 7 or less in a case of the (A), and the number of shortest linking atoms of L¹² is 7 or less in a case of the (B).
[9] The compound according to any one of [3] to [8], in which the structure represented by General Formula (I) includes a structure in which X¹ to X³ are >CR²R³.
[10] The compound according to [9], in which at least one R² in the structure in which X¹ to X³ are >CR²R³ included in the structure represented by General Formula (I) is -NR⁸R⁹, -OR¹⁰, an anionic group, a betaine residue, a polyol residue, a sugar residue, a polyamino acid residue, -(O-L)_{g}R^{E}, or -(L-O)_{g}R^{E}.
[11] The compound according to any one of [1] to [10], in which at least two of the phosphor moieties are phosphor moieties having light absorption characteristics that are equivalent to each other.
[12] The compound according to any one of [1] to [10], in which at least two of the phosphor moieties are phosphor moieties having light absorption characteristics that are non-equivalent to each other.
[13] The compound according to any one of [6] to [8], in which m in General Formula (III) is an integer of 3 or less.
[14] A labeled biological substance that is obtained by bonding the compound according to any one of [1] to [13] to a biological substance.
[15] The labeled biological substance according to [14], in which the biological substance is any one of a protein, an amino acid, a nucleic acid, a nucleotide, a sugar chain, or a phospholipid.

The compound according to the embodiment of the present invention is excellent in suppressing association in a solution state. In addition, the labeled biological substance according to the embodiment of the present invention is obtained from a compound being excellent in suppressing association in a solution state.

### DESCRIPTION OF THE PREFERRED EMBODIMENTS

In the present invention, in a case where there is a plurality of substituents, linking groups, structural units, or the like (hereinafter, referred to as substituents or the like), which are represented by a specific symbol or Formula, or in a case where a plurality of substituents or the like are regulated at the same time, the substituents or the like may be the same or different from each other, unless otherwise specified. The same applies to the regulation of the number of substituents or the like. In addition, in a case where a plurality of substituents or the like come close to each other (particularly in a case where they are adjacent to each other), they may be linked to each other to form a ring, unless otherwise specified. In addition, unless otherwise specified, rings such as an alicyclic ring, an aromatic ring, and a heterocyclic ring may be fused to form a fused ring.

For example, in the present invention, a structure represented by General Formula (I) described below means that n (n is an integer of 2 or more) pieces of structures represented by General Formula (i) are connected. In this case, the n pieces of structures represented by General Formulae (i) may be the same or different from each other. It is noted that X¹ to X³, and R¹¹ in General Formula (i) have the same meanings as X¹ to X³, and R¹¹ in General Formula (I) described later. The same applies to a structure parenthesized in ( )ₘ, a structure parenthesized in ( )ₙ₁, a structure parenthesized in ( )ₙₐ, and a structure parenthesized in ( )_{nb}, where m pieces of structures may be the same or different from each other, n1 pieces of structures may be the same or different from each other, na pieces of structures may be the same or different from each other, and nb pieces of structures may be the same or different from each other.

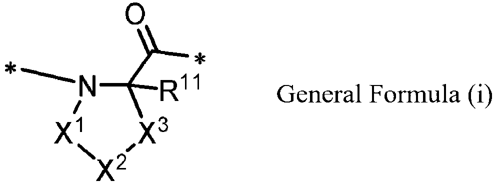

In the present invention, in a case where an E type double bond and a Z type double bond are present in a molecule, the double bond may be any one thereof or may be a mixture thereof, unless otherwise specified. In addition, unless otherwise specified, in a case where a chiral carbon atom or a chiral center is present in a compound, the steric conformation may be any of R or S in the R-S notation, and a mixture thereof may be may be allowed.

In the present invention, the denotation of a compound or substituent is meant to include not only the compound itself but also a salt thereof, and an ion thereof. For example, the dissociable anionic group such as the carboxy group, the sulfo group, and the phosphono group (-P(=O)(OH)₂) may have an ionic structure by a hydrogen ion being dissociated therefrom, or they may have a salt structure. That is, in the present invention, the "carboxy group" is meant to include a group of an ion or salt of a carboxylic acid, the "sulfo group" is meant to include a group of an ion or salt of a sulfonic acid, and the "phosphono group" is meant to include a group of an ion or salt of a phosphonic acid. The monovalent or polyvalent cation in forming the salt structure is not particularly limited. Examples thereof include an inorganic cation and an organic cation, and specific examples thereof include alkali metal cations such as Na⁺, Li⁺, and K⁺, alkaline earth metal cations such as Mg²⁺, Ca²⁺, and Ba²⁺, organic ammonium cations such as a trialkylammonium cation and a tetraalkylammonium cation, and an organic phosphonium cation such as a quaternary phosphonium ion.

In a case of a salt structure, the type of the salt may be one type, two or more types thereof may be mixed, a salt-type group and a group having a free acid structure may be mixed in a compound, and a compound having a salt structure and a compound having a free acid structure compound may be mixed.

Any compound according to the embodiment of the present invention is a neutral compound. In the present invention, the fact that the compound is neutral means that the compound is electrically neutral. Specifically, the charge of the compound as a whole is adjusted to be 0 by a group having a charge or by a counterion in the compound. For example, in the cyanine dye represented by General Formula (α), which is exemplified as an example of the coloring agent that constitutes the phosphor moiety, the formal charge of the nitrogen atom to which R⁴² is bonded is +1, and the dissociable group such as a sulfo group in another structure of the cyanine dye or the compound according to the embodiment of the present invention has an ionic structure such as a sulfonate ion to be paired with this formal charge, whereby the compound according to the embodiment of the present invention is to be a compound having a charge of 0 as a whole.

In each general formula pertaining to the cyanine dye, which is defined in the present invention, the positive charge possessed by the compound is specified and indicated, for convenience, as a structure of a specific nitrogen atom. Provided that since the cyanine dye defined in the present invention has a conjugated system, another atom other than the nitrogen atom actually may be capable of being positively charged, and thus any cyanine dye capable of adopting a structure represented by each general formula as one of the chemical structures is included in the cyanine dye represented by each general formula. This also applies to the negative charge.

In addition, it is meant to include those in which a part of the structure is changed within the scope that does not impair the effect of the present invention. Furthermore, it is meant that a compound, which is not specified to be substituted or unsubstituted, may have any substituent within the scope that does not impair the effect of the present invention. The same applies to a substituent (for example, a group represented by "alkyl group", "methyl group", "methyl") and a linking group (for example, a group represented by "alkylene group", "methylene group", "methylene"). Among such substituents, a preferred substituent in the present invention is a substituent selected from a substituent group T described later.

In the present invention, in a case where the number of carbon atoms of a certain group is specified, this number of carbon atoms means the number of carbon atoms of the entire group thereof unless otherwise specified in the present invention or the present specification. That is, in a case where this group has a form that further has a substituent, it means the total number of carbon atoms, to which the number of carbon atoms of this substituent is included.

In addition, in the present invention, the numerical range represented by using "to" means a range including the numerical values before and after "to" as the lower limit value and the upper limit value.

The compound according to the embodiment of the present invention is a compound containing two or more phosphor moieties, in which phosphor moieties adjacent to each other are linked by a linking group having a Molecular Flexibility of 0.510 or less and a cLogP of 6.0 or less. Although the details of the reason why the compound according to the embodiment of the present invention is excellent in suppressing association in a solution state are not clear, it is conceived as follows.

The compound according to the embodiment of the present invention is a compound containing two or more phosphor moieties, in which phosphor moieties adjacent to each other are linked by a linking group having rigidity, which is defined by a rigidity parameter of a Molecular Flexibility of 0.510 or less and hydrophilicity, which is defined by a hydrophilicity parameter of a cLogP of 6.0 or less. As described above, because phosphor moieties adjacent to each other are linked by a rigid linking group having a Molecular Flexibility of 0.510 or less, the mobility of the linking group is reduced and the intramolecular dye association can be suppressed, and because the linking group is a hydrophilic linking group having a cLogP of 6.0 or less, the intermolecular dye association can be suppressed by reducing intermolecular interaction in solution. As a result, it is possible to effectively suppress the intermolecular or intramolecular association of the phosphor moieties in a solution state of the compound (solution state in which the compound is dissolved in an aqueous medium). Therefore, it is considered that the compound according to the embodiment of the present invention can sufficiently suppress quenching due to association, and the labeled biological substance obtained by using the compound according to the embodiment of the present invention exhibits an excellent fluorescence intensity.

In consideration of the linking group that links the phosphor moiety I and the phosphor moiety II described in Nisha Geng, "Synthesis of Polyproline Spacers between NIR Dye Pairs for FRET to Enhance Photoacoustic Imaging", Theses, RIT Scholar Works, July 2017, WO2010/117420A, JP2003-508080A, WO99/002544A, and WO2017/105927A, the following linking groups are described as the linking group having the smallest Molecular Flexibility (abbreviated as MF in this paragraph). A linking group having an MF of 0.555 and a cLogP of -3.14 in the compound 30 of Nisha Geng, "Synthesis of Polyproline Spacers between NIR Dye Pairs for FRET to Enhance Photoacoustic Imaging", Theses, RIT Scholar Works, July 2017, a linking group having an MF of 0.551 and a cLogP of -1.82 in the compound 25 of Fig. 10 of WO2010/117420A, a linking group having an MF of 0.620 and a cLogP of -2.13 in the compound described in the second line of Table 5 of JP2003-508080A, a linking group having an MF of 0.512 and a cLogP of -3.09 in the compound 10 described on page 46 of WO99/002544A, and a linking group having an MF of 0.268 and a cLogP of 21.65 in the compound of Example 1 of WO2017/105927A.

However, in all of the linking groups described in Nisha Geng, "Synthesis of Polyproline Spacers between NIR Dye Pairs for FRET to Enhance Photoacoustic Imaging", Theses, RIT Scholar Works, July 2017, WO2010/117420A, JP2003-508080A, and WO99/002544A, MF is more than 0.510, and in the linking group described in WO2017/105927A, cLogP is more than 6.0. As shown in Examples described later, in a case where the phosphor moieties adjacent to each other are linked by a linking group having an MF of more than 0.510 or a linking group having a cLogP of more than 6.0, the suppression of the intermolecular or intramolecular association of the phosphor moieties is inferior.

In addition, Fig. 4 of JP2004-508838A also describes a compound in which a phosphor moiety I (energy donor), which is excited with excitation light, and another phosphor moiety II (energy acceptor), which receives energy from the phosphor moiety I and emits or quenches light, are linked by a proline linker. However, specific numerical values for the repetition numbers m and n of the proline unit constituting the proline linker are not described in the specification, and in paragraph [0148] of the specification, it is merely described that a library of CFET-dUTP having a unique fluorescence signal can be developed by changing the number of proline spacers between Fam and Tam and between Tam and Cy5. As described above, JP2004-508838A does not describe or suggest the technical significance of linking the phosphor moieties adjacent to each other by a linking group having a Molecular Flexibility of 0.510 or less and a cLogP of 6.0 or less, which is defined in the present invention.

Hereinafter, the compound according to the embodiment of the present invention will be described in detail.

### <Compound according to embodiment of present invention>

The compound according to the embodiment of the present invention is a compound containing two or more phosphor moieties, in which phosphor moieties adjacent to each other are linked by a linking group having a Molecular Flexibility of 0.510 or less and a cLogP of 6.0 or less.

The compound according to the embodiment of the present invention may be a compound classified into a polymer or an oligomer.

In addition, the "compound in which phosphor moieties adjacent to each other are linked by a linking group having a Molecular Flexibility of 0.510 or less and a cLogP of 6.0 or less" includes an aspect in which a phosphor moiety is bonded to a main chain of a linking group having a Molecular Flexibility of 0.510 or less and a cLogP of 6.0 or less, and an aspect in which a phosphor moiety is bonded to a side chain thereof, and a compound in which a phosphor moiety is bonded to a side chain of the linking group having a Molecular Flexibility of 0.510 or less and a cLogP of 6.0 or less is preferable.

In the compound according to the embodiment of the present invention, the number of the phosphor moieties is 2 or more. The upper limit value is not particularly limited, and it can be set to, for example, 30 or less, preferably 20 or less, more preferably 15 or less, still more preferably 10 or less, and particularly preferably 4 or less.

In the present invention, the Molecular Flexibility is preferably 0.505 or less. The preferred range is from 0.100 to 0.510, the more preferred range is 0.100 or more and 0.505 or less, and the still more preferred range is 0.100 to 0.502.

In the present invention, the cLogP is preferably 3.0 or less and more preferably 1.5 or less. The preferred range is -50.0 to 6.0, a more preferred range is -40.0 to 3.0, and a still more preferred range is -30.0 to 1.5.

Both the "Molecular Flexibility" which is a rigidity parameter and the "cLogP" which is a hydrophilicity parameter are values calculated using DataWarrior (available from https://openmolecules.org/datawarrior/) V5.5.0. The "Molecular Flexibility" is a value obtained by rounding off the fourth decimal place of the calculated value, and the "cLogP" is a value obtained by rounding off the second decimal place of the calculated value.

The structures of the linking groups used for the calculation are determined as follows.

### 1) Determination of main chain of compound

The main chain of the compound is determined such that each phosphor moiety is located on the side chain of the compound. In a case where at least one ring-constituting atom of the ring is included in the main chain of the compound, this ring is a structure constituting the main chain of the compound. For example, in a case where at least one of the ring-constituting atoms of the pyrrolidine ring is included in the main chain of the compound, the pyrrolidine ring is a structure constituting the main chain of the compound.

Provided that =O and =S are not counted as side chains, but are counted as atoms constituting the main chain.

In a case where the phosphor moiety is located at the terminal of the compound and there is no side chain, it is assumed that the phosphor moiety is bonded to the terminal of the main chain (that is, bonded to the main chain).

### 2) Determination of main chain constituting linking group

Among the main chains of the compounds determined above, a chain portion overlapping with a linking chain of two phosphor moieties adjacent to each other is defined as "the main chain 1 constituting the linking group". In a case where at least one of the ring-constituting atoms of the ring is included in the linking chain of two phosphor moieties adjacent to each other, the ring is a structure constituting the linking chain of the two phosphor moieties adjacent to each other.

### (i) Case where phosphor moiety is located in side chain of compound

In a case where a linking chain of two phosphor moieties adjacent to each other is traced from the terminal of the "main chain 1 constituting the linking group" to the phosphor moiety side, the "main chain constituting the linking group" is defined to include up to and including the following [bond formed by reaction of substituent group 1] that appears first.

In a case of tracing from the terminal of the "main chain 1 constituting the linking group" described above, a substituent selected from the following substituent group 1 is bonded to the terminal end side of the "main chain 1 constituting the linking group", and the substituent reacts with a substituent present on the phosphor moiety side to form the following [bond formed by reaction of substituent group 1].

### (ii) Case where phosphor moiety is located at terminal of main chain of compound

In the phosphor moiety (structural moiety consisting of an organic compound exhibiting fluorescence), in a case where "the main chain 1 constituting the linking group" is traced from a dye mother nucleus structure (conjugated structural moiety) contributing to fluorescence, a side chain on the phosphor moiety side is defined to include up to and including the front of the following [bond formed by reaction with substituent group 1] that appears first, and the "main chain 1 constituting the linking group" after this is defined as the "main chain constituting the linking group".

In addition, in a case of tracing from the above-described dye mother nucleus structure (conjugated structural moiety) contributing to fluorescence, a substituent selected from the following substituent group 1 is bonded to a terminal end side of the main chain 1 constituting the linking group, and the substituent reacts with a substituent present on a side of the dye mother nucleus structure (conjugated structural moiety) contributing to fluorescence to form the following [bond formed by reaction of substituent group 1].

Based on the (i) and (ii), a portion corresponding to the "main chain constituting the linking group" is determined among the linking chain of two phosphor moieties adjacent to each other.

### 3) Determination of structure of linking group used for calculation

### (Structure of linking group used for calculating Molecular Flexibility)

For the "main chain constituting the linking group" determined as described above, a structure in which Dye is replaced with a methyl group and a side chain (provided that =O and =S are not counted as side chains) with respect to the "main chain constituting the linking group" after the replacement is replaced with a hydrogen atom is used as the structure of the linking group used for the calculation of Molecular Flexibility as described in the following "bond formed by reaction of substituent group 1". It is noted that (i) in a case where the phosphor moiety is located at the side chain of the compound, a structure in which chain, different from the linking chain of the phosphor moiety and present at the terminal atom of the "main chain 1 constituting the linking group", is replaced with a methyl group is adopted.

### (Structure of linking group used for calculation of cLogP)

The structure of the linking group used in the calculation of cLogP is determined in the same manner as in the determination of the structure of the linking group used in the calculation of the Molecular Flexibility, except that the side chain (provided that =O and =S are not counted as the side chain) with respect to the "main chain constituting the linking group" is not replaced with the hydrogen atom in the determination of the structure of the linking group used in the calculation of the Molecular Flexibility.

### [Substituent group 1]

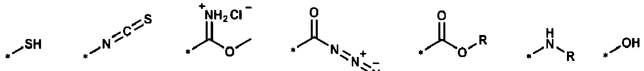

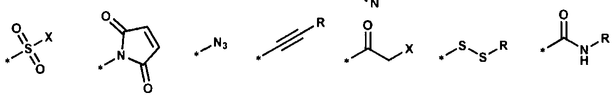

In the substituent, R represents a hydrogen atom, a group selected from an alkyl group, an alkenyl group, an alkynyl group, an aryl group, a heteroaryl group, and X, or a group obtained by combining two or more of these groups, a nitrogen atom, an oxygen atom, a sulfur atom, and a carbonyl group. R may be bonded to a part on the terminal end side of the main chain 1 constituting the linking group, which is represented by *, to form a ring. X represents a halogen atom. * represents a bonding site to the terminal end side of the main chain 1 constituting the linking group.

It is noted that R is not particularly limited as long as the group listed in the substituent group 1 can form a bond as a reactive group. Specific examples of the group listed in the substituent group 1 include the description of a substituent allowing binding to a biological substance described later.

Examples of the ring formed by bonding R to a part of the terminal end side of the main chain 1 constituting the linking group, which is represented by *, include a cyclooctine ring.

### [Bond formed by reaction of substituent group 1]

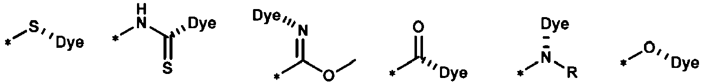

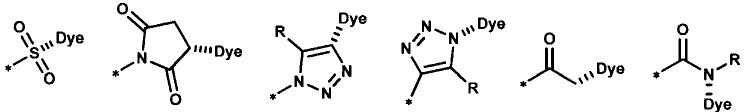

In the above-described substituent, Dye represents a structure containing a phosphor moiety. In the above-described calculation, a structure in which Dye bonded by a broken line is replaced with a methyl group is calculated. R has the same meaning as R in [Substituent group 1]. * represents a bonding site to the terminal end side of the main chain 1 constituting the linking group.

The linking group that links the above-described phosphor moieties adjacent to each other is preferably a group containing at least one of an alkynyl group, an aliphatic hydrocarbon ring, a heterocyclic ring, or a carbonyl group (>C=O).

The alkynyl group may be unsubstituted or may have a substituent.

The aliphatic hydrocarbon rings include a cycloalkane and a cycloalkene, and the aliphatic hydrocarbon ring may be a monocyclic ring or a fused ring. Examples of the aliphatic hydrocarbon ring include cyclopropane, cyclobutane, cyclopentane, cyclohexane, and cycloheptane, and cyclohexane is preferable.

As the heterocycle, the description according to the heterocyclic ring in the heterocyclic group of the substituent group T described later can be applied. The heterocyclic ring may be an aromatic heterocyclic ring or an aliphatic heterocyclic ring, and is preferably an aliphatic heterocyclic ring, and more preferably a 5-membered aliphatic heterocyclic ring. Examples of the heterocyclic ring include oxetane, thietane, azetidine, pyrrolidine, pyrazolidine, imidazolidine, tetrahydrothiophene, tetrahydrofuran, piperazine, tetrahydropyran, morpholine, γ-lactam, and β-lactam, and pyrrolidine is preferable.

The above-described alkynyl group, aliphatic hydrocarbon ring, and heterocyclic ring may be contained as a divalent group constituting the main chain of the linking group or as a monovalent or divalent group constituting the side chain of the linking group in the linking group that links the above-described phosphor moieties adjacent to each other.

Among these, it is preferable that the above-described alkynyl group, aliphatic hydrocarbon ring, and heterocyclic ring are at least contained in the linking group that links the above-described phosphor moieties adjacent to each other, as a divalent group constituting the main chain of the linking group. In this case, in a case where a linking group linking the above-described phosphor moieties adjacent to each other is structurally designed such that the above-described Molecular Flexibility is 0.510 or less, the main chain can be adjusted in a direction in which it is rigid (the Molecular Flexibility is reduced).

The above-described carbonyl group may be contained as a divalent group constituting the main chain of the linking group or as a divalent group constituting the side chain of the linking group in the linking group that links the above-described phosphor moieties adjacent to each other.

In addition, in a case where the above-described heterocyclic ring and carbonyl group are contained in the linking group that links the above-described phosphor moieties adjacent to each other, the linking group can be adjusted in a direction in which the linking group is hydrophilic (the cLogP is decreased) such that the cLogP is 6.0 or less in a case of structurally designing the linking group that links the above-described phosphor moieties adjacent to each other.

In the present invention, the relationship between the light absorption characteristics of two or more phosphor moieties present in the compound is not particularly limited, and it is preferable that at least two of the phosphor moieties are phosphor moieties having light absorption characteristics that are equivalent to each other.

The phrase "phosphor moieties having light absorption characteristics that are equivalent to each other" means those that satisfy a relationship in which a difference in the maximum absorption wavelength between the respective phosphor moieties in the absorption spectra is within 15 nm.

In a case where at least two of the phosphor moieties are phosphor moieties having light absorption characteristics that are equivalent to each other, the compound is preferably such that all the phosphor moieties contained in the compound satisfy a relationship in which a difference between the maximum absorption wavelength on the lowest wavelength side and the maximum absorption wavelength on the highest wavelength side among the maximum absorption wavelengths in the absorption spectra of the respective phosphor moieties is within 15 nm.

As a compound having two phosphor moieties in the compound, the compound that causes the FRET phenomenon is known as described above. In this compound, a difference in maximum absorption wavelength generally exceeds 15 nm between an absorption spectrum of a phosphor moiety I (an energy donor) that is excited with excitation light and an absorption spectrum of another phosphor moiety II (an energy acceptor) that receives energy from the phosphor moiety I and emits light or is quenched. In such a compound, the energy is transferred to the phosphor moiety II instead of emitting fluorescence from the phosphor moiety I, and thus the fluorescence intensity of the compound is decreased.

Therefore, in a case where all the phosphor moieties contained in the compound satisfy a relationship in which a difference between the maximum absorption wavelength on the lowest wavelength side and the maximum absorption wavelength on the highest wavelength side among the maximum absorption wavelengths in the absorption spectra of the respective phosphor moieties is within 15 nm, the fluorescence intensity can be exhibited in proportion to the number of phosphor moieties.

The chemical structures of the above-described phosphor moieties having light absorption characteristics that are equivalent to each other are not particularly limited as long as the above-described difference in maximum absorption wavelength is satisfied, and they preferably have the same structure in terms of the main skeleton of the phosphor moiety. Provided that the steric conformation, chain length, and the like of the substituent may be different from each other, and in a case where an anionic group or a cationic group is contained, a counter ion thereof may be different from each other. The difference in the maximum absorption wavelength is preferably within 10 nm and more preferably within 5 nm.

In addition, in the present invention, it is also preferable that at least two of the phosphor moieties are phosphor moieties having light absorption characteristics that are non-equivalent to each other.

The phrase "phosphor moieties having light absorption characteristics that are non-equivalent to each other" means those that satisfy a relationship in which a difference in the maximum absorption wavelength between the respective phosphor moieties in the absorption spectra is more than 15 nm.

In a case where at least two of the phosphor moieties are phosphor moieties having light absorption characteristics that are non-equivalent to each other, the compound is preferably such that all the phosphor moieties contained in the compound satisfy a relationship in which a difference between the maximum absorption wavelength on the lowest wavelength side and the maximum absorption wavelength on the highest wavelength side among the maximum absorption wavelengths in the absorption spectra of the respective phosphor moieties is more than 15 nm

The chemical structures of the above-described phosphor moieties having light absorption characteristics that are non-equivalent to each other are not particularly limited as long as the above-described difference in maximum absorption wavelength is satisfied, and is preferably a so-called different type of phosphor moiety having a different structure of the main skeleton of the phosphor moiety. The difference in maximum absorption wavelength is preferably more than 30 nm and more preferably more than 50 nm.

It is noted that the absorption spectrum of the phosphor moiety is a spectrum that is obtained by measuring, by using a spectrophotometer, a simple body of a phosphor constituting the phosphor moiety, which is diluted with a PBS buffer solution (phosphate-buffered saline).

In addition to the above, the description, the specific examples, and the like of the phosphor moiety in General Formula (II) described later can be applied to the phosphor moiety contained in the compound according to the embodiment of the present invention.

The linking group that links the above-described phosphor moieties adjacent to each other is more preferably a group containing a structure represented by General Formula (I).

It is noted that it is preferable that "the compound in which phosphor moieties adjacent to each other are each linked through a group having a structure represented by General Formula (I)" is a compound in which two structures having a phosphor moiety as a substituent are linked by a group including a structure represented by General Formula (I).

It is noted that the structure having the above-described phosphor moiety as a substituent is not particularly limited as long as the phosphor moieties adjacent to each other are each linked through a group having a structure represented by General Formula (I). For example, the structure may have, as a substituent, a group in which a 5-membered ring contains a phosphor moiety, where the 5-membered ring described in the structure represented by General Formula (I) is formed to have a carbon atom, a nitrogen atom, and X¹ to X³ as ring-constituting atoms by a combination of L¹ and L², a combination of L¹ and L³, a combination of L⁴ and L⁵, or a combination of L⁴ and L⁶, as described in detail in General Formula (II) described later. That is, the structure having a phosphor moiety as a substituent may be a structure in which the 5-membered ring described in the structure represented by General Formula (I), which has a carbon atom, a nitrogen atom, and X¹ to X³ as ring-constituting atoms, has a group in which a phosphor moiety is contained as a substituent as long as the phosphor moieties adjacent to each other are each linked through a group having a structure represented by General Formula (I). Examples of such a compound include a compound represented by General Formula (III) or (IV) described later.

### (Structure represented by General Formula (I))

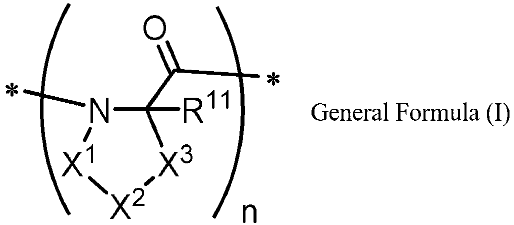

In the formula, X¹ to X³ represent -O-, -S-, >NR¹, or >CR²R³,
R¹ to R³ and R¹¹ represent a hydrogen atom, an alkyl group, an alkenyl group, an alkynyl group, an aryl group, a heteroaryl group, an acyl group, -NR⁸R⁹, -OR¹⁰, an anionic group, a betaine residue, a polyol residue, a sugar residue, a polyamino acid residue, -(O-L)_{g}R^{E}, or -(L-O)_{g}R^{E},
R⁸ to R¹⁰ represent a hydrogen atom, an alkyl group, an alkenyl group, an alkynyl group, an acyl group, an aryl group, a heteroaryl group, an anionic group, a betaine residue, a polyol residue, a sugar residue, a polyamino acid residue, -(O-L)_{g}R^{E}, or -(L-O)_{g}R^{E},
L represents an alkylene group, R^{E} has the same meaning as R⁸ to R¹⁰ described above, and g represents 2 to 50, provided that R^{E} represents neither -(O-L)_{g}R^{E} nor -(L-O)_{g}R^{E},
n represents an integer of 2 or more, and
* represents a bonding site.

In the present invention, the structure represented by General Formula (I) is preferably a structure represented by any one of General Formula (IA) or (IB) in consideration of stereoisomers. It is noted that X¹ to X³, R¹¹, and n in the following general formula have the same meanings as X¹ to X³, R¹¹, and n in General Formula (I).

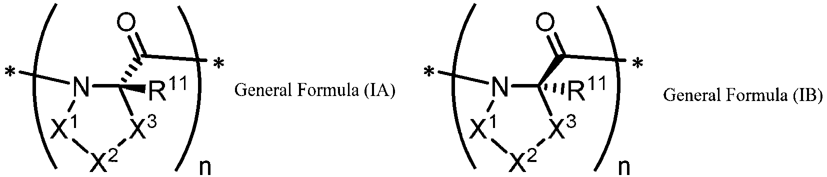

X¹ to X³ represent -O-, -S-, >NR¹, or >CR²R³, and R¹ to R³ and R¹¹ represent a hydrogen atom, an alkyl group, an alkenyl group, an alkynyl group, an acyl group, an aryl group, a heteroaryl group, -NR⁸R⁹, -OR¹⁰, an anionic group, a betaine residue, a polyol residue, a sugar residue, a polyamino acid residue, -(O-L)_{g}R^{E}, or -(L-O)_{g}R^{E}.

The alkyl group, the alkenyl group, the alkynyl group, the acyl group, the aryl group, the heteroaryl group, the anionic group, the betaine residue, the polyol residue, the sugar residue, the polyamino acid residue, -(O-L)_{g}R^{E}, and -(L-O)_{g}R^{E}, which can be adopted as R¹ to R³ and R¹¹, have the same meanings as the alkyl group, the alkenyl group, the alkynyl group, the acyl group, the aryl group, the heteroaryl group, the anionic group, the betaine residue, the polyol residue, the sugar residue, the polyamino acid residue, -(O-L)_{g}R^{E}, and -(L-O)_{g}R^{E} in the substituent group T described later, and the same applies to the preferred ranges thereof.

The alkyl group, the alkenyl group, the alkynyl group, the acyl group, the aryl group, the heteroaryl group, which can be adopted as R¹ to R³ and R¹¹ may be unsubstituted or may have a substituent.

Examples of the substituent which may be contained in the alkyl group, the alkenyl group, the alkynyl group, the acyl group, the aryl group, the heteroaryl group, as R¹ to R³ and R¹¹ include substituents in the substituent group T which will be described later. For example, a halogen atom, an anionic group, a betaine residue, a polyol residue, a sugar residue, a polyamino residue, -(O-L)_{g}R^{E}, or -(L-O)_{g}R^{E} is preferable.

In -NR⁸R⁹ and -OR¹⁰ which can be adopted as R¹ to R³ and R¹¹, R⁸ to R¹⁰ represent a hydrogen atom, an alkyl group, an alkenyl group, an alkynyl group, an acyl group, an aryl group, a heteroaryl group, an anionic group, a betaine residue, a polyol residue, a sugar residue, a polyamino acid residue, -(O-L)_{g}R^{E}, or -(L-O)_{g}R^{E}.

The alkyl group, the alkenyl group, the alkynyl group, the acyl group, the aryl group, the heteroaryl group, the anionic group, the betaine residue, the polyol residue, the sugar residue, the polyamino acid residue, -(O-L)_{g}R^{E}, and -(L-O)_{g}R^{E}, which can be adopted as R⁸ to R¹⁰, have the same meanings as the alkyl group, the alkenyl group, the alkynyl group, the acyl group, the aryl group, the heteroaryl group, the anionic group, the betaine residue, the polyol residue, the sugar residue, the polyamino acid residue, -(O-L)_{g}R^{E}, and -(L-O)_{g}R^{E} in the substituent group T described later, and the same applies to the preferred ranges thereof.

The alkyl group, the alkenyl group, the alkynyl group, the acyl group, the aryl group, the heteroaryl group, which can be adopted as R⁸ to R¹⁰, may be unsubstituted or may have a substituent.

Examples of the substituent which may be contained in the alkyl group, the alkenyl group, the alkynyl group, the acyl group, the aryl group, and the heteroaryl group as R⁸ to R¹⁰ include substituents in the substituent group T which will be described later, and a halogen atom, a carbamoyl group, an acylamino group, an alkoxy group (preferably an alkoxy group having an anionic group, a betaine residue, a polyol residue, a sugar residue, a polyamino acid residue, -(O-L)_{g}R^{E} or -(L-O)_{g}R^{E}), a heteroaryl group, an anionic group, a betaine residue, a polyol residue, a sugar residue, a polyamino acid residue, -(O-L)_{g}R^{E}, or -(L-O)_{g}R^{E} is preferable, and a carbamoyl group, an acylamino group, an alkoxy group (preferably an alkoxy group having an anionic group, a betaine residue, a polyol residue, a sugar residue, a polyamino acid residue, -(O-L)_{g}R^{E}, or -(L-O)_{g}R^{E}), a heteroaryl group, an anionic group, a betaine residue, a polyol residue, a sugar residue, a polyamino acid residue, -(O-L)_{g}R^{E}, or -(L-O)_{g}R^{E} is more preferable.

R¹ is preferably a hydrogen atom, an alkyl group, -NR⁸R⁹, -OR¹⁰, an anionic group, a betaine residue, a polyol residue, a sugar residue, a polyamino acid residue, -(O-L)_{g}R^{E}, or -(L-O)_{g}R^{E}.

As R² and R³, a hydrogen atom, -NR⁸R⁹, -OR¹⁰, an anionic group, a betaine residue, a polyol residue, a sugar residue, a polyamino acid residue, -(O-L)_{g}R^{E}, or -(L-O)_{g}R^{E} is preferable, and it is more preferable that R² is a hydrogen atom, -NR⁸R⁹, -OR¹⁰, an anionic group, a betaine residue, a polyol residue, a sugar residue, a polyamino acid residue, -(O-L)_{g}R^{E}, or -(L-O)_{g}R^{E}, and R³ is a hydrogen atom.

R¹¹ is preferably a hydrogen atom, an alkyl group, -NR⁸R⁹, -OR¹⁰, or an anionic group, and more preferably a hydrogen atom.

R⁸ to R¹⁰ are preferably a hydrogen atom, an alkyl group, or an acyl group. The alkyl group may be an alkyl group containing at least one of an anionic group, a betaine residue, a polyol residue, a sugar residue, a polyamino acid residue, -(O-L)_{g}R^{E}, or -(L-O)_{g}R^{E}, and the acyl group may be an acyl group containing at least one of an anionic group, a betaine residue, a polyol residue, a sugar residue, a polyamino acid residue, -(O-L)_{g}R^{E}, or -(L-O)_{g}R^{E}.

The acyl group containing at least one of the anionic group, the betaine residue, the polyol residue, the sugar residue, the polyamino acid residue, -(O-L)_{g}R^{E}, or -(L-O)_{g}R^{E} is an acyl group substituted with preferably an acyl group having at least one of the anionic group, the betaine residue, the polyol residue, the sugar residue, the polyamino acid residue, -(O-L)_{g}R^{E}, or -(L-O)_{g}R^{E}, or with an alkoxy group or a heteroaryl group, having at least one of the anionic group, the betaine residue, the polyol residue, the sugar residue, the polyamino acid residue, -(O-L)_{g}R^{E}, or -(L-O)_{g}R^{E}. The alkyl group and the acyl group may have a substituent other than the anionic group, the betaine residue, the polyol residue, the sugar residue, the polyamino acid residue, -(O-L)_{g}R^{E}, and -(L-O)_{g}R^{E}, and examples thereof include substituents in the substituent group T which will be described later, and for example, a carbamoyl group or an acylamino group is preferable.

It is noted that -(O-L)_{g}R^{E} and -(L-O)_{g}R^{E} are included as a divalent group obtained by removing one hydrogen atom from a hydrogen atom or a substituent, which can be adopted as R^{E}, and an anionic group, a sulfo group, a phosphono group, a betaine residue, a polyol residue, a sugar residue, or a polyamino acid residue may be bonded thereto. Examples of the divalent group obtained by removing one hydrogen atom from the hydrogen atom or the substituent, which can be adopted as R^{E}, in -(O-L)_{g}R^{E} or -(L-O)_{g}R^{E} include -(O-L)_{g}-, -(O-L)_{g}-NHCO-, -(O-L)_{g}-CONH-, -(O-L)_{g}-CONH alkylene-, -(O-L)_{g}-NHCO alkylene-, -(L-O)_{g}-, -(L-O)_{g}-NHCO-, -(L-O)_{g}-CONH-, -(L-O)_{g}-CONH alkylene-, and -(L-O)_{g}-NHCO alkylene-.

Regarding X¹ to X³, it is preferable that at least any one thereof is >CR²R³, and it is more preferable that at least two thereof are >CR²R³ and the remaining one is -O-, -S-, or >CR²R³.

In the present invention, from the viewpoint of making the structure represented by General Formula (I) a more rigid structure, it is preferable that the structure represented by General Formula (I) includes a structure in which X¹ to X³ are >CR²R³. That is, "the structure represented by General Formula (I) includes a structure in which X¹ to X³ are >CR²R³" means that all of X¹ to X³ are >CR²R³ in at least one structure represented by General Formula (i) among n pieces of the consecutive structures represented by General Formula (i) described above.

The proportion of the number of the structures in which X¹ to X³ are >CR²R³ among the structures represented by General Formula (I) is preferably 30% or more, more preferably 60% or more, and still more preferably 80%. The upper limit of the ratio is not particularly limited and may be 100% or less. It is noted that it is also preferable that all of the structures represented by General Formula (I) are structures in which X¹ to X³ described above are >CR²R³.

From the viewpoint of suppressing the intermolecular interaction and further the intramolecular interaction between structures represented by General Formula (I) in the case where a plurality of structures are present, at least one R² in the structure in which X¹ to X³ described above are >CR²R³ is preferably -NR⁸R⁹, -OR¹⁰, an anionic group, a betaine residue, a polyol residue, a sugar residue, a polyamino acid residue, -(O-L)_{g}R^{E}, or -(L-O)_{g}R^{E}, and more preferably a group which is -NR⁸R⁹, -OR¹⁰, an anionic group, a betaine residue, a polyol residue, a sugar residue, a polyamino acid residue, -(O-L)_{g}R^{E}, or -(L-O)_{g}R^{E}, where at least one of R⁸, ..., or R¹⁰ is a group containing at least one of an acyl group, an anionic group, a betaine residue, a polyol residue, a sugar residue, a polyamino acid residue, -(O-L)_{g}R^{E}, or -(L-O)_{g}R^{E}.

It is noted that the above-described "at least one R² in the structure in which X¹ to X³ are >CR²R³ is -NR⁸R⁹, -OR¹⁰, an anionic group, a betaine residue, a polyol residue, a sugar residue, a polyamino acid residue, -(O-L)_{g}R^{E}, or -(L-O)_{g}R^{E}, and at least one of R⁸, ..., or R¹⁰ is a group containing at least one of an acyl group, an anionic group, a betaine residue, a polyol residue, a sugar residue, a polyamino acid residue, -(O-L)_{g}R^{E}, or -(L-O)_{g}R^{E}" means that, that is, in a case where R² is -NR⁸R⁹, at least one of R⁸ or R⁹ is a group containing at least one of an acyl group, an anionic group, a betaine residue, a polyol residue, a sugar residue, a polyamino acid residue, -(O-L)_{g}R^{E}, or -(L-O)_{g}R^{E}, and in a case where R² is -OR¹⁰, R¹⁰ is a group containing at least one of an anionic group, a betaine residue, a polyol residue, a sugar residue, a polyamino acid residue, -(O-L)_{g}R^{E}, or -(L-O)_{g}R^{E}.

In addition, the case where R¹⁰ is a group containing at least one of an anionic group, a betaine residue, a polyol residue, a sugar residue, a polyamino acid residue, -(O-L)_{g}R^{E}, or -(L-O)_{g}R^{E} means that R¹⁰ is an anionic group, a betaine residue, a polyol residue, a sugar residue, a polyamino acid residue, -(O-L)_{g}R^{E}, or -(L-O)_{g}R^{E}, or a group containing, as a substituent, at least one of an anionic group, a betaine residue, a polyol residue, a sugar residue, a polyamino acid residue, -(O-L)_{g}R^{E}, or -(L-O)_{g}R^{E}. The same applies to a case where at least one of R⁸ or R⁹ is a group containing at least one of an anionic group, a betaine residue, a polyol residue, a sugar residue, a polyamino acid residue, -(O-L)_{g}R^{E}, or -(L-O)_{g}R^{E}.

Preferred examples of the "group containing at least one of an acyl group, an anionic group, a betaine residue, a polyol residue, a sugar residue, a polyamino acid residue, -(O-L)_{g}R^{E}, or -(L-O)_{g}R^{E}" include "an alkyl group containing at least one of an anionic group, a betaine residue, a polyol residue, a sugar residue, a polyamino acid residue, -(O-L)_{g}R^{E}, or -(L-O)_{g}R^{E} or an acyl group containing at least one of an anionic group, a betaine residue, a polyol residue, a sugar residue, a polyamino acid residue, -(O-L)_{g}R^{E}, or -(L-O)_{g}R^{E}" described above. In addition, preferred examples thereof also include an acyl group, an anionic group, a betaine residue, a polyol residue, a sugar residue, a polyamino acid residue, -(O-L)_{g}R^{E}, and -(L-O)_{g}R^{E} themselves, and an acyl group is preferable.

n represents an integer of 2 or more. Since n is an integer of 2 or more, the compound according to the embodiment of the present invention can impart the rigidity that is effective for suppressing the association of dyes (phosphor moieties), to a structure represented by General Formula (I), and a decrease in the fluorescence intensity can be suppressed.

In the present invention, in a case where the number of phosphor moieties is 2, the lower limit value of n is preferably an integer of 3 or more, more preferably an integer of 6 or more, still more preferably an integer of 9 or more, and particularly preferably an integer of 12 or more, from the viewpoint of further suppressing the dye association. The upper limit value thereof is not particularly limited and can be set to, for example, an integer of 72 or less, and it is preferably an integer of 36 or less, more preferably an integer of 24 or less, and still more preferably an integer of 18 or less. That is, n is preferably an integer of 3 to 72, more preferably an integer of 6 to 36, still more preferably an integer of 9 to 24, and particularly preferably an integer of 12 to 18.

On the other hand, in a case where the number of phosphor moieties is 3 or more, a certain degree of dye association can be allowed since the number of phosphor moieties (the number of dyes) per one molecule is large as compared with a case where the number of phosphor moieties is 2, and in a case where the number of phosphor moieties is an integer of 3 or more, the fluorescence intensity can be further improved, which is preferable. Among the above, in a case where n is an integer of 6, there is a certain degree of effect of suppressing the association, which is insufficient. Therefore, n is more preferably an integer of 7 or more. In a case where the number of phosphor moieties is 3 or more, the upper limit value of n is, for example, preferably an integer of 72 or less, more preferably an integer of 36 or less, and still more preferably an integer of 24 or less. That is, in a case where the number of phosphor moieties is 3 or more, n is preferably an integer of 3 to 72, more preferably an integer of 7 to 36, and still more preferably an integer of 7 to 24.

### <Compound represented by General Formula (II)>

Among the compounds according to the embodiment of the present invention, a compound in which the linking group that links the phosphor moieties adjacent to each other is a group containing a structure represented by General Formula (I) is preferably represented by General Formula (II).

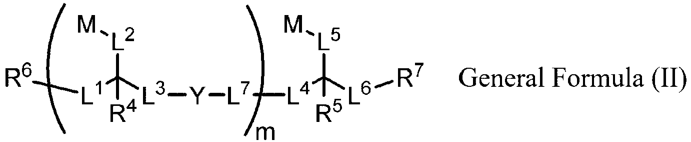

In the formula, R⁴ and R⁵ represent a hydrogen atom, an alkyl group, an alkenyl group, an alkynyl group, an acyl group, an amino group, a hydroxy group, an alkoxy group, a sulfanyl group, an aryl group, or a heteroaryl group,
R⁶ and R⁷ represent a hydrogen atom, a hydroxy group, a sulfanyl group, an alkyl group, an alkenyl group, an alkynyl group, an aryl group, an alkoxy group, an amino group, an acyl group, a heteroaryl group, or Q,
Q represents an anionic group, a cationic group, a betaine residue, a polyol residue, a sugar residue, a polyamino acid residue, -(O-L)_{g}R^{E}, -(L-O)_{g}R^{E}, a substituent allowing binding to a biological substance, or a substituent allowing binding to a solid support,
L¹ to L⁷ represent a single bond or a divalent linking group,
M represents a phosphor moiety, a physiologically active substance moiety, a prodrug moiety, or a radioactive isotope-containing moiety,
Y represents the structure represented by General Formula (I) described above,
m is an integer of 1 or more, and
L, R^{E}, and g have the same meanings as L, R^{E}, and g, all of which are described above,
provided that at least two of M's represent the phosphor moieties described above.

R⁴ and R⁵ represent a hydrogen atom, an alkyl group, an alkenyl group, an alkynyl group, an acyl group, an amino group, a hydroxy group, an alkoxy group, a sulfanyl group, an aryl group, or a heteroaryl group.

The alkyl group, the alkenyl group, the alkynyl group, the acyl group, the amino group, the alkoxy group, the aryl group, and the heteroaryl group, which can be adopted as R⁴ or R⁵, have the same meanings as the alkyl group, the alkenyl group, the alkynyl group, the acyl group, the amino group, the alkoxy group, the aryl group, and the heteroaryl group in the substituent group T which will be described later, and the same also applies to the preferred ranges thereof.

The alkyl group, the alkenyl group, the alkynyl group, the acyl group, the amino group, the alkoxy group, the aryl group, and the heteroaryl group, which can be adopted as R⁴ or R⁵, may be unsubstituted or may have a substituent, and examples of the substituent which may be contained therein include substituents in the substituent group T which will be described later. For example, a halogen atom is preferable.

From the viewpoint of ease of synthesis, R⁴ and R⁵ are preferably a hydrogen atom. R⁴ and R⁵ are often a hydrogen atom in a case where an amino acid is used as a raw material; however, the substituents in R⁴ and R⁵ do not greatly contribute to the exhibition of the excellent effect of suppressing the association in a solution state by the compound according to the embodiment of the present invention, and thus R⁴ and R⁵ may be another substituent (an alkyl group, an alkenyl group, an alkynyl group, an acyl group, an amino group, a hydroxy group, an alkoxy group, a sulfanyl group, an aryl group, or a heteroaryl group) other than the hydrogen atom.

R⁶ and R⁷ represent a hydrogen atom, a hydroxy group, a sulfanyl group, an alkyl group, an alkenyl group, an alkynyl group, an aryl group, an alkoxy group, an amino group, an acyl group, a heteroaryl group, or Q.

The alkyl group, the alkenyl group, the alkynyl group, the aryl group, the alkoxy group, the amino group, the acyl group, and the heteroaryl group, which can be adopted as R⁶ or R⁷, have the same meanings as the alkyl group, the alkenyl group, the alkynyl group, the aryl group, the alkoxy group, the amino group, the acyl group, and the heteroaryl group in the substituent group T which will be described later, and the same also applies to the preferred ranges thereof.

The alkyl group, the alkenyl group, the alkynyl group, the aryl group, the alkoxy group, the amino group, the acyl group, and the heteroaryl group, which can be adopted as R⁶ or R⁷, may be unsubstituted or may have a substituent.

Examples of the substituent which may be contained in the alkyl group, the alkenyl group, the alkynyl group, the aryl group, the alkoxy group, the amino group, the acyl group, and the heteroaryl group, as R⁶ or R⁷, include substituents in the substituent group T which will be described later, where an alkyl group, an acyl group, an alkoxy group, an amino group, or Q, or a substituent obtained by combining two or more thereof is preferable, and an alkyl group, an acyl group, an alkoxy group, an amino group, -(O-L)_{g}R^{E}, -(L-O)_{g}R^{E}, a carboxy group, a substituent allowing binding to a biological substance, or a substituent allowing binding to a solid support, or a substituent obtained by combining two or more of these substituents is more preferable.

Q that can be adopted as R⁶ or R⁷ represents an anionic group, a cationic group, a betaine residue, a polyol residue, a sugar residue, a polyamino acid residue, -(O-L)_{g}R^{E}, -(L-O)_{g}R^{E}, a substituent allowing binding to a biological substance, or a substituent allowing binding to a solid support.

The anionic group, the cationic group, the betaine residue, the polyol residue, the sugar residue, the polyamino acid residue, -(O-L)_{g}R^{E}, and -(L-O)_{g}R^{E} have the same meanings as the anionic group, the cationic group, the betaine residue, the polyol residue, the sugar residue, the polyamino acid residue, -(O-L)_{g}R^{E}, and -(L-O)_{g}R^{E} in the substituent group T which will be described later, and the preferred ranges thereof are also the same.

As the substituent allowing binding to a biological substance, the description of the substituent allowing binding to a biological substance described later can be applied, and as the substituent allowing binding to a solid support, the description of the substituent allowing binding to a solid support described later can be applied.

Q is preferably a carboxy group, a substituent allowing binding to a biological substance, or a substituent allowing binding to a solid support, and more preferably a substituent allowing binding to a biological substance or a substituent allowing binding to a solid support.

For R⁶, the description of the substituent represented by -L¹³R^{6A} described later is preferably described, and For R⁷, the description of the substituent represented by -L¹²R^{7A} described later is preferably described. Specifically, the description of the substituent represented by -L¹³R^{6A} means a substituent obtained by a combination of a group that can be adopted as L¹³ and a group that can be adopted as R^{6A} . This also applies to -L¹²R^{7A}.

L² to L⁵ and L⁷ represent a single bond or a divalent linking group and are preferably a single bond or a linking group obtained by combining one or two or more of an alkylene group, an alkenylene group, an alkynylene group, an arylene group, a heteroarylene group, -O-, -S-, >C=O, >NR^{A}, >S=O, >S(=O)₂, and >P(=O)OR^{B}. R^{A} and R^{B} respectively have the same meanings as R^{A} and R^{B} described later and represent a hydrogen atom or a substituent.

L¹ and L⁶ represent a single bond or a divalent linking group and are preferably a single bond, an alkylene group, an alkenylene group, an alkynylene group, an arylene group, a heteroarylene group, -O-, -S-, >C=O, >NR^{A}, >S=O, >S(=O)₂, or >P(=O)OR^{B}. R^{A} and R^{B} respectively have the same meanings as R^{A} and R^{B} described later and represent a hydrogen atom or a substituent.

The alkylene group that can constitute L¹ to L⁷ has the same meaning as the group in which one hydrogen atom is further removed from the alkyl group selected from the substituent group T, which is described later, and the same applies to the preferred one thereof.

The alkenylene group that can constitute L¹ to L⁷ has the same meaning as the group in which one hydrogen atom is further removed from the alkenyl group selected from the substituent group T, which is described later, and the same applies to the preferred one thereof.

The alkenylene group that can constitute L¹ to L⁷ has the same meaning as the group in which one hydrogen atom is further removed from the alkynyl group selected from the substituent group T, which is described later, and the same applies to the preferred one thereof.

The arylene group that can constitute L¹ to L⁷ has the same meaning as the group in which one hydrogen atom is further removed from the aryl group selected from the substituent group T, which is described later, and the same applies to the preferred one thereof.

The heteroarylene group that can constitute L¹ to L⁷ is synonymous with the group in which one hydrogen atom is further removed from the heteroaryl group selected from the substituent group T, which is described later, and the same applies to the preferred one thereof.

The alkylene group, the alkenylene group, the alkynylene group, the arylene group, and the heteroarylene group, which can constitute L¹ to L⁷, may be an unsubstituted group or a group having a substituent.

The substituent which may be contained in the alkylene group, the alkenylene group, the alkynylene group, the arylene group, and the heteroarylene group, which can constitute L¹ to L⁷, is not particularly limited, and it is preferably selected from a substituent group T described later. More preferred examples thereof include a halogen atom, an alkyl group, an acylamino group, and a carbamoyl group. In addition, the substituent which may be contained in the alkylene group, the alkenylene group, the alkynylene group, the arylene group, and the heteroarylene group, which can constitute L¹ to L⁷, may be substituted with a substituent selected from the substituent group T which will be described later, and it is, for example, preferably an amino group.

In addition, the number of substituents which may be contained in the alkylene group, the alkenylene group, the alkynylene group, the arylene group, and the heteroarylene group, which can constitute L¹ to L⁷, is not particularly limited as long as it can be adopted as a structure. The number thereof can be set to at least one or more, the upper limit thereof is not particularly limited, and for example, all hydrogen atoms in the alkylene group, the alkenylene group, the alkynylene group, the arylene group, and the heteroarylene group may be substituted with a substituent.

The substituent which can be adopted as R^{A} in >NR^{A} and as R^{B} in >P(=O)OR^{B}, which can constitute L¹ to L⁷, is not particularly limited, and it is preferably selected from the substituent group T which will be described later. R^{A} is preferably a hydrogen atom, an alkyl group, an alkenyl group, an alkynyl group, an aryl group, a heteroaryl group, or an anionic group, more preferably a hydrogen atom or an alkyl group, and still more preferably a hydrogen atom. R^{B} is preferably a hydrogen atom, an alkyl group, an alkenyl group, an alkynyl group, an aryl group, or a heteroaryl group, more preferably a hydrogen atom or an alkyl group, and still more preferably a hydrogen atom.

It is noted that all the alkyl group, the alkenyl group, the alkynyl group, the aryl group, and the heteroaryl group, which can be adopted as R^{A} and R^{B}, may be an unsubstituted group or a group having a substituent.

In the linking group obtained by combining two or more of an alkylene group, an alkenylene group, an alkynylene group, an arylene group, a heteroarylene group, -O-, -S-, >C=O, >NR^{A}, >S=O, >S(=O)₂, and >P(=O)OR^{B}, which can constitute L² to L⁵ and L⁷, the type of the group to be combined is not particularly limited as long as the group to be combined has a reasonable chemical structure; however, it is preferably 2 to 6 types and more preferably 2 to 4 types. It is noted that in a case of counting, as one type, each of the alkylene group, the alkenylene group, the alkynylene group, the arylene group, the heteroarylene group, -O-, -S-, >C=O, >NR^{A}, >S=O, >S(=O)₂, and >P(=O)OR^{B}, the maximum number of types is 12.

It is noted that in the linking group obtained by combining two or more of an alkylene group, an alkenylene group, an alkynylene group, an arylene group, a heteroarylene group, -O-, -S-, >C=O, >NR^{A}, >S=O, >S(=O)₂, and >P(=O)OR^{B}, which can constitute L² to L⁵ and L⁷, the number of groups to be combined is not particularly limited; however, examples thereof preferably include 2 to 10 groups, more preferably include 2 to 6 groups, and still more preferably 2 to 4 groups.

### (i) L¹ and L⁶

L¹ and L⁶ are more preferably a single bond, >C=O, >NR^{A}, an arylene group, an alkylene group, -O-, or -S-, still more preferably a single bond, >C=O, >NR^{A}, an arylene group, or an alkylene group, and particularly preferably a single bond, >C=O or >NR^{A}.

### (ii) L³, L⁴, and L⁷

L³, L⁴, and L⁷ are more preferably a single bond, >C=O, >NR^{A}, an alkylene group, an alkenylene group, an alkynylene group, an arylene group, or a heteroarylene group, or a combination of at least one of an alkylene group, an alkenylene group, an alkynylene group, an arylene group, or a heteroarylene group, and >C=O and >NR^{A}, and still more preferably a single bond, >C=O, >NR^{A}, an alkylene group, an alkenylene group, an alkynylene group, an arylene group, or a heteroarylene group, or a group that links -C(=O)NR^{A}- to >C=O, or >NR^{A}C(=O)- to >NR^{A} by at least one of an alkylene group, an alkenylene group, an alkynylene group, an arylene group, or a heteroarylene group.

### (iii) L² and L⁵

L² and L⁵ are more preferably a group obtained by combining one or two or more of a single bond, an alkylene group, an alkenylene group, an alkynylene group, an arylene group, a heteroarylene group, -O-, -S-, >C=O, and >NR^{A}, and are still more preferably a group represented by * -L^{x}-L^{y}-**.

L^{x} is a single bond or a group obtained by combining one or two or more of an alkylene group, an alkenylene group, an alkynylene group, an arylene group, and a heteroarylene group, and L^{y} is a single bond, -O-, -S-, >C=O, or >NR^{A}. * represents a bonding site to a carbon atom to which L¹ and L³ are bonded or a carbon atom to which L⁴ and L⁶ are bonded, and ** represents a bonding site to M. Provided that in a case where L^{y} is a single bond, L^{x} is a heteroarylene group or a group consisting of a group obtained by combining one or two or more of an alkylene group, an alkenylene group, an alkynylene group, and an arylene group, which are located on the * side, and a heteroarylene group which is located on the ** side.

Among the groups represented by * -L^{x}-L^{y}-**, L² and L⁵ are preferably a group in which L^{y} is a single bond, -S-, >C=O, or >NR^{A}, more preferably a group in which L^{y} is >C=O or >NR^{A}, and still more preferably a group in which L^{x} is an alkylene group and L^{y} is >C=O or >NR^{A}.

It is noted that in the compound represented by General Formula (II), it suffices that the number of shortest-distance atoms in the linking chain (each linking chain of the linking chain including L² and the linking chain including L⁵) that connects M to a carbon atom to which L¹ and L³ are bonded or a carbon atom to which L⁴ and L⁶ are bonded is, for example, 1 to 60, where 1 to 40 is preferable. In a case where M is a phosphor moiety, the above-described number of shortest-distance atoms means the number of atoms that constitute the shortest chain in the linking chain that connects a conjugated structural moiety for exhibiting fluorescence in the phosphor moiety M to a carbon atom to which L¹ and L³ are bonded or a carbon atom to which L⁴ and L⁶ are bonded.

It is noted that in the compound represented by General Formula (II), it is also preferable that a structure represented by -(CH₂-CH₂-O)_{b}- described later (b is also as described later) is provided at any portion of the linking chain represented by "-(linking group ZZZ described later)-L²-" in the structure represented by (the conjugated structural moiety of M)-(the linking group ZZZ described later)-L²- and any portion of the linking chain represented by "-(linking group ZZZ described later)-L⁵-" in the structure represented by (the conjugated structural moiety of M)-(the linking group ZZZ described later)-L⁵-.

In the compound represented by General Formula (II), adjacent groups may be bonded to each other to form a ring. Examples of the combination of adjacent groups which may be bonded to each other to form a ring include a combination of L¹ and L², a combination of L¹ and L³, a combination of L² and R⁴, a combination of L⁴ and L⁵, a combination of L⁴ and L⁶, or a combination of L⁵ and R⁵.

The above-described ring which may be formed by bonding adjacent groups to each other may be any one of an aromatic ring or an aliphatic ring or may be any one of a hydrocarbon ring or a hetero ring, and it is preferably a 5- or 6-membered ring.

The preferred examples of the aliphatic ring include a cyclopentane ring, a cyclohexane ring, or a 5-membered ring having a carbon atom, a nitrogen atom, and X¹ to X³ as ring-constituting atoms, which is described in the structure represented by General Formula (I) described above, where a 5-membered ring having a carbon atom, a nitrogen atom, and X¹ to X³ as ring-constituting atoms, which is described in the structure represented by General Formula (I) described above, is more preferable.

The aromatic ring is preferably a benzene ring or a nitrogen-containing aromatic heterocyclic ring, more preferably a benzene ring or a nitrogen-containing aromatic heterocyclic ring in which the ring-constituting atom is composed of a carbon atom and a nitrogen atom, and still more preferably a benzene ring or a pyridine ring.

These rings may have a substituent, and the substituent which may be contained is not particularly limited and is selected from the substituent group T.

The ring formed by the combination of L² and R⁴ or the combination of L⁵ and R⁵ may be any one of the above-described aliphatic ring or aromatic ring, where the above-described aliphatic ring is preferable.

The ring formed by a combination of L¹ and L², a combination of L¹ and L³, a combination of L⁴ and L⁵, or a combination of L⁴ and L⁶, may be any one of the above-described aliphatic ring or aromatic ring, where a 5-membered ring having a carbon atom, a nitrogen atom, and X¹ to X³ as ring-constituting atoms, which is described in the structure represented by General Formula (I) described above, or a benzene ring is preferable.

For example, the following structures surrounded by broken lines in General Formula (II) 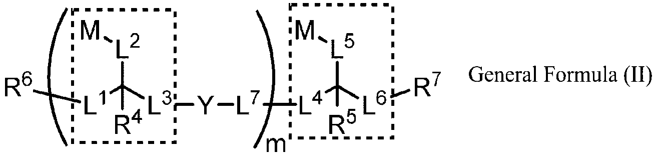 can be set to structures including the following structures. In the following structure, * indicates a connecting portion.

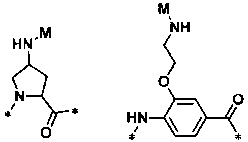

m is an integer of 1 or more. The upper limit value is not particularly limited, and can be set to, for example, an integer of 30 or less, and it is preferably an integer of 20 or less, more preferably an integer of 15 or less, still more preferably an integer of 10 or less, and particularly preferably an integer of 3 or less. That is, m can be an integer of 1 to 30, and is preferably an integer of 1 to 20, more preferably an integer of 1 to 15, and still more preferably an integer of 1 to 10.

M represents a phosphor moiety, a physiologically active substance moiety, a prodrug moiety, or a radioactive isotope-containing moiety. Provided that at least two of M's represent a phosphor moiety.

The phosphor moiety which can be adopted as M (hereinafter, also referred to as the phosphor moiety M) can be used without particular limitation as long as it is a structural moiety consisting of an organic compound that exhibits fluorescence. In addition, the phosphor moiety M may be a structural moiety in which a structural moiety consisting of an organic compound exhibiting fluorescence further has a linking group. Such a linking group is not particularly limited; however, examples thereof include a linking group ZZZ described later. For example, in the compound represented by General Formula (II), preferred examples of the compound include a compound in which the phosphor moiety M is bonded to L² or L⁵ by this linking group ZZZ.

Examples of the phosphor moiety M include a structural moiety consisting of at least one dye of a xanthene dye, a rhodamine dye, a coumarin dye, a cyanine dye, a pyrene dye, an oxazine dye, a squarylium dye, a pyridyloxazole dye, or a pyrromethene dye, which is preferable.

As the xanthene dye, the rhodamine dye, the coumarin dye, the cyanine dye, the pyrene dye, the oxazine dye, the squarylium dye, the pyridyloxazole dye, and the pyrromethene dye, dyes that are generally known as these dyes can be used without particular limitation.

As one aspect, the phosphor moiety M is preferably a structural moiety consisting of a pyrromethene dye. Examples of the pyrromethene dye include a dipyrromethene boron complex. As the dipyrromethene boron complex, it is possible to use a fluorescent compound (a dipyrromethene boron complex) represented by General Formula (1) or (4) described in WO2019/230963A, or a compound (a dipyrromethene boron complex) represented by General Formula (1) described in WO2021/100814A, and the description thereof can be incorporated into the present specification by reference.

It is noted that the incorporation is made such that the dye that constitutes the phosphor moiety M does not have a substituent allowing binding to a biological substance.

As another aspect, the phosphor moiety M is preferably a structural moiety consisting of a cyanine dye, and it is more preferably a structural moiety consisting of a cyanine dye represented by General Formula (α).

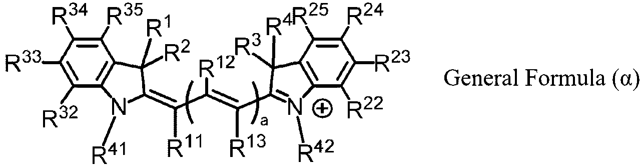

In the formula, R¹ to R⁴ represent an alkyl group or -(CH₂-CH₂-O)_{b}-R²¹. b is 1 to 50, and R²¹ represents an alkyl group.

R¹¹ to R¹³ represent a hydrogen atom, an alkyl group, an alkoxy group, an aryloxy group, an alkylthio group, an arylthio group, an amino group, or a halogen atom, where adjacent groups may be bonded to each other to form a 5-membered or 6-membered ring.

R²² to R²⁵ and R³² to R³⁵ represent a hydrogen atom, an alkyl group, an alkoxy group, an aryl group, a sulfo group, a sulfamoyl group, a carboxy group, an alkoxycarbonyl group, an aryloxycarbonyl group, an acyloxy group, a carbamoyl group, an acylamino group, a nitro group, or a halogen atom.

R⁴¹ and R⁴² represent an alkyl group or -(CH₂-CH₂-O)_{b}-R²¹. R²¹ and b respectively have the same meanings as R²¹ and b described above. R⁴¹ and R⁴² may be bonded to each other to form a ring.

a is an integer of 1 to 3.

In a case where one hydrogen atom is removed from any of R¹ to R⁴, R¹¹ to R¹³, R²² to R²⁵, R³² to R³⁵, R⁴¹, or R⁴² described above, a monovalent structural moiety is provided.

Provided that the cyanine dye represented by Formula (α) is neutral.

Depending on the length of the methine chain having a repetition number of 2a + 3, which is connected by a conjugated double bond, the cyanine dyes represented by General Formula (α) respectively have an excitation absorption wavelength in a wavelength range of 520 to 600 nm (in the vicinity of 585 nm) in a case of a = 1, in a wavelength range of 620 to 700 nm (in the vicinity of 685 nm) in a case of a = 2, and in a wavelength range of 740 to 830 nm (in the vicinity of 785 nm) in a case of a = 3. As a result, each of the compounds according to the embodiment of the present invention which has, as the phosphor moiety M, a structural moiety consisting of a cyanine dye represented by General Formula (α) can be used as a compound exhibiting an excellent fluorescence intensity, in the fluorescence labeling in which a light source having any wavelength in a wavelength range of about 500 to 800 nm (for example, in the vicinity of 600 nm, in the vicinity of 700 nm, or in the vicinity of 800 nm) matching with the absorption excitation wavelength of the compound is used as an excitation light source.

In multicolor WB, a plurality of luminescence colors are detected in the range from the visible range to the near infrared range. As a result, it is necessary to select wavelengths so that the absorption and luminescence waveforms of a plurality of dyes have a suitable wavelength relationship so that crosstalk does not occur due to mutual interference in a case where the dyes are excited to emit light. Ideally, it should be adjusted so that only one dye emits light at one excitation light and the other dyes do not emit light. From this point of view, two types of excitation light sources having wavelengths separated to some extent, for example, in the vicinity of 700 nm and in the vicinity of 800 nm, are used for luminescence in the near infrared range of the multicolor WB.

As compared with the detection by visible light excitation, the fluorescence detection by near-infrared light excitation can suppress the autofluorescence of the membrane, that is, the background fluorescence, and thus it is easy to increase the signal to noise ratio (the S/N ratio) and it is possible to detect a target protein with high sensitivity. As a result, in recent years, there has been an increasing need for fluorescence detection WB using luminescence in the near infrared range in the analytical research on the trace amount of proteins.

However, in the near infrared range, the fluorescence quantum yield of the fluorescent dye is generally low, and thus it is difficult to obtain a high signal amount. Among the compounds according to the embodiment of the present invention which has, as the phosphor moiety M, a structural moiety consisting of a cyanine dye represented by General Formula (α), the compound in which a = 2 or 3 can be used as a compound that exhibits an excellent fluorescence intensity even in the multicolor WB having the above-described two kinds of excitation light sources in the vicinity of 700 nm and in the vicinity of 800 nm, and in particular, it can exhibit an excellent fluorescence intensity even with respect to a request for observing and detecting proteins with higher sensitivity, as compared with the fluorescence labeling using cyanine dyes in the related art.

### (i) R¹ to R⁴

R¹ to R⁴ represent an alkyl group or -(CH₂-CH₂-O)_{b}-R²¹,

The alkyl group which can be adopted as R¹ to R⁴ has the same meaning as the alkyl group in the substituent group T which will be described later.

The unsubstituted alkyl group preferably has 1 to 6 carbon atoms, more preferably has 1 to 4 carbon atoms, and still more preferably has 1 or 2 carbon atoms.

In a case where the alkyl group has a substituent, the alkyl group moiety of the alkyl group having a substituent preferably has 1 to 10 carbon atoms, more preferably has 1 to 8 carbon atoms, still more preferably has 2 to 6 carbon atoms, and particularly preferably has 2 to 5 carbon atoms. In addition, the number of atoms that constitute the longest chain of the alkyl group having a substituent is preferably 3 to 35, more preferably 3 to 25, still more preferably 3 to 15, and particularly preferably 3 to 11.

In the present invention, the "number of carbon atoms of the alkyl group moiety of the alkyl group having a substituent" means the number of carbon atoms excluding the substituent moiety contained in the alkyl group.

In the present invention, the "number of atoms that constitute the longest chain of the alkyl group having a substituent" means the number of atoms including the substituent moiety (that is, the number of atoms obtained by subtracting the number of atoms of the molecular chain that does not constitute the longest chain, from the number of total atoms). It is noted that in a case where a substituent having a dissociative hydrogen atom such as a sulfo group or a carboxy group constitutes the longest chain, the calculation is carried out including the hydrogen atom regardless of the presence or absence of dissociation. In addition, the number of atoms in the substituent moiety allowing binding to a biological substance described later is not included.

Examples of the substituent which may be contained in the alkyl group which can be adopted as R¹ to R⁴ include an alkoxy group, a carboxy group, an alkoxycarbonyl group, an acyloxy group, a carbonyl group, an acylamino group, a sulfo group, a phosphono group, and -(CH₂-CH₂-O)₈-R²¹, as well as a group consisting of a combination of these substituents.

The alkyl group having a substituent, which can be adopted as R¹ to R⁴, is not particularly limited as long as it is the above-described alkyl group having a substituent.

The alkyl group which can be adopted as R¹ to R⁴ is preferably an unsubstituted alkyl group.

### (-(CH₂-CH₂-O)_{b}-R²¹)

In -(CH₂-CH₂-O)_{b}-R²¹ which can be adopted as R¹ to R⁴, b is 1 to 50, and R²¹ represents an alkyl group.

b means an average repetition number (simply, also referred to as a repetition number), and it is preferably 1 to 24, more preferably 1 to 12, still more preferably 1 to 10, particularly preferably 4 to 10, and most preferably 4 to 8.

The average repetition number can be calculated from the average integrated value obtained by subjecting a compound to ¹H-NMR measurement. The average repetition number defined in the present invention means a number that is obtained by rounding off the first decimal place of the average repetition number calculated according to the above method.

To the alkyl group as R²¹, the description of the alkyl group which can be adopted as R¹ to R⁴ described above can be applied.

The -(CH₂-CH₂-O)_{b}-R²¹ which can be adopted as R¹ to R⁴ and -(CH₂-CH₂-O)_{b}-R²¹ which can be adopted as a substituent by an alkyl group as R¹ to R⁴ are preferably an alkyl group of -(CH₂-CH₂-O_{b}-unsubstituted.

From the viewpoint of further improving the fluorescence intensity of the fluorescent dye itself, it is preferable that at least one of R¹, ..., or R⁴ includes a structure represented by -(CH₂-CH₂-O)_{b}-, and it is more preferable at least one of R¹ or R² and at least one of R³ or R⁴ include a structure represented by -(CH₂-CH₂-O)_{b}-.

It is still more preferable that the entire phosphor moiety M in the compound according to the embodiment of the present invention is a structural moiety consisting of a cyanine dye represented by General Formula (α), where at least one of R¹ or R² and at least one of R³ or R⁴ includes a structure represented by -(CH₂-CH₂-O)_{b}-.

It is preferable that the structure represented by -(CH₂-CH₂-O)_{b}- is introduced by employing -(CH₂-CH₂-O)_{b} -R²¹ as R¹ to R⁴.

The b in -(CH₂-CH₂-O)_{b}- described above has the same meaning as the b in -(CH₂-CH₂-O)_{b}-R²¹ described above.

Since the substituents of R¹ to R⁴ protrude in a direction perpendicular to the cyanine dye skeleton (plane), it is presumed that in a case of including a structure represented by -(CH₂-CH₂-O)_{b}- as this substituent, the fused ring portion is difficult to undergo the π-π interaction (the effect of suppressing the association is strengthened), and thus the decrease in the fluorescence intensity due to the association can be suppressed.

### (ii) R¹¹ to R¹³

R¹¹ to R¹³ each independently represent a hydrogen atom, an alkyl group, an alkoxy group, an aryloxy group, an alkylthio group, an arylthio group, an amino group, or a halogen atom. Adjacent groups may be bonded to each other to form a 5- or 6-membered ring.

The alkyl group, the alkoxy group, the aryloxy group, the alkylthio group, the arylthio group, the amino group, and the halogen atom, which can be adopted as R¹¹ to R¹³, have the same meanings as the alkyl group, the alkoxy group, the aryloxy group, the alkylthio group, the arylthio group, the amino group, and the halogen atom in the substituent group T described later, and the same applies to the preferred ranges thereof.

Examples of the substituent which may be contained in the alkyl group, the alkoxy group, the aryloxy group, the alkylthio group, the arylthio group, and the amino group, as R¹¹ to R¹³, include the substituents in the substituent group T described below.

Among R¹¹ to R¹³, the 5- or 6-membered ring formed by bonding adjacent groups to each other may be either aromatic or aliphatic, and it is preferably aliphatic. In addition, it is preferable to form a 6-membered ring. The number of the above-described 5- or 6-membered rings in the compound is not particularly limited; however, it is preferably 1 or 2 and more preferably 1.

In a case of taking a case of a = 3 as an example, preferred examples of the structure having a ring formed by bonding adjacent groups among R¹¹ to R¹³ include the following structures. It is noted that in the following examples, R¹¹ to R¹³ that do not form a ring structure are a hydrogen atom, and the ring structure is described as a structure that does not have a substituent, which are not limited thereto. It is noted that, hereinafter, the structure beyond the wavy line will be omitted.

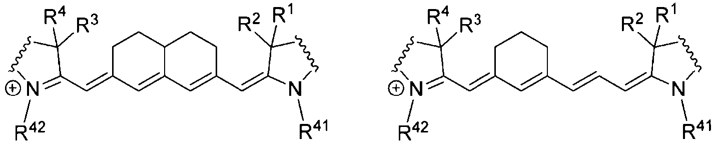

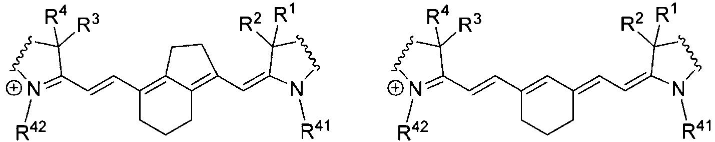

R¹¹ and R¹³ possessed by the carbon atom bonded to the indolenine ring are preferably a hydrogen atom.

R¹² and the remaining R¹³ are preferably a hydrogen atom or an alkyl group.

Among R¹¹ to R¹³, adjacent groups in R¹² and R¹³ other than R¹¹ and R¹³ possessed by the carbon atom bonded to the indolenine ring (that is, adjacent groups of R¹³ and R¹² other than R¹³ possessed by the carbon atom bonded to the indolenine ring) are preferably bonded to each other to form a 5- or 6-membered ring and more preferably to form a 6-membered ring. In addition, it is preferable that the 5- or 6-membered ring is formed at the central portion of the bond that connects the indoline ring and the indolenine ring. The ring formed in the central portion of the bond connecting the indoline ring and the indolenine ring means a ring containing carbon atoms as ring-constituting atoms so that the numbers of bonded atoms from the indoline ring and the indolenine ring are the same.

In a case where one hydrogen atom is removed from any of R¹ to R⁴, R¹¹ to R¹³, R²² to R²⁵, R³² to R³⁵, R⁴¹, or R⁴² described above, the phosphor moiety M is a monovalent structural moiety.

Specifically, a monovalent structural moiety is provided in a case where one hydrogen atom is removed from a substituent which can be adopted as R¹ to R⁴, R¹¹ to R¹³, R²² to R²⁵, R³² to R³⁵, R⁴¹, or R⁴², or a hydrogen atom which can be adopted as R¹¹ to R¹³, R²² to R²⁵, or R³² to R³⁵ is removed to provide a monovalent structural moiety which has a bonding site on the carbon atom to which R¹¹ to R¹³, R²² to R²⁵, or R³² to R³⁵ has been bonded.

Among the above, it is preferable that the phosphor moiety M is to be a monovalent structural moiety by removing one hydrogen atom from the ring formed by the bonding of R⁴¹ and R⁴² described above or is to be a monovalent structural moiety by removing one hydrogen atom from a substituent which can be adopted as R¹² (preferably R¹² on the carbon atom at which the numbers of bonded atoms from the indoline ring and the indolenine ring are the same).

### (iii) R²² to R²⁵ and R³² to R³⁵

R²² to R²⁵ and R³² to R³⁵ represent a hydrogen atom, an alkyl group, an alkoxy group, an aryl group, a sulfo group, a sulfamoyl group, a carboxy group, an alkoxycarbonyl group, an aryloxycarbonyl group, an acyloxy group, a carbamoyl group, an acylamino group, a nitro group, or a halogen atom. Regarding this R²² to R²⁵ and R³² to R³⁵, adjacent groups may be bonded to each other to form a fused ring.

The alkyl group, the alkoxy group, the aryl group, the sulfo group, the sulfamoyl group, the carboxy group, the alkoxycarbonyl group, the aryloxycarbonyl group, the acyloxy group, the carbamoyl group, the acylamino group, the nitro group, and the halogen atom, which can be adopted as R²² to R²⁵ and R³² to R³⁵, have the same meanings as the alkyl group, the alkoxy group, the aryl group, the sulfo group, the sulfamoyl group, the carboxy group, the alkoxycarbonyl group, the aryloxycarbonyl group, the acyloxy group, the carbamoyl group, the acylamino group, the nitro group, and the halogen atom in the substituent group T which will be described later.

The fused ring formed by bonding adjacent groups among R²² to R²⁵ and R³² to R³⁵ to each other is not particularly limited. However, examples thereof include a naphthalene ring (a naphthalene ring that is formed together with a benzene ring to which R²² to R²⁵ are bonded or a benzene ring to which R³² to R³⁵ are bonded in a case where adjacent groups are bonded to each other to form the benzene ring). From the viewpoint of suppressing association, it is preferable that adjacent groups among R²² to R²⁵ and R³² to R³⁵ are not bonded to each other and do not form a fused ring.

From the viewpoint of improving water solubility and suppressing association, it is preferable that at least one of R²², ..., or R²⁵ and at least one of R³², ..., or R³⁵ have a hydrophilic group, and it is more preferable that at least one hydrophilic group is contained per the number of rings of one, to which R²² to R²⁵ are bonded and rings to which R³² to R³⁵ are bonded. For example, in a case where adjacent groups among R²² to R²⁵ and R³² to R³⁵ are bonded to each other to form a naphthalene ring as a fused ring, the number of rings to which R²² to R²⁵ are bonded is two, and the number of rings to which R³² to R³⁵ are bonded to each other is two, which means that it is more preferable that at least two of R²² to R²⁵ and at least two of R³² to R³⁵ have a hydrophilic group. The upper limit value thereof is not particularly limited as long as it is allowed in terms of structure, and it can be appropriately adjusted in accordance with the number of hydrophilic groups in the compound as a whole, which will be described later.

The hydrophilic group is not particularly limited; however, examples thereof include an alkoxy group having a substituent, a carboxy group, a sulfo group, and a phosphono group, where a sulfo group is preferable.

R²² to R²⁵ and R³² to R³⁵ are preferably a hydrogen atom, an alkyl group, a sulfo group, a nitro group, or a halogen atom, more preferably a hydrogen atom, an alkyl group, a sulfo group, or a halogen atom, and still more preferably a hydrogen atom, an alkyl group, or a sulfo group.

### (iv) R⁴¹ and R⁴²

R⁴¹ and R⁴² represent an alkyl group or -(CH₂-CH₂-O)_{b}-R²¹. R²¹ and b respectively have the same meanings as R²¹ and b described above.

Examples of the substituent which may be contained in the alkyl groups as R⁴¹ and R⁴² include an alkoxy group, a carboxy group, an alkoxycarbonyl group, an acyloxy group, a carbamoyl group, an acylamino group, a sulfo group, and a phosphono group, as well as a group consisting of a combination of these substituents.

The alkyl group which can be adopted as R⁴¹ and R⁴² has the same meaning as the alkyl group in the substituent group T which will be described later.

The unsubstituted alkyl group preferably has 1 to 6 carbon atoms, more preferably has 1 to 4 carbon atoms, and still more preferably has 1 to 3 carbon atoms.

The alkyl group moiety of the alkyl group having a substituent preferably has 1 to 10 carbon atoms, more preferably has 1 to 8 carbon atoms, still more preferably has 1 to 7 carbon atoms, even still more preferably has 1 to 6 carbon atoms, and even further still more preferably has 1 to 5 carbon atoms. In addition, the number of atoms that constitute the longest chain of the alkyl group having a substituent is preferably 3 to 14, more preferably 3 to 12, and still more preferably 3 to 10.

The alkyl group having a substituent, which can be adopted as R⁴¹ and R⁴², is preferably an alkyl group having, as a substituent, at least one of an alkoxy group, a carboxy group, a sulfo group, or a phosphono group, and more preferably an alkyl group having, as a substituent, at least one of a carboxy group or a sulfo group, from the viewpoint of further improving water solubility. It is noted that it may be an alkyl group having a substituent consisting of a combination of the above-described preferred substituents (the alkoxy group, the carboxy group, the sulfo group, and the phosphono group) and a group other than these substituents.

In addition, the form of the alkyl group having a substituent, which can be adopted by R¹ to R⁴, can be also preferably applied.

To -(CH₂-CH₂-O)_{b}-R²¹ which can be adopted as R⁴¹ and R⁴², the description of -(CH₂-CH₂-O)_{b}-R²¹ in R¹ to R⁴ can be preferably applied.

R⁴¹ and R⁴² may be bonded to each other to form a ring.

Among the cyanine dyes represented by General Formula (α), preferred examples of the structure in which R⁴¹ and R⁴² are bonded to each other to form a ring include a cyanine dye represented by General Formula (β).

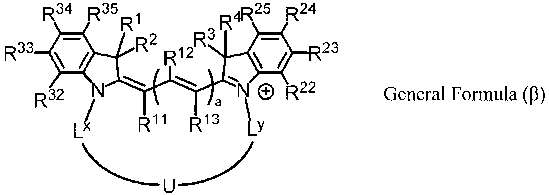

In the formula, L^{x} to L^{y} represent an alkylene group or -(CH₂-CH₂-O)_{b}-alkylene-*. * represents a bonding position to U.

The linking group U represents a divalent linking group having 1 to 100 atoms.

R¹ to R⁴, R¹¹ to R¹³, R²² to R²⁵, R³² to R³⁵, b, and a respectively have the same meanings as R¹ to R⁴, R¹¹ to R¹³, R²² to R²⁵, R³² to R³⁵, b, and a in General Formula (α), and the same also applies to the preferred ranges thereof unless otherwise specified.

At least one of R¹, R², R³, R⁴, L^{x}, or U includes a structure represented by-(CH₂-CH₂-O)_{b}-. m has the same meaning as m described above.

Provided that the cyanine dye represented by Formula (β) is neutral.

The alkylene group which can be adopted as L^{x} and L^{y} corresponds to an alkylene group obtained by removing one hydrogen atom or one substituent from an alkyl group having a substituent which can be adopted as R⁴¹ and R⁴².

For the number of carbon atoms of the alkylene group moiety of the alkylene group which can be adopted as L^{x} to L^{y}, the description of the number of carbon atoms of the alkyl group moiety of the alkyl group having a substituent, as R⁴¹ and R⁴², can be preferably applied.

The -(CH₂-CH₂-O)_{b}-alkylene-* which can be adopted as L^{x} and L^{y} corresponds to -(CH₂-CH₂-O)_{b}-alkylene- obtained by removing one hydrogen atom or one substituent from the alkyl group as R²¹, among the -(CH₂-CH₂-O)_{b}-R²¹ which can be adopted as R⁴¹ and R⁴² (R²¹ represents an alkyl group having a substituent).

In the -(CH₂-CH₂-O)_{b}-alkylene-* which can be adopted as L^{x} and L^{y}, b is preferably 1 to 10 and more preferably 1 to 8, and as the number of carbon atoms of the alkylene group moiety, the description of the number of carbon atoms of the alkyl group moiety of the alkyl group having a substituent in R⁴¹ to R⁴² can be preferably applied.

From the viewpoint of further improving the fluorescence intensity, it is preferable that both L^{x} and L^{y} include a structure represented by -(CH₂-CH₂-O)_{b}-.

The total number of atoms constituting the linking group U is 1 to 100, and it is preferably 10 to 90, more preferably 20 to 90, and still more preferably 30 to 80.

The linking group U is preferably a divalent linking group formed by bonding three or more selected from an alkylene group, -O-, -NR⁵⁰-, -COO-, -CONR⁵⁰-, and -SO₂NR⁵⁰-R⁵⁰ represents a hydrogen atom or an alkyl group.

The number of carbon atoms in the alkylene moiety of the alkylene group which can be adopted as the linking group U is preferably 1 to 10, more preferably 1 to 8, still more preferably 1 to 7, particularly preferably 1 to 6, and most preferably 1 to 5.

In the present invention, "the number of carbon atoms in the alkylene moiety of the alkylene group" means the number of carbon atoms excluding the substituent moiety contained in the alkylene group.

As the alkyl group which can be adopted as R⁵⁰, the description of the alkyl group as R¹ to R⁴ can be preferably applied.

R⁵⁰ is preferably a hydrogen atom.

The number of the above-described alkylene group, -O-, -NR⁵⁰-, -COO-, -CONR⁵⁰-, and -SO₂NR⁵⁰-, constituting the linking group U, is preferably 3 to 11, more preferably 3 to 7, still more preferably 3 to 5, and particularly preferably 3.

In the linking group U, the connecting portion to L^{x} to L^{y} is preferably -O-, -NR⁵⁰-, -COO-, -CONR⁵⁰-, or -SO₂NR⁵⁰-. That is, the linking group U is preferably bonded to the alkylene groups of L^{x} to L^{y} through -O-, -NR⁵⁰-, -COO-, -CONR⁵⁰-, or -SO₂NR⁵⁰-, which constitutes the linking group U. In the linking group U, it is more preferable that connecting portions to L^{x} to L^{y} are -O-, -NR⁵⁰-, -COO-, -CONR⁵⁰-, or -SO₂NR⁵⁰-, where the linking group U is a divalent linking group in which the connecting portions are connected to each other through an alkylene group.

It is preferable that the linking group U is to be a monovalent structural moiety, that is, a phosphor moiety M, by removing one hydrogen atom therefrom. In the linking group U, examples of the position where one hydrogen atom is removed include an alkylene group and an alkyl group as R⁵⁰, where an alkylene group is preferable.

In a case where in the linking group U, one hydrogen atom is removed in the alkylene group or the alkyl group as R⁵⁰, one hydrogen atom may be directly removed from the alkylene group or the alkyl group as R⁵⁰, and the linking group ZZZ may be bonded to the alkylene group or the alkyl group as R⁵⁰, thereby the linking group ZZZ serving as a bonding site.

Examples of the linking group ZZZ include an alkylene group, an alkenylene group, an alkynylene group, an arylene group, a heteroarylene group, -O-, -S-, >C=O, >NR⁶⁰, >S=O, >S(=O)₂, >P(=O)OR⁷⁰, -COO-, -CONR⁶⁰-, and -(CH₂-CH₂-O)ₚ-, as well as a group consisting of a combination of these substituents. The number of those to be combined is not particularly limited; however, it can be set to, for example, 2 to 20, and it is preferably 2 to 7 and more preferably 2 to 5.

R⁶⁰ and R⁷⁰ are a hydrogen atom or an alkyl group and are preferably a hydrogen atom. To the alkyl group which can be adopted as R⁶⁰ or R⁷⁰, the description of the alkyl group as R⁵⁰ can be preferably applied.

p represents the repetition number, and it is preferably 1 to 10, more preferably 1 to 8, and still more preferably 1 to 4.

### (v) a

a is an integer of 1 to 3, where it is preferably an integer of 2 or 3.

In the cyanine dye represented by General Formula (α), it is preferable that at least one of R¹, R², R³, R⁴, R⁴¹, or R⁴² includes a structure represented by -(CH₂-CH₂-O)_{b}-. b has the same meaning as b described above. It is conceived that this makes it possible for the compound according to the embodiment of the present invention, which has a structural moiety consisting of a cyanine dye represented by General Formula (α), to have a proper hydrophilicity and a proper excluded volume effect, whereby a labeled biological substance to be obtained can exhibit an excellent fluorescence intensity.

In addition, from the viewpoint that sufficient hydrophilicity is imparted as the compound according to the embodiment of the present invention, the cyanine dye represented by General Formula (α) is such that the number of hydrophilic groups per one molecule of the cyanine dye represented by General Formula (α) is preferably 2 or more, more preferably 2 to 8, still more preferably 2 to 6, and particularly preferably 3 to 6.

To the hydrophilic group, the description of the hydrophilic group which can be adopted by R²² to R²⁵ and R³² to R³⁵ described above can be applied.

The position of the hydrophilic group is not particularly limited unless specified otherwise, and examples of the group having the hydrophilic group preferably include R¹¹ to R¹³, R²² to R²⁵, R³² to R³⁵, R⁴¹, or R⁴².

It is noted that in the structure represented by General Formula (α) or (β), one hydrogen atom may be removed from any substituent to provide a monovalent structural moiety (the phosphor moiety M); however, it is preferable that, for example, one hydrogen atom is removed from the linking group U to provide a monovalent structural moiety, or one hydrogen atom is removed from a substituent, which can be adopted as R¹² (preferably R¹² on the carbon atom at which the numbers of bonded atoms from the indoline ring and the indolenine ring are the same), to provide a monovalent structural moiety.

Unless otherwise specified, the description of the substituent in General Formulae (α) and (β) is a description of a substituent that is applied only to General Formulae (α) and (β).

In addition, as the phosphor moiety M, a structural moiety consisting of a commercially available fluorescent dye can also be used without particular limitation. Examples of such commercially available fluorescent dye include Alexa Fluor 350, Alexa Fluor 405, Alexa Fluor 430, Alexa Fluor 488, Alexa Fluor 514, Alexa Fluor 532, Alexa Fluor 546, Alexa Fluor 555, Alexa Fluor 568, Alexa Fluor 594, Alexa Fluor 633, Alexa Fluor 635, Alexa Fluor 647, Alexa Fluor 660, Alexa Fluor 680, Alexa Fluor 700, Alexa Fluor 750, Alexa Fluor 790, DyLight 350, DyLight 405, DyLight 425Q, DyLight 488, DyLight 510-LS, DyLight 515-LS, DyLight 550, DyLight 594, DyLight 633, DyLight 650, DyLight 680, DyLight 730-B1, DyLight 747-B4, DyLight 800, DyLight 650-4xPEG, DyLight 800-4xPEG, and the like, all of which are product names of products manufactured by Thermo Fisher Scientific Inc.; ATTO 390, ATTO 425, ATTO 465, ATTO 488, ATTO 495, ATTO 514, ATTO 520, ATTO 532, ATTO RHO6G, ATTO 540Q, ATTO 550, ATTO 565, ATTO RHO11, ATTO 580Q, ATTO 590, ATTO 594, ATTO 610, ATTO 612Q, ATTO 620, ATTO 633, ATTO RHO14, ATTO 647, ATTO 647N, ATTO 655, ATTO 665, ATTO 680, ATTO 700, ATTO 725, ATTO 740, and the like, all of which are product names of products manufactured by ATTO-TEC GmbH; Cy3, Cy3B, Cy5, Cy5.5, Cy7, and the like, all of which are product names of products manufactured by Global Life Science Technologies Japan, Inc.; and CF 350, CF 405S, CF 405M, CF 405L, CF 430, CF 440, CF 450, CF 488A, CF 503R, CF 514, CF 532, CF 535ST, CF 543, CF 550R, CF 555, CF 568, CF 570, CF 583, CF 583R, CF 594, CF 594ST, CF 597R, CF 620R, CF 633, CF 640R, CF 647, CF 660C, CF 660R, CF 680, CF 680R, CF 700, CF 750, CF 770, CF 790, CF 800, CF 820, CF 850, CF 870, and the like, all of which are product names of products manufactured by Biotium, Inc. In addition, a fluorescent dye subjected to activation such as NHS esterization can also be preferably used such that these commercially available fluorescent dyes can be introduced (can be bonded by a chemical reaction) as the phosphor moiety M in the compound according to the embodiment of the present invention.

The physiologically active substance moiety which can be adopted as M can be used without particular limitation as long as it is a structural moiety consisting of a physiologically active substance. Examples of the physiologically active substance include a vitamin, a coenzyme, a hormone, an antibiotic, a neurotransmitter, and a cytokine. More specific examples thereof are calicheamicin, doxorubicin, daunorubicin, mitomycin C, bleomycin, cyclocytidine, vincristine, vinblastine, methotrexate, cisplatin or a derivative thereof, auristatin or a derivative thereof, maytansine or a derivative thereof, taxol or a derivative thereof, and camptothecin or a derivative thereof, which are described in paragraph [0095] of JP2021-020956A, and the description of paragraphs [0095] to [0099] of JP2021-020956A can be applied thereto.

The prodrug moiety which can be adopted as M can be used without particular limitation as long as it is a structural moiety consisting of a compound that is metabolized in vivo to be changed to a physiologically active substance. To the prodrug, for example, the description (a prodrug form of 2-pyrrolinodoxorubicin) in paragraph [0003] of JP2020-105187A can be applied.

The radioactive isotope-containing moiety which can be adopted as M can be used without particular limitation as long as it is a structural moiety containing a radioactive isotope that is capable of being used in the medical field. Examples of the radioactive isotope include iodine 131, indium 111, yttrium 90, lutetium 177, and copper 64, which are not limited thereto. The description of paragraph [0225] of JP2021-11483A can be applied thereto. Examples of the structural moiety containing a radioactive isotope include a structural moiety in which the radioactive isotope is bonded or coordinated to a nitrogen atom of an amino group or a tertiary amine, a sulfanyl group, an aryl group, a heteroaryl group, or the like. Examples of the structural moiety in which a nitrogen atom of a tertiary amine is coordinated to the radioactive isotope) include a structural moiety in which 1,4,7,10-tetraazacyclododecane-1,4,7,10-tetraacetic acid (DOTA) or the like is coordinated to the radioactive isotope to form a complex, and examples of the structural moiety in which a sulfanyl group is coordinated to the radioactive isotope include a structural moiety consisting of a complex such as diacetyl bis(N(4)-methylthiosemicarbazonato)copper (II).

### <Compound represented by General Formula (III)>

The compound represented by General Formula (II) is preferably represented by General Formula (III). The compound represented by General Formula (III) corresponds to a compound in which L¹ and L⁴ represent >NH, L³ represents >C=O, L⁶ represents a single bond or >C=O, L⁷ represents a single bond, and L¹ and L², and L⁴ and L⁵ are respectively bonded to each other to form a specific 5-membered ring in the compound represented by General Formula (II).

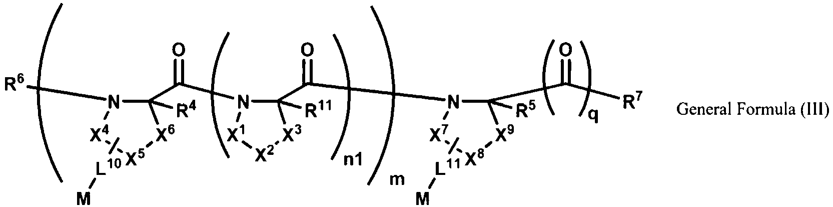

In the formula, X⁴ to X⁹ represent -O-, -S-, >NR¹⁰¹, or >CR¹⁰²R¹⁰³,
provided that at least one of X⁴, X⁵, or X⁶ represents >N-L¹⁰-M or >C(R¹⁰²)-L¹⁰-M, and at least one of X⁷, X⁸, or X⁹ is >N-L¹¹-M or >C(R¹⁰²)-L¹¹-M,
R¹⁰¹ to R¹⁰³ which are neither -L¹⁰-M nor -L¹¹-M represent a hydrogen atom, an alkyl group, an alkenyl group, an alkynyl group, an aryl group, a heteroaryl group, an acyl group, -NR⁸R⁹, -OR¹⁰, an anionic group, a betaine residue, a polyol residue, a sugar residue, a polyamino acid residue, -(O-L)_{g}R^{E}, or -(L-O)_{g}R^{E},
L¹⁰ and L¹¹ represent a single bond or a divalent linking group,
n1 represents an integer of 2 or more, and q represents an integer of 0 or 1, and
R⁴ to R¹¹, X¹ to X³, M, L, R^{E}, g, and m have the same meanings as R⁴ to R¹¹, X¹ to X³, M, L, R^{E}, g, and m in General Formula (II).

In X⁴ to X⁶, the description of X¹ to X³ in General Formula (I) can be applied except that at least one of X⁴, ..., or X⁶ is >N-L¹⁰-M or >C(R¹⁰²)-L¹⁰-M, unless otherwise specified.

In addition, in X⁷ to X⁹, the description of X¹ to X³ in General Formula (I) can be applied except that at least one of X⁷, ..., or X⁹ is >N-L¹¹-M or >C(R¹⁰²)-L¹¹-M, unless otherwise specified.

That is, the description of X¹ described above is applied to X⁴ and X⁷, the description of X² described above is applied to X⁵ and X⁸, and the description of X³ described above is applied to X⁶ and X⁹, and the descriptions of R¹, R², and R³ in General Formula (I) described above can be respectively applied to R¹⁰¹, R¹⁰², and R¹⁰³ which are neither -L¹⁰-M nor -L¹¹-M.

In >NR¹⁰¹ and >CR¹⁰²R¹⁰³ among X⁴ to X⁹, which have neither -L¹⁰-M nor -L¹¹-M, R¹⁰¹ is preferably an alkyl group, and R¹⁰² and R¹⁰³ are preferably a hydrogen atom.

Regarding the two groups among X⁴ to X⁶, which do not have -L¹⁰-M described above, it is preferable that at least one thereof is >CR¹⁰²R¹⁰³, it is more preferable that at least one thereof is >CR¹⁰²R¹⁰³ and the remaining one is -O-, -S-, or >CR¹⁰²R¹⁰³, and it is still more preferable that both the two are >CR¹⁰²R¹⁰³.

Regarding the two groups among X⁷ to X⁹, which do not have -L¹¹-M described above, it is preferable that at least one thereof is >CR¹⁰²R¹⁰³, it is more preferable that at least one thereof is >CR¹⁰²R¹⁰³ and the remaining one is -O-, -S-, or >CR¹⁰²R¹⁰³, and it is still more preferable that both the two are >CR¹⁰²R¹⁰³.

Among X⁴ to X⁶, as >N-L¹⁰-M or >C(R¹⁰²)-L¹⁰-M, >C(R¹⁰²)-L¹⁰-M is preferable and >CH-L¹⁰-M is more preferable.

Among X⁷ to X⁹, as >N-L¹¹-M or >C(R¹⁰²)-L¹¹-M, >C(R¹⁰²)-L¹¹-M is preferable and >CH-L¹¹-M is more preferable.

Among X⁴ to X⁶, the group having -L¹⁰-M is not particularly limited; however, it is preferably X⁵.

Among X⁷ to X⁹, the group having -L¹¹-M is not particularly limited; however, it is preferably X⁸.

L¹⁰ and L¹¹ are preferably a single bond, or a group obtained by combining one or two or more of an alkylene group, an alkenylene group, an alkynylene group, an arylene group, a heteroarylene group, -O-, -S-, >C=O, and >NR^{A}, more preferably a group obtained by combining one or two or more of an alkylene group, an alkenylene group, an alkynylene group, an arylene group, a heteroarylene group, -O-, -S-, >C=O, and >NR^{A}, and still more preferably a group represented by * -L^{x1}-L^{y1}-**.

To the alkylene group, the alkenylene group, the alkynylene group, the arylene group, the heteroarylene group, and >NR^{A}, which can constitute L¹⁰ and L¹¹, the description of the alkylene group, the alkenylene group, the alkynylene group, the arylene group, the heteroarylene group, and >NR^{A}, which can constitute L² and L⁵ described above, can be applied unless otherwise specified.

L^{x1} is a single bond or a group obtained by combining one or two or more of an alkylene group, an alkenylene group, an alkynylene group, an arylene group, and a heteroarylene group, and L^{y1} is a single bond, -O-, -S-, >C=O, or >NR^{A}. It is noted that in *-L^{x1}-L^{y1}-**, * represents a bonding site to X⁴ to X⁹, and ** represents a bonding site to M.

Among the groups represented by * -L^{x1}-L^{y1}-**, L¹⁰ and L¹¹ are preferably a group in which L^{y1} is a single bond, -S-, >C=O, or >NR^{A}, more preferably a group in which L^{y1} is >C=O or >NR^{A}, and still more preferably a group in which L^{x1} is a single bond and L^{y1} is >C=O or >NR^{A}.

In the present invention, from the viewpoint of making the main chain of the linking group that links the phosphor moieties adjacent to each other more rigid (making the Molecular Flexibility smaller), among the groups constituting L¹⁰ and L¹¹, a group directly bonded to a carbon atom or a nitrogen atom constituting X⁴ to X⁹ is preferably an alkenylene group, an alkynylene group, an arylene group, a heteroarylene group, -O-, -S-, >C=O, or >NR^{A}, and more preferably an alkynylene group, an arylene group, a heteroarylene group, >C=O, or >NR^{A}.

It is noted that in the compound represented by General Formula (III), it suffices that the number of shortest-distance atoms in the linking chain (each linking chain of the linking chain including L¹⁰ and the linking chain including L¹¹) that connects M to any one of X⁴ to X⁹ is, for example, 1 to 60, where 1 to 40 is preferable. In a case where M is a phosphor moiety, the above-described number of shortest-distance atoms means the number of atoms that constitute the shortest chain in the linking chain that connects a conjugated structural moiety for exhibiting fluorescence to any one of X⁴ to X⁹ in the phosphor moiety M.

It is noted that in the compound represented by General Formula (III), it is also preferable that the structure represented by -(CH₂-CH₂-O)_{b}- described above (b is also as described above) is provided at any portion of the linking chain represented by "-linking group ZZZ-L¹⁰-" in the structure represented by "the conjugated structural moiety of M - the linking group ZZZ-L¹⁰- described above" and any portion of the linking chain represented by "-linking group ZZZ-L¹¹-" in the structure represented by "the conjugated structural moiety of M - the linking group ZZZ-L¹¹- described above".

n1 is an integer of 2 or more.

In the compound represented by General Formula (III), the linker main chain that connects two phosphor moieties is more rigid, and the dye association can be further suppressed, as compared with a compound in which L¹ and L², and L⁴ and L⁵ in General Formula (II) are each bonded to each other to form a specific 5-membered ring. Therefore, the lower limit value of n1 is preferably an integer of 3 or more, more preferably an integer of 6 or more, still more preferably an integer of 9 or more, and particularly preferably an integer of 12 or more. The same applies even in a case where the number of phosphor moieties is two or more and a case where the number thereof is 2 or more.

The upper limit value of n1 is, for example, preferably an integer of 36 or less, more preferably an integer of 24 or less, and still more preferably an integer of 18 or less. That is, n1 is preferably an integer of 2 to 36, more preferably an integer of 3 to 36, still more preferably an integer of 6 to 24, particularly preferably an integer of 9 to 24, and among these, preferably an integer of 12 to 18.

q is an integer of 0 or 1, and is preferably 0.

### <Compound represented by General Formula (IV)>

The compound represented by General Formula (III) is preferably represented by General Formula (IV).

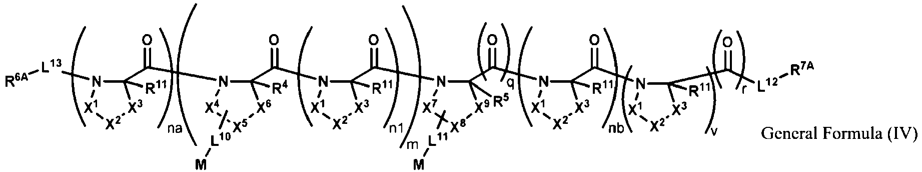

In the formula, R^{6A} and R^{7A} represent a hydrogen atom, a hydroxy group, a sulfanyl group, an alkyl group, an alkenyl group, an alkynyl group, an aryl group, an alkoxy group, a heteroaryl group, an amino group, an acyl group, or Q, provided that at least one of R^{6A} or R^{7A} represents a carboxy group, a substituent allowing binding to a biological substance, or a substituent allowing binding to a solid support,
L¹² and L¹³ represent a single bond or a linking group,
na and nb represent an integer of 0 or more, and r and v represent an integer of 0 or 1, and
R⁴, R⁵, R¹¹, L¹⁰, L¹¹, X¹ to X⁹, M, Q, q, n1, and m have the same meanings as R⁴, R⁵, R¹¹, L¹⁰, L¹¹, X¹ to X⁹, M, Q, q, n1, and m in General Formula (III) described above.

R^{6A} or R^{7A} and L¹² or L¹³ are each determined such that R^{6A} or R^{7A} is an unsubstituted group and L¹² or L¹³ is the longest group. Provided that in a case where the group represented by -L¹³R^{6A} or -L¹²R^{7A} has Q, it is determined such that Q which is located on the most terminal end side (the R^{6A} side in -L¹³R^{6A} or the R^{7A} side in -L¹²R^{7A}), is R^{6A} or R^{7A}.

R^{6A} and R^{7A} represent a hydrogen atom, a hydroxy group, a sulfanyl group, an alkyl group, an alkenyl group, an alkynyl group, an aryl group, an alkoxy group, a heteroaryl group, an amino group, an acyl group, or Q. Provided that at least one of R^{6A} or R^{7A} represents a carboxy group, a substituent allowing binding to a biological substance, or a substituent allowing binding to a solid support.

The alkyl group, the alkenyl group, the alkynyl group, the aryl group, the alkoxy group, the heteroaryl group, the amino group, and the acyl group, which can be adopted as R^{6A} or R^{7A}, have the same meanings as the alkyl group, the alkenyl group, the alkynyl group, the aryl group, the alkoxy group, the heteroaryl group, the amino group, and the acyl group in the substituent group T which will be described later, and the same also applies to the preferred ranges thereof. Provided that all the groups are unsubstituted groups.

Q which can be adopted as R^{6A} and R^{7A} has the same meaning as Q described above, and the same applies to the preferred range thereof.

R^{6A} is preferably an alkyl group, a sulfanyl group, an aryl group, a heteroaryl group, or Q, more preferably an alkyl group or Q, and still more preferably an alkyl group, a carboxy group, a substituent allowing binding to a biological substance, or a substituent allowing binding to a solid support.

R^{7A} is preferably an alkyl group, a sulfanyl group, an aryl group, a heteroaryl group, or Q, more preferably an alkyl group or Q, and still more preferably an alkyl group, a carboxy group, a substituent allowing binding to a biological substance, or a substituent allowing binding to a solid support.

L¹² and L¹³ represent a single bond or a linking group.

The linking group which can be adopted as L¹² and L¹³ is, for example, more preferably a divalent linking group obtained by combining one or two or more of an alkylene group, an alkenylene group, an alkynylene group, an arylene group, a heteroarylene group, -O-, >C=O, and >NR^{A}. R^{A} represents a hydrogen atom or a substituent.

For the alkylene group, the alkenylene group, the alkynylene group, the arylene group, the heteroarylene group, and >NR^{A}, which can constitute L¹² and L¹³, the description of the alkylene group, the alkenylene group, the alkynylene group, the arylene group, the heteroarylene group, and >NR^{A}, which can constitute L¹ to L⁷ described above, can be preferably applied.

The substituent which may be contained in the alkylene group, the alkenylene group, the alkynylene group, the arylene group, and the heteroarylene group, which can constitute L¹² and L¹³, is not particularly limited, and it is preferably selected from a substituent group T which will be described later. More preferred examples thereof include a halogen atom, an aryl group, and an alkyl group. In addition, the substituent which may be contained in the alkylene group, the alkenylene group, the alkynylene group, the arylene group, and the heteroarylene group, which can constitute L¹² or L¹³, may be substituted with a substituent selected from the substituent group T which will be described later, and it is, for example, preferably a carboxy group, a sulfo group, a phosphono group, a cationic group, a betaine residue, a polyol residue, a sugar residue, a polyamino acid residue, -(O-L)_{g}R^{E} or -(L-O)_{g}R^{E}.

In addition, the number of substituents which may be contained in the alkylene group, the alkenylene group, the alkynylene group, the arylene group, and the heteroarylene group, which can constitute L¹² and L¹³, is not particularly limited as long as it can be adopted as a structure. The number thereof can be set to at least one or more, the upper limit thereof is not particularly limited, and for example, all hydrogen atoms in the alkylene group, the alkenylene group, the alkynylene group, the arylene group, and the heteroarylene group may be substituted with a substituent.

In the compound represented by General Formula (IV), it is preferable that (A) na is an integer of 0 or more (preferably an integer of 1 or more) and R^{6A} is a carboxy group, a substituent allowing binding to a biological substance, or a substituent allowing binding to a solid support, and/or (B) the sum of nb and v is an integer of 0 or more (preferably an integer of 1 or more) and R^{7A} is a carboxy group, a substituent allowing binding to a biological substance, or a substituent allowing binding to a solid support.

Provided that the number of shortest linking atoms of L¹³ is 7 or less in a case of the (A), and the number of shortest linking atoms of L¹² is 7 or less in a case of the (B).

Regarding the divalent linking group L¹³, "the number of shortest linking atoms in L¹³" means the number of atoms that constitute the shortest chain connecting N directly bonded to L¹³ to R^{6A}, in a case where na is an integer of 1 or more, where the N is one of those indicated in the structure parenthesized in ( )ₙₐ, and it means the number of atoms that constitute the shortest chain connecting N directly bonded to L¹³ to R^{6A}, in a case where na is 0, where the N is one of those indicated in the structure parenthesized in ( )ₘ.

In addition, regarding the divalent linking group L¹², the "number of shortest linking atoms in L¹²" means the number of atoms constituting the shortest chain connecting R^{7A} and an atom to which L¹² positioned on the left side of L¹² on the paper surface is bonded, similarly to the "number of shortest linking atoms in L¹³" described above.

For example, in a compound (1) that is used in Examples described later, since L¹² is -NHC₂H₄- and R^{7A} is -COOH, the number of shortest linking atoms of L¹² is 3.

In the compound represented by General Formula (IV), at least one of R^{6A} or R^{7A} is a carboxy group, a substituent allowing binding to a biological substance, or a substituent allowing binding to a solid support. In particular, it is conceived that in a case of satisfying (A) and/or (B) described above, the mobility of the linker main chain is reduced, and thus the dye association can be further suppressed.

The number of shortest linking atoms of L¹² and the number of shortest linking atoms of L¹³, which are described, are preferably 1 to 5 and more preferably 1 to 4.

In the present invention, from the viewpoint of ease of synthesis, it is also preferable that any one of L¹² or L¹³ is a group including -(L-O)_{g}-, and it is also more preferable that L¹² is a group including -(L-O)_{g}-.

L¹² is more preferably a single bond or a linking group obtained by combining one or two or more of an alkylene group, -O-, >C=O, and >NR^{A}, more preferably a single bond, an -NR^{A}-alkylene group or an -NR^{A}-(L-O)_{g}-alkylene group, and still more preferably a single bond or an -NR^{A}-alkylene group.

L¹³ is more preferably a single bond or a linking group obtained by combining one or two or more of an alkylene group, -O-, >C=O, and >NR^{A}, and still more preferably a single bond, >NR^{A} or >C=O.

na and nb represent an integer of 0 or more. r and v are integers of 0 or 1.

In a case where R^{6A} is a carboxy group, a substituent allowing binding to a biological substance, or a substituent allowing binding to a solid support, na is preferably an integer of 0 to 40, more preferably an integer of 0 to 20, and still more preferably an integer of 0 to 15.

In a case where R^{7A} is a carboxy group, a substituent allowing binding to a biological substance, or a substituent allowing binding to a solid support, the sum of nb and v is preferably an integer of 0 to 40, more preferably an integer of 0 to 20, and still more preferably an integer of 0 to 15.

In a case where R^{6A} is not a carboxy group, a substituent allowing binding to a biological substance, or a substituent allowing binding to a solid support, na is preferably an integer of 0 to 20, more preferably an integer of 0 to 10, and still more preferably an integer of 0 to 5.

In a case where R^{7A} is not a carboxy group, a substituent allowing binding to a biological substance, or a substituent allowing binding to a solid support, the sum of nb and v is preferably an integer of 0 to 20, more preferably an integer of 0 to 10, and still more preferably an integer of 0 to 5.

r is preferably 0, and v is preferably 0.

In a case where a compound represented by any one of General Formulae (II) to (IV) among the compounds according to the embodiment of the present invention is obtained by using a peptide synthesis method, the structure is, in general, such that the C-terminal structure is on the right side of the paper surface, and the N-terminal structure is on the left side of the paper surface.

The compound according to the embodiment of the present invention preferably contains at least one of a carboxy group, a substituent allowing binding to a biological substance, or a substituent allowing binding to a solid support, which are substituent represented by Q.

The compound according to the embodiment of the present invention can be bonded to a biological substance by the carboxy group or a substituent allowing binding to a biological substance described later, whereby a targeted labeled biological substance can be obtained. It is noted that a substituent allowing binding to a biological substance can be easily derived from a carboxy group by a conventional method.

In addition, the compound according to the embodiment of the present invention can be bonded to a solid support such as microparticles by the carboxy group or a substituent allowing binding to a solid support described later, whereby a targeted labeled microparticles can be obtained. The microparticle is not particularly limited; however, examples thereof include small particles that are useful for bonding to the compound according to the embodiment of the present invention, including a glass bead, a non-polymer bead such as a magnetic bead, and a polymer bead. In a certain embodiment, the microparticle includes a polystyrene bead. The small particle is not particularly limited as long as it has a size that is commonly used in the fluorescence labeling; however, the average particle diameter thereof is generally 10 nm to 10 µm. It is noted that a substituent allowing binding to a solid support can be easily derived from a carboxy group by a conventional method.

In the present invention, for convenience, the substituent allowing binding to a biological substance and the substituent allowing binding to a solid support do not include a carboxy group, where "the substituent allowing binding to a biological substance" includes a substituent allowing binding to a biological substance, which is derived from a carboxy group, and "the substituent allowing binding to a solid support" includes a substituent allowing binding to a solid support, which is derived from a carboxy group. Provided that as described above, it is also possible to carry out bonding to a biological substance or a solid support by a carboxy group.

In the compound according to the embodiment of the present invention, a position where a carboxy group, a substituent allowing binding to a biological substance, or a substituent allowing binding to a solid support is provided is not particularly limited; however, it is preferably provided in a structure other than the structure represented by General Formula (I) and the phosphor moiety and more preferably provided in at least one of R⁶ or R⁷ in the compound represented by General Formula (II).

It suffices that the number of the carboxy groups, substituents allowing binding to a biological substance, or substituents allowing binding to a solid support in the compound according to the embodiment of the present invention is at least 1 or more in total, and it is preferably 1 to 3, more preferably 1 or 2, and still more preferably 1, from the viewpoint of the quantification of the target substance to be detected.

In addition, from the viewpoint of imparting sufficient hydrophilicity as a compound, the compound according to the embodiment of the present invention also preferably has an anionic group, a betaine residue, a polyol residue, a sugar residue, a polyamino acid residue, -(O-L)_{g}R^{E} or -(L-O)_{g}R^{E}, which will be described later at a position other than the phosphor moiety, and for example, it preferably has one or more anionic groups, more preferably 1 to 8 anionic groups, and still more preferably 1 to 6 anionic groups.

The position of the anionic group, the betaine residue, the polyol residue, the sugar residue, the polyamino acid residue, -(O-L)_{g}R^{E}, or -(L-O)_{g}R^{E} is not particularly limited unless otherwise specified, and preferred examples of the group having the anionic group, the betaine residue, the polyol residue, the sugar residue, the polyamino acid residue, -(O-L)_{g}R^{E}, or -(L-O)_{g}R^{E} in the compound represented by General Formula (III) include X¹ to X³ or R¹¹.

Specific examples of the compound according to the embodiment of the present invention will be shown below; however, the present invention is not limited to these compounds. In the following specific examples, the sulfo group may adopt a salt structure in which a hydrogen ion is dissociated. In a case of having a salt structure, examples of the monovalent cation and the polyvalent cation, which can be adopted as X⁺, include the alkali metal cation, alkaline earth metal cation, organic ammonium cation, organic phosphonium cation, and the like, which are described above.

In the following specific examples, Dye indicates a phosphor moiety.

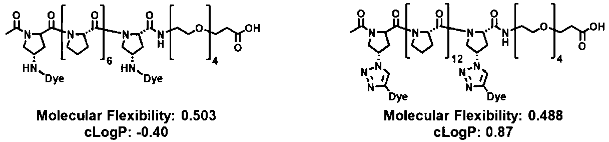

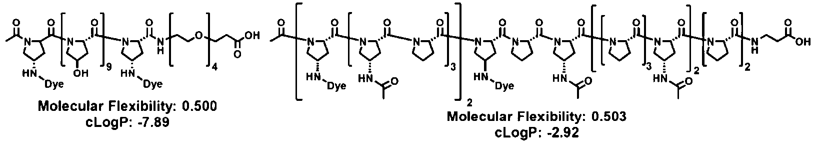

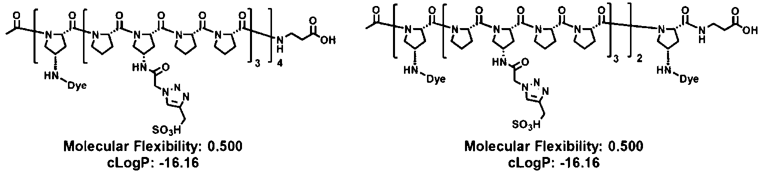

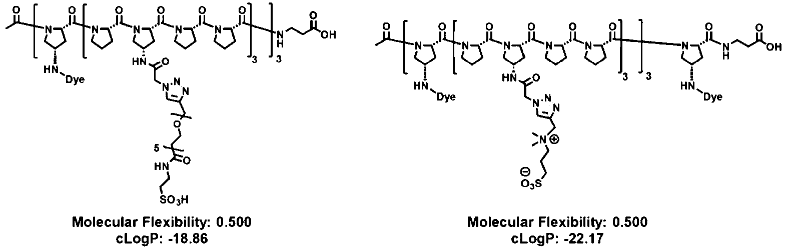

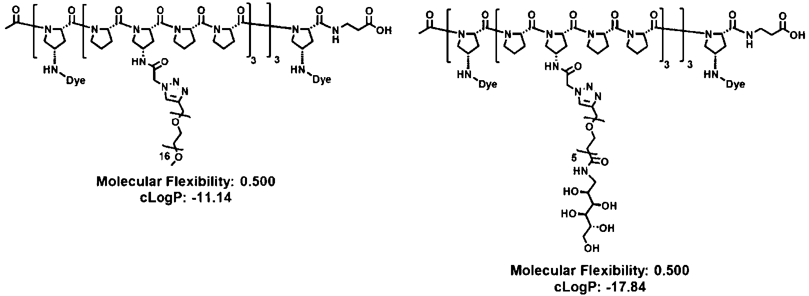

It is noted that in the compound according to the embodiment of the present invention, which are exemplified as described above, the structure used for the calculation of the Molecular Flexibility of the linking group which links the phosphor moieties adjacent to each other is referred to as "calculation structure (Molecular Flexibility)", and the structure used for the calculation of the cLogP is referred to as "calculation structure (cLogP)" as shown below. In a case where the structures used for the calculation of the Molecular Flexibility and the calculation of the cLogP are the same, they are simply shown collectively as "calculation structure".

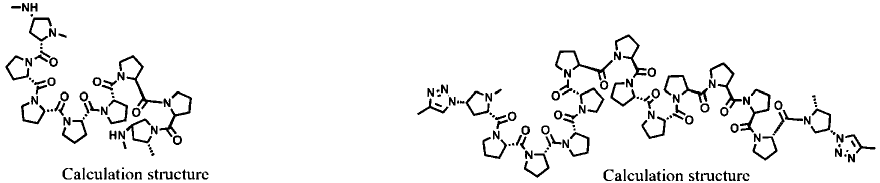

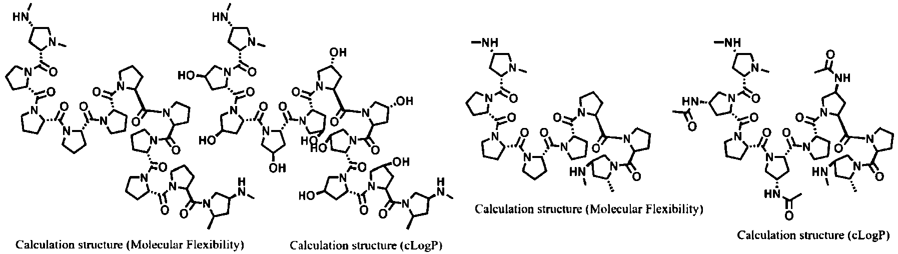

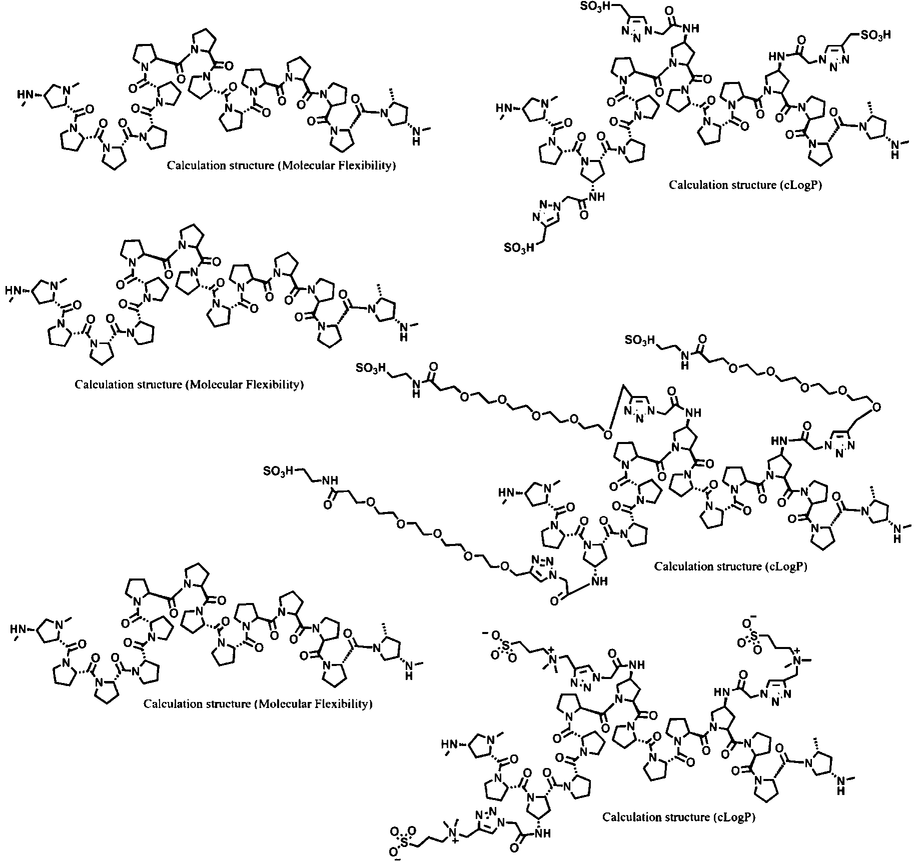

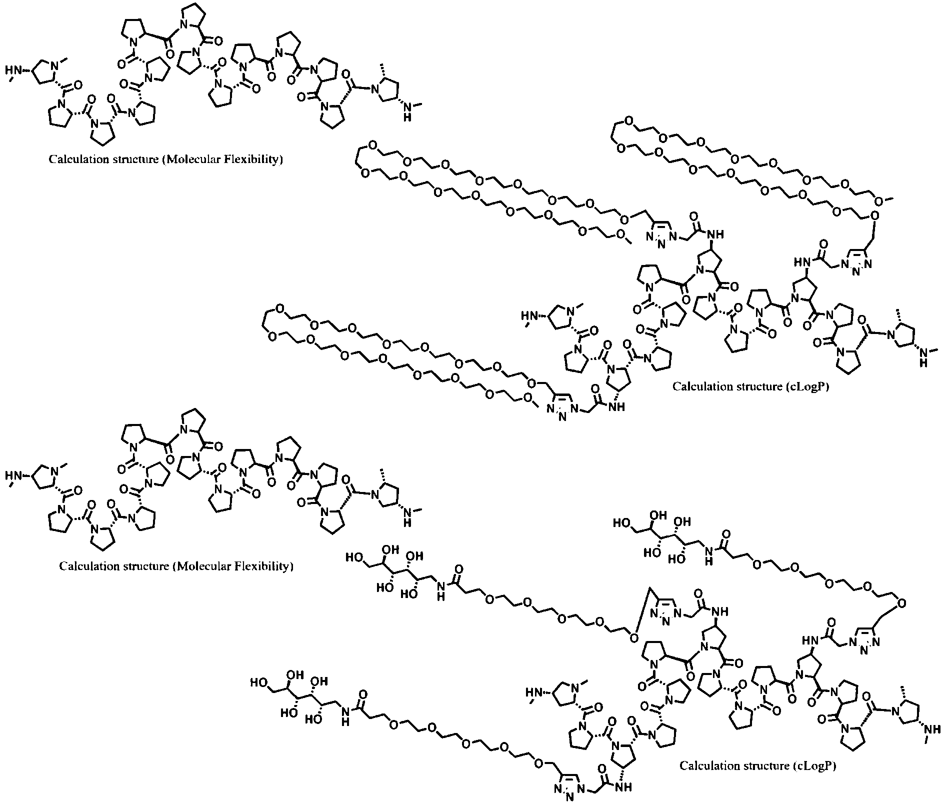

The compound according to the embodiment of the present invention can be bonded to a biological substance such as a protein (including a peptide), an amino acid, a nucleic acid, a nucleotide, a sugar chain, or a lipid, by at least one substituent allowing binding to a biological substance, where the substituent is contained in the compound, and the compound can be used as a labeled biological substance.

The substituent allowing binding to a biological substance can be used without particular limitation as long as it is a group for acting (including adhering) or bonding to a biological substance, and examples thereof include the substituents described in WO2002/026891A. Specifically, the description of the electrophilic group and the nucleophilic group described in Table 2 of WO2002/026891A and the reactive group Rx described on page 18, line 16 to page 19, line 13 of WO2002/026891A can be applied to the present invention.

Specific examples of "the substituent allowing binding to a biological substance" include the following structures.

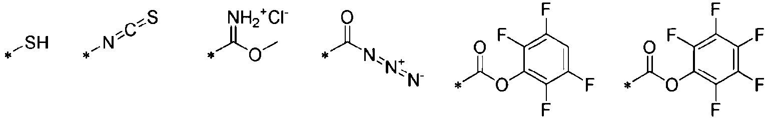

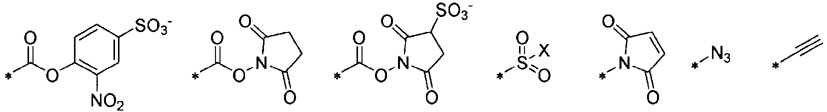

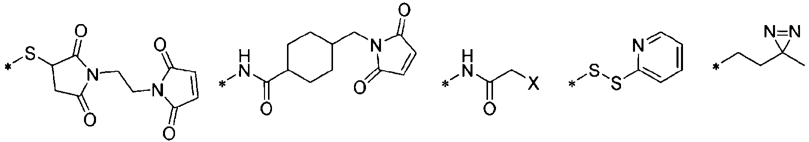

X means a halogen atom such as an iodine atom or a bromine atom. * represents a bonding site.

In addition to the above, it is possible to use a peptide structure (a polyamino acid structure), a long-chain alkyl group, or the like can be used as the "substituent allowing binding to a biological substance".

Among them, preferred examples thereof include an N-hydroxysuccinimide ester structure (an NHS ester structure), a succinimide structure, a maleimide structure, an azide group, an acetylene group, a peptide structure (a polyamino acid structure), a long-chain alkyl group (preferably having 12 to 30 carbon atoms), and a quaternary ammonium group.

Among the compounds according to the embodiment of the present invention, specific examples of the compound having at least one substituent allowing binding to a biological substance also include, as a specific example, a form in which the carboxy group in the above-described exemplary compound according to the present invention is appropriately replaced with the substituent allowing binding to a biological substance described above. It is noted that the present invention is not limited to these compounds. For example, in the specific examples thereof, a group having a dissociative hydrogen atom such as a carboxy group or a sulfo group may adopt a salt structure by a hydrogen atom being dissociated therefrom.

The compound according to the embodiment of the present invention can be bonded to a solid support such as the above-described microparticles by a substituent allowing binding to at least one solid support, the substituent being contained in the compound, and it can be used as a solid support reagent.

The substituent that can be bonded to a solid support can be used without particular limitation as long as it is a group for acting on (including adhering to) or bonding to a solid support, and examples thereof preferably include the substituents described as the above-described substituent allowing binding to a biological substance. Among them, preferred examples thereof include an N-hydroxysuccinimide ester (NHS ester) structure, a succinimide structure, and a maleimide structure.

Among the compounds according to the embodiment of the present invention, specific examples of the compound having at least one substituent allowing binding to a solid support also include, as a specific example, a form in which the carboxy group in the above-described exemplary compound of the compound according to the embodiment of the present invention is appropriately replaced with the substituent allowing binding to a solid support described above. It is noted that the present invention is not limited to these compounds. For example, in the specific examples thereof, a group having a dissociative hydrogen atom such as a carboxy group or a sulfo group may adopt a salt structure by a hydrogen atom being dissociated therefrom.

The compound according to the embodiment of the present invention can be synthesized according to a conventional method. For example, it can be synthesized based on the peptide synthesis such as solid phase peptide synthesis, and a method that uses an automatic peptide synthesizer described in WO2018/174078A can also be preferably applied. The phosphor moiety, the physiologically active substance moiety, the prodrug moiety, and the radioactive isotope-containing moiety can also be synthesized based on a conventional method and introduced into the compound according to the embodiment of the present invention.

A compound having a substituent allowing binding to a biological substance can also be synthesized according to a conventional method. For example, Bioconjugate Techniques (Third Edition, written by Greg T. Hermanson) can be referred to.

### <<Labeled biological substance>>

The labeled biological substance according to the embodiment of the present invention is a substance in which the compound according to the embodiment of the present invention, is bonded to a biological substance. Since the compound according to the embodiment of the present invention has fluorescence due to the phosphor moiety and exhibits an excellent effect of suppressing the association in a solution state, it can be preferably used for a labeled biological substance. The bond between the compound according to the embodiment of the present invention and a biological substance may have a form in which the compound according to the embodiment of the present invention and the biological substance are directly bonded or a form of being linked via a linking group.

Preferred examples of the biological substance include a protein (including a peptide), an amino acid, a nucleic acid, a nucleotide, a sugar chain, and a lipid. Preferred examples of the protein include an antibody, and preferred examples of the lipid include a phospholipid, a fatty acid, and sterol, where a phospholipid is more preferable.

Among the above biological substances, the clinically useful substance is not particularly limited; however, examples thereof include immunoglobulins such as immunoglobulin (Ig) G, IgM, IgE, IgA, and IgD; blood plasma proteins such as complement, C-reactive protein (CRP), ferritin, α₁ microglobulin, β₂ microglobulin, and antibodies thereof; tumor markers such as α-fetoprotein, carcinoembryonic antigen (CEA), prostate acid phosphatase (PAP), carbohydrate antigen (CA) 19-9, and CA-125, and antibodies thereof; hormones such as luteinizing hormone (LH), follicle-stimulating hormone (FSH), human ciliated gonadotropin (hCG), estrogen, and insulin, and antibodies thereof; and viral infection-related substances of viruses such HIV and ATL, hepatitis B virus (HBV)-related antigens (HBs, HBe, and HBc), human immunodeficiency virus (HIV), adult T-cell leukemia (ATL), and antibodies thereof.

The examples thereof further include bacteria such as Corynebacterium diphtheriae, Clostridium botulinum, mycoplasma, and Treponema pallidum, and antibodies thereof; protozoa such as Toxoplasma, Trichomonas, Leishmania, Trypanosoma, and malaria parasites, and antibodies thereof; embryonic stem (ES) cells such as ELM3, HM1, KH2, v6.5, v17.2, v26.2 (derived from mice, 129, 129/SV, C57BL/6, and BALB/c), and antibodies thereof; antiepileptic drugs such as phenytoin and phenobarbital; cardiovascular drugs such as quinidine and digoxin; anti-asthma drugs such as theophylline; drugs such as antibiotics such as chloramphenicol and gentamicin, and antibodies thereof; and enzymes, extracellular toxins (for example, streptolysin O), and the like, and antibodies thereof. In addition, antibody fragments such as Fab'2, Fab, and Fv can also be used.

The specific form in which the compound according to the embodiment of the present invention and a biological substance interact with each other to be bonded to each other includes, for example, the forms described below.
i) A non-covalent bond (for example, hydrogen bond, ionic bond including chelate formation) or a covalent bond between a peptide in the compound according to the embodiment of the present invention and a peptide in the biological substance,
ii) a Van der Waals force between a long-chain alkyl group in the compound according to the embodiment of the present invention and a lipid bilayer, a lipid, or the like in the biological substance,
iii) an amide bond formed by reacting an N-hydroxysuccinimide ester (NHS ester) in the compound according to the embodiment of the present invention with an amino group in the biological substance,
iv) a thioether bond formed by reacting a maleimide group in the compound according to the embodiment of the present invention with a sulfanyl group (-SH) in the biological substance, and
v) a formation of a triazole ring, which is formed by a Click reaction between an azide group in the compound according to the embodiment of the present invention and an acetylene group in the biological substance, or a Click reaction between an acetylene group in the compound according to the embodiment of the present invention and an azide group in the biological substance.

Provided that in the form of the i) described above, the peptide in the compound according to the embodiment of the present invention is not particularly limited as long as it is a peptide that is capable of forming a non-covalent bond or a covalent bond with a peptide in the biological substance, and preferred examples of the position where such a peptide is provided include R⁶ or R⁷ in General Formula (II).

In addition to the forms i) to v) described above, the bonding can be formed, for example, in the form described in Lucas C. D. de Rezende and Flavio da Silva Emery. A Review of the Synthetic Strategies for the Development of BODIPY Dyes for Conjugation with Proteins, Orbital: The Electronic Journal of Chemistry, 2013, Vol 5, No. 1, p. 62-83. In addition, the method described in the same document can be appropriately referred to for the preparation of the labeled biological substance according to the embodiment of the present invention.

Among the compounds according to the embodiment of the present invention, the labeled biological substance according to the embodiment of the present invention, which is obtained from a compound having a substituent allowing binding to a biological substance and a biological substance that is bonded to the compound by an interaction includes the compound in which a moiety other than the substituent allowing binding to a biological substance is replaced with the compound according to the embodiment of the present invention, and a product thereof, in the description of the compound example and the product in paragraph [0038] of JP2019-172826A. Provided that the present invention is not limited to these labeled biological substances and the like.

### <Reagent containing labeled biological substance>

In the reagent containing the labeled biological substance according to the embodiment of the present invention, the form of the labeled biological substance according to the embodiment of the present invention, for example, a solution form dissolved in an aqueous medium such as saline or a phosphate buffer solution, and a solid form such as a fine particle powder or a freeze-dried powder, is not particularly limited and can be appropriately selected depending on the purpose of use.

For example, in a case where the labeled biological substance according to the embodiment of the present invention is used as a fluorescence labeling reagent, it can be used as a reagent containing the labeled biological substance having any one of the forms described above.

### <Use application of labeled biological substance>

It is considered that the labeled biological substance according to the embodiment of the present invention, which is obtained from the compound according to the embodiment of the present invention, can exhibit an excellent fluorescence intensity because of the excellent effect of suppressing association of the compound according to the embodiment of the present invention in a solution state, and can stably detect fluorescence emitted from the labeled biological substance excited by irradiation with light. As a result, the labeled biological substance according to the embodiment of the present invention can be applied to various techniques using the fluorescence labeling, and it can be suitably used, for example, as a fluorescence labeling reagent in a multicolor WB or dot blotting or as a reagent for in vivo fluorescence imaging.

The fluorescence detection carried out using the labeled biological substance according to the embodiment of the present invention usually includes the following processes (i) to (iii) or (iv) to (vii). The fluorescence detection including the processes (i) to (iii) corresponds to the direct method using a primary antibody fluorescently labeled with the compound according to the embodiment of the present invention, and the fluorescence detection including the processes (iv) to (vii) corresponds to the indirect method using a secondary antibody fluorescently labeled with the compound according to the embodiment of the present invention.
(i) The process of preparing each of the following (a) and (b)
   (a) A sample containing a targeted biological substance (hereinafter, also referred to as a "target biological substance")
   (b) A labeled biological substance according to the embodiment of the present invention (hereinafter, also referred to as a "labeled biological substance A according to the embodiment of the present invention") obtained by bonding the biological substance (hereinafter, also referred to as a "primary biological substance") capable of binding to the target biological substance in the above (a) to the compound according to the embodiment of the present invention
(ii) The process of preparing a conjugate (hereinafter, also referred to as a "fluorescently labeled conjugate A") in which the target biological substance in the above (a) is bonded to the primary biological substance in the labeled biological substance A according to the embodiment of the present invention in the above (b)
(iii) The process of irradiating the fluorescently labeled conjugate A with light having the range of the wavelength which is absorbed by the labeled biological substance A according to the embodiment of the present invention, and detecting the fluorescence emitted by the labeled biological substance A according to the embodiment of the present invention
(iv) The process of preparing each of the following (c) to (e)
   (c) A sample containing a target biological substance
   (d) A biological substance capable of binding to the target biological substance in the above (c) (hereinafter, also referred to as a "primary biological substance")
   (e) A labeled biological substance according to the embodiment of the present invention (hereinafter, also referred to as a "labeled biological substance B according to the embodiment of the present invention") obtained by bonding the biological substance (hereinafter, also referred to as a "secondary biological substance") capable of binding to the primary biological substance in the above (d) to the compound according to the embodiment of the present invention (hereinafter, also referred to as a "labeled biological substance B according to the embodiment of the present invention")
(v) The process of preparing a conjugate (hereinafter, also referred to as a "conjugate b") in which the target biological substance in the above (c) is bonded to the primary biological substance of the above (d)
(vi) The process of preparing a conjugate (hereinafter, also referred to as a "fluorescently labeled conjugate B2") in which the primary biological substance in the conjugate b is bonded to the secondary biological substance in the labeled biological substance B according to the embodiment of the present invention
(vii) The process of irradiating the fluorescently labeled conjugate B2 with light having the range of the wavelength which is absorbed by the labeled biological substance B according to the embodiment of the present invention, and detecting the fluorescence emitted by the labeled biological substance B according to the embodiment of the present invention

Examples of the biological substance (the primary biological substance) capable of binding to the target biological substance and the biological substance (the secondary biological substance) capable of binding to the primary biological substance include the biological substances in the labeled biological substance according to the embodiment of the present invention. The above biological substance can be appropriately selected in accordance with the target biological substance (a biological substance in the test object) or the primary biological substance, and a biological substance capable of specifically binding to the biological substance in the test object or to the primary biological substance can be selected.

Examples of the protein among the target biological substances include a protein, which is a so-called disease marker. The disease marker is not particularly limited; however, examples thereof include α-fetoprotein (AFP), protein induced by vitamin K absence or antagonist II (PIVKA-II), breast carcinoma-associated antigen (BCA)225, basic fetoprotein (BFP), carbohydrate antigen (CA) 15-3, CA19-9, CA72-4, CA125, CA130, CA602, CA54/61 (CA546), carcinoembryonic antigen (CEA), DUPAN-2, elastase 1, immunosuppressive acidic protein (IAP), NCC-ST-439, γ-seminoprotein (γ-Sm), prostate specific antigen (PSA), prostatic acid phosphatase (PAP), nerve specific enolase (NSE), Iba1, amyloid β, tau, flotillin, squamous cell carcinoma associated antigen (SCC antigen), sialyl LeX-i antigen (SLX), SPan-1, tissue polypeptide antigen (TPA), sialyl Tn antigen (STN), cytokeratin (CYFRA), pepsinogen (PG), C-reactive protein (CRP), serum amyloid A protein (SAA), myoglobin, creatine kinase (CK), troponin T, and ventricular muscle myosin light chain I.

The target biological substance may be a bacterium. Examples of the bacterium include a bacterium to be subjected to a cellular and microbiological test, which is not particularly limited. Specific examples thereof include Escherichia coli, Salmonella, Legionella, and bacteria causing problems in public health.

The target biological substance may be a virus antigen. Although the virus antigen is not particularly limited, examples thereof include hepatitis virus antigens such as hepatitis C and B virus antigens, p24 protein antigen of HIV virus, and pp65 protein antigen of cytomegalovirus (CMV), and E6 and E7 protein antigens of human papillomavirus (HPV).

In the above (i) or (iv), the sample containing the target biological substance is not particularly limited and can be prepared according to a conventional method.

In addition, the labeled biological substance according to the embodiment of the present invention is not particularly limited and can be prepared by bonding a biological substance capable of binding to a target biological substance to the compound according to the embodiment of the present invention, according to a conventional method. The form of the bond and the reaction to form the bond are as described above in the labeled biological substance according to the embodiment of the present invention.

In the above (v), the target biological substance may be directly bonded to the primary biological substance or may be bonded through another biological substance which is different from the target biological substance and the primary biological substance. In addition, in the above (vi), the primary biological substance in the conjugate b may be directly bonded to the secondary biological substance in the labeled biological substance B according to the embodiment of the present invention or may be bonded through another biological substance which is different from the primary biological substance and the secondary biological substance.

The labeled biological substance according to the embodiment of the present invention can be used as a fluorescently labeled antibody in both the direct method and the indirect method but is preferably used as a fluorescently labeled antibody in the indirect method.

In the above (ii) or (v) and the above (vi), the binding of the labeled biological substance or the like according to the embodiment of the present invention to the target biological substance is not particularly limited and can be carried out according to a conventional method.

In the above (iii) or (vii), the wavelength for exciting the labeled biological substance according to the embodiment of the present invention is not particularly limited as long as the wavelength is light having wavelength capable of exciting the labeled biological substance according to the embodiment of the present invention. Generally, the wavelength for excitation is preferably 300 to 1,000 nm and more preferably 400 to 800 nm.

The fluorescence excitation light source used in the present invention is not particularly limited as long as it emits light having light having wavelength capable of exciting the labeled biological substance according to the embodiment of the present invention, and for example, various laser light sources can be used. In addition, various optical filters can be used to obtain a preferred excitation wavelength or detect only fluorescence.

Other matters in the above (i) to (vii) are not particularly limited, and conditions of a method, a reagent, a device, and the like, which are generally used in the fluorescence detection using fluorescence labeling, can be appropriately selected.

In addition, also regarding the steps other than the above (i) to (vii), conditions of a method, a reagent, a device, and the like, which are generally used, can be appropriately selected in accordance with various methods using fluorescence labeling.

For example, in the multicolor WB using the labeled biological substance according to the embodiment of the present invention, it is possible to detect a target biological substance with excellent fluorescence intensity by preparing a blotted membrane according to a method generally used for a target biological substance (protein separation by electrophoresis, blotting to a membrane, and blocking of a membrane) and using the labeled biological substance according to the embodiment of the present invention as a labeled antibody (preferably, as a secondary antibody). In the dot blotting using the labeled biological substance according to the embodiment of the present invention, as in the case of the multicolor WB, it is possible to detect a target biological substance with excellent fluorescence intensity by preparing a blotted nitrocellulose membrane, a blotted PVDF (polyvinylidene fluoride) membrane, or the like according to a method generally used for a target biological substance and using the labeled biological substance according to the embodiment of the present invention as a labeled antibody (preferably, as a secondary antibody).

### - Substituent group T -

In the present invention, the preferred substituents include those selected from the following substituent group T.

In addition, in the present invention, in a case where the substituent is simply described as a substituent, the description of the corresponding substituent in the substituent group T can be referred to and applied. For example, in a case where only "alkyl group" is described, the description of "alkyl group" in the substituent group T can be referred to and applied. The same applies to other substituents other than the "alkyl group".

In addition, in the present invention, examples of a substituent which may be contained in a certain substituent such as an "alkyl group", include a substituent selected from the following substituent group T. In addition, in a case where a certain substituent such as an "alkyl group" has a substituent and further has a substituent, examples of the substituent which is contained in a certain substituent include a substituent obtained by combining two or more substituents selected from the following substituent group T.

Furthermore, in the present invention, in a case where an alkyl group is described separately from a cyclic (cyclo)alkyl group, the alkyl group is meant to include a linear alkyl group and a branched alkyl group. On the other hand, in a case where an alkyl group is not described separately from a cyclic alkyl group, and unless otherwise specified, the alkyl group is meant to include a linear alkyl group, a branched alkyl group, and a cycloalkyl group. The same applies to groups (alkoxy group, alkylthio group, alkenyloxy group, and the like) containing a group capable of having a cyclic structure (alkyl group, alkenyl group, alkynyl group, and the like) and compounds containing a group capable of having a cyclic structure. In a case where a group is capable of forming a cyclic skeleton, the lower limit of the number of atoms of the group forming the cyclic skeleton is 3 or more and preferably 5 or more, regardless of the lower limit of the number of atoms specifically described below for the group that can adopt this structure.

In the following description of the substituent group T, a group having a linear or branched structure and a group having a cyclic structure, such as an alkyl group and a cycloalkyl group, are sometimes described separately for clarity.

The groups included in the substituent group T include the following groups.

Examples thereof include an alkyl group (preferably having 1 to 30 carbon atoms, more preferably having 1 to 20 carbon atoms, still more preferably having 1 to 12 carbon atoms, even still more preferably having 1 to 8 carbon atoms, yet even still more preferably having 1 to 6 carbon atoms, and particularly preferably having 1 to 3 carbon atoms), an alkenyl group (preferably having 2 to 30 carbon atoms, more preferably having 2 to 20 carbon atoms, still more preferably having 2 to 12 carbon atoms, even still more preferably having 2 to 6 carbon atoms, and yet even still more preferably having 2 to 4 carbon atoms), an alkynyl group (preferably having 2 to 30 carbon atoms, more preferably having 2 to 20 carbon atoms, still more preferably having 2 to 12 carbon atoms, even still more preferably having 2 to 6 carbon atoms, and yet even still more preferably having 2 to 4 carbon atoms), a cycloalkyl group (preferably having 3 to 20 carbon atoms), a cycloalkenyl group (preferably having 5 to 20 carbon atoms), an aryl group (may be a monocyclic group or a fused ring group (preferably a fused group of 2 to 6 rings); in a case of a fused ring group, the fused ring group consists of a 5-membered to 7-membered ring; preferably having 6 to 40 carbon atoms, more preferably 6 to 30 carbon atoms, still more preferably 6 to 26 carbon atoms, and particularly preferably 6 to 10 carbon atoms), a heterocycle group (having at least one nitrogen atom, an oxygen atom, a sulfur atom, a phosphorus atom, a silicon atom, or selenium atom as a ring-constituting atom; may be a monocyclic ring or a fused ring (preferably a fused group in which 2 to 6 rings); in a case of a monocyclic group, the monocyclic ring is preferably a 5-membered to 7-membered ring and more preferably a 5-membered or 6-membered ring; preferably having 2 to 40 carbon atoms and more preferably having 2 to 20 carbon atoms; and the heterocyclic group includes an aromatic heterocyclic group (heteroaryl group) and an aliphatic heterocyclic group (aliphatic heterocyclic group)), an alkoxy group (preferably having 1 to 20 carbon atoms and more preferably having 1 to 12 carbon atoms), an alkenyloxy group (preferably having 2 to 20 carbon atoms and more preferably having 2 to 12 carbon atoms), an alkynyloxy group (preferably having 2 to 20 carbon atoms and more preferably having 2 to 12 carbon atoms), a cycloalkyloxy group (preferably having 3 to 20 carbon atoms), an aryloxy group (preferably having 6 to 40 carbon atoms, more preferably having 6 to 26 carbon atoms, and still more preferably having 6 to 14 carbon atoms), a heterocyclic oxy group (preferably having 2 to 20 carbon atoms), a polyalkyleneoxy group,
an alkoxycarbonyl group (preferably having 2 to 20 carbon atoms), a cycloalkoxycarbonyl group (preferably having 4 to 20 carbon atoms), an aryloxycarbonyl group (preferably having 6 to 20 carbon atoms), an amino group (preferably having 0 to 20 carbon atoms; including an unsubstituted amino group (-NH₂), a (mono- or di-) alkylamino group, a (mono- or di-) alkenylamino group, a (mono- or di-) alkynylamino group, a (mono- or di-) cycloalkylamino group, a (mono- or di-) cycloalkenylamino group, a (mono- or di-) arylamino group, and a (mono- or di-) heterocyclic amino group; and the above-described groups that substitute an unsubstituted amino group have the same meanings as the corresponding groups in the substituent group T), a sulfamoyl group (preferably having 0 to 20 carbon atoms; and preferably an alkyl, cycloalkyl, or aryl sulfamoyl group), an acyl group (preferably having 1 to 20 carbon atoms and more preferably having 2 to 15 carbon atoms; and including -C(=O)H, an alkylcarbonyl group, a cycloalkylcarbonyl group, an arylcarbonyl group, and a heterocyclic carbonyl group), an acyloxy group (preferably having 1 to 20 carbon atoms), a carbamoyl group (preferably having 1 to 20 carbon atoms; and preferably an alkyl, cycloalkyl, or aryl carbamoyl group),
an acylamino group (preferably having 1 to 20 carbon atoms), a sulfonamide group (preferably having 0 to 20 carbon atoms; and preferably an alkyl, cycloalkyl, or aryl sulfonamide group), an alkylthio group (preferably having 1 to 20 carbon atoms and more preferably having 1 to 12 carbon atoms), a cycloalkylthio group (preferably having 3 to 20 carbon atoms), an arylthio group (preferably having 6 to 40 carbon atoms, more preferably having 6 to 26 carbon atoms, and still more preferably having 6 to 14 carbon atoms), a heterocyclic thio group (preferably having 2 to 20 carbon atoms), an alkyl, cycloalkyl, or aryl sulfonyl group (preferably having 1 to 20 carbon atoms),
a silyl group (preferably having 1 to 30 carbon atoms and more preferably 1 to 20 carbon atoms; preferably a silyl group at which an alkyl, aryl, alkoxy, or aryloxy has been substituted), a silyloxy group (preferably having 1 to 20 carbon atoms: preferably a silyloxy group at which an alkyl, aryl, alkoxy, or aryloxy has been substituted), a hydroxy group, a cyano group, a nitro group, a halogen atom (for example, a fluorine atom, a chlorine atom, a bromine atom or an iodine atom), an oxygen atom (specifically replacing >CH₂ which constitutes a ring with >C=O), a carboxy group (-CO₂H), a phosphono group [-PO(OH)₂], a phosphonooxy group [-O-PO(OH)₂], a sulfo group (-SO₃H), a boric acid group [-B(OH)₂], onio group (referred to as a cationic group; including an ammonio group containing a cyclic ammonio group, a sulfonio group, and a phosphonio group; and preferably having 0 to 30 carbon atoms and more preferably having 1 to 20 carbon atoms), a sulfanyl group (-SH), a guanidino group (-NHC(=NH)NH₂), a betaine residue, a polyol residue, a sugar residue, or a polyamino acid residue.

### (Anionic group)

In the present invention, the anionic group may be any group having an anion. Examples of such an anionic group include a carboxy group, a phosphono group (a phosphonate group, -PO(OH)₂), a phosphonooxy group (a phosphate group, -OPO(OH)₂), and a sulfo group, where a phosphono group, a phosphonooxy group, or a sulfo group is preferable, and a phosphonooxy group or a sulfo group is more preferable.

The anionic group may have an ionic structure in which a hydrogen ion is dissociated, or may have a salt structure. To the monovalent or polyvalent cation in a case where the anionic group has a salt structure, the description of the monovalent or polyvalent cation in the description of the salt structure described above can be preferably applied.

### (Cationic group)

In the present invention, the cationic group may be any group having a cation. Examples of such a cationic group include a group having a quaternary ammonium ion (an ammonio group) and a group having a quaternary phosphonium ion (a phosphonio group), where a group having a quaternary ammonium ion is preferable. It is noted that preferred examples of the substituent possessed by N⁺ in the group having a quaternary ammonium ion and the substituent possessed by P⁺ in the group having a quaternary phosphonium ion include an alkyl group and an aryl group, where groups in which all the substituents possessed by N⁺ and P⁺ are alkyl groups are preferable.

The cationic group may have a salt structure in addition to the ionic structure. Examples of the monovalent or polyvalent anion in a case where the cationic group has a salt structure include halide ions such as F⁻ and Cl⁻, and monovalent or polyvalent organic anions such as BF₄⁻, PF₆⁻, and a bis(trifluoromethylsulfonyl)imide ion.

### (Betaine residue)

In the present invention, the betaine residue means a group obtained by removing one hydrogen atom from a compound having a betaine structure. The compound having a betaine structure is only required to be a compound containing an anionic group and a cationic group in the same molecule, and is preferably a compound containing, in the same molecule, at least one anionic group among a carboxy group, a phosphono group, a phosphonooxy group, and a sulfo group, and at least one cationic group among an ammonio group and a phosphonio group.

A group obtained by removing one hydrogen atom from a compound that does not contain an anionic group and a cationic group in the same molecule does not correspond to a betaine residue, and is classified into an anionic group having a salt structure or a cationic group having a salt structure.

### (Polyol residue)

In the present invention, the polyol residue means a group obtained by removing one hydrogen atom from a polyol compound having two or more hydroxy groups in a molecule, the group not corresponding to any of a sugar residue and a polyalkyleneoxy group, which will be described later. The polyol residue may be a chain-like group or a group having a cyclic structure, and examples thereof include a cyclodextrin residue.

### (Sugar residue)

In the present invention, the sugar residue means a group obtained by removing one hydrogen atom from a sugar compound. The sugar compound may be any of a monosaccharide, a polysaccharide in which two or more sugars are bonded, a sugar alcohol, or a chemically modified sugar obtained by copolymerizing a sugar with epichlorohydrin or the like.

It is noted that the polyol residue corresponding to the sugar residue is classified as the sugar residue.

Examples of the sugar residue include groups obtained by removing one hydrogen atom from sugar compounds such as glucose, sucrose, maltose, lactose, trehalose, ribitol, sorbitol, mannitol, maltitol, lactitol, xylitol, fruit sugar (fructose), 1-kestose, nystose trihydrate, fucose, dulcitol, galactooligosaccharide, 4'-galactosyl lactose, isomalto-oligosaccharide, lactulose, palatinit, palatinose monohydrate, raffinose pentahydrate, arabinose, dihydroxyacetone dimer, galactose, glyceryl aldehyde dimer, mannose, ribose, xylose, lactosyl fructoside, erythritol, dulcoside A, isosteviol, rebaudioside A, rebaudioside B, rebaudioside C, rebaudioside D, rebaudioside F, rubusoside, stevioside, 1-deoxynojirimycin, fucoidan, rhamnose, threose, arabinose, lyxose, allose, altrose, gulose, idose, talose, 1,3-dihydroxyacetone, erythrose, xylulose, ribulose, psicose, sorbose, tagatose, polysucrose, and fructooligosaccharide.

### (Polyamino acid residue)

In the present invention, the polyamino acid residue means a group obtained by removing one hydrogen atom from an amino acid compound or a polyamino acid compound in which two or more amino acids are bonded. In the present invention, the polyamino acid residue is a group that is adjusted to be neutral (the sum of charges is 0) in the use form of the compound and the labeled biological substance according to the embodiment of the present invention, and used.

### (Polyalkyleneoxy group)

In the present invention, the polyalkyleneoxy group may be any group represented by -(O-L)_{g}R^{E} or -(L-O)_{g}R^{E}.

It is noted that the polyol residue corresponding to the polyalkyleneoxy group is classified as a polyalkyleneoxy group.

The L represents an alkylene group obtained by removing one hydrogen atom from an alkyl group in the substituent group T described above, where it preferably has 2 to 4 carbon atoms, more preferably has 2 or 3 carbon atoms, and still more preferably has 2 carbon atoms. The number of carbon atoms contained in the shortest chain that links two carbon atoms which are bonding sites of the group is preferably 0 to 2, more preferably 0 or 1, and still more preferably 0. That is, L is most preferably an ethylene group.

The g means an average repetition number (simply, also referred to as a repetition number), which is preferably 2 to 50, more preferably 12 to 50, and still more preferably 24 to 50. Even in a case where the repetition number is small, for example, even in a case where g is 2, a proper hydrophilicity and a proper excluded volume effect can be exhibited.

R^{E} has the same meaning as R⁸ to R¹⁰ described above, and represents a hydrogen atom, an alkyl group, an alkenyl group, an alkynyl group, an acyl group, an aryl group, a heteroaryl group, an anionic group, a betaine residue, a polyol residue, a sugar residue, or a polyamino acid residue, and a hydrogen atom or an alkyl group is preferable. For the alkyl group which can be adopted as R^{E}, the description of the alkyl group in the above-described substituent group T can be preferably applied, and among the above, an alkyl group having 1 to 3 carbon atoms is preferable. The alkyl group which can be adopted as R^{E} may have a substituent.

It is noted that in the present invention, in a case where the substituent group: "anionic group, betaine residue, polyol residue, sugar residue, polyamino acid residue, -(O-L)_{g}R^{E}, -(L-O)_{g}R^{E}" is described as all or a part of the options of the specific substituent, the substituent group: "anionic group, betaine residue, polyol residue, sugar residue, polyamino acid residue, -(O-L)_{g}R^{E}, -(L-O)_{g}R^{E}" in the options of the specific substituent is preferably replaced with the substituent group: "anionic group, polyol residue, sugar residue, polyamino acid residue, -(O-L)_{g}R^{E}, -(L-O)_{g}R^{E}", and more preferably replaced with the substituent group: "phosphono group, phosphonooxy group, sulfo group, polyol residue, sugar residue, polyamino acid residue, -(O-L)_{g}R^{E}, -(L-O)_{g}R^{E}" unless otherwise specified.

The same applies to a case where the substitent group "anionic group, betaine residue, polyol residue, sugar residue, polyamino acid residue, -(O-L)_{g}R^{E}, -(L-O)_{g}R^{E}" is described as all or a part of the preferred range of the substituent.

In addition, examples of the group obtained by combining a plurality of substituents selected from the substituent group T include the alkyl group, the alkenyl group, the alkynyl group, the cycloalkyl group, the cycloalkenyl group, the aryl group, the heterocyclic group, the alkoxy group, the alkenyloxy group, the alkynyloxy group, the cycloalkyloxy group, the aryloxy group, the heterocyclic oxy group, the alkoxycarbonyl group, the cycloalkoxycarbonyl group, the aryloxycarbonyl group, the amino group, the sulfamoyl group, the acyl group, the acyloxy group, the carbamoyl group, the acylamino group, the sulfonamide group, the alkylthio group, the cycloalkylthio group, the arylthio group, the heterocyclic thio group, and the alkyl, the cycloalkyl, and the aryl sulfonyl group, which have, as a substituent, an anionic group (a carboxy group, a phosphono group, or a sulfo group), a cationic group (an onio group), a betaine residue, a polyol residue, a sugar residue, a polyamino acid residue, -(O-L)_{g}R^{E}, or -(L-O)_{g}R^{E}.

The substituent selected from the substituent group T is more preferably an alkyl group, an alkenyl group, a cycloalkyl group, an aryl group, a heterocyclic group, an alkoxy group, a cycloalkoxy group, an aryloxy group, an acyl group, an alkoxycarbonyl group, a cycloalkoxycarbonyl group, an amino group, an acylamino group, a carbamoyl group, a cyano group, a halogen atom, an anionic group, a cationic group, a betaine residue, a polyol residue, a sugar residue, a polyamino acid residue, -(O-L)_{g}R^{E}, or -(L-O)_{g}R^{E}, and particularly preferably an alkyl group, an alkenyl group, an aryl group, a heterocyclic group, an alkoxy group, an acyl group, an alkoxycarbonyl group, an amino group, an acylamino group, a carbamoyl group, a cyano group, an anionic group, a cationic group, a betaine residue, a polyol residue, a sugar residue, a polyamino acid residue, -(O-L)_{g}R^{E}, or -(L-O)_{g}R^{E}.

In addition to the group obtained by combining a plurality of substituents selected from the substituent group T described above, the substituent selected from the substituent group T also includes a group obtained by combining a plurality of the above groups, unless otherwise specified. For example, in a case where a compound, a substituent, or the like contains an alkyl group, an alkenyl group, or the like, the alkyl group, the alkenyl group, or the like may be substituted or unsubstituted. In addition, in a case where a compound, a substituent, or the like contains an aryl group, a heterocyclic group, or the like, the aryl group, the heterocyclic group, or the like may be a monocyclic ring or a fused ring moiety, and may be substituted or unsubstituted.

### Examples

Hereinafter, the present invention will be described in more detail based on Examples, but the present invention is not limited thereto. It is noted that room temperature means 25°C.

Compounds (1) to (2) according to the embodiment of the present invention and comparative compounds (1) to (3) are shown below.

It is noted that in each compound, the sulfo group may include a salt structure (for example, a potassium salt, a sodium salt, a triethylammonium (TEA) salt, or an N,N-diisopropylethylammonium (DIPEA) salt), even unless otherwise specified.

It is noted that in each of the compounds, the structure used for the calculation of the Molecular Flexibility of the linking group which links the phosphor moieties adjacent to each other is referred to as "calculation structure (Molecular Flexibility)", and the structure used for the calculation of the cLogP is referred to as "calculation structure (cLogP)" as shown below. In a case where the structures used for the calculation of the Molecular Flexibility and the calculation of the cLogP are the same, they are simply shown collectively as "calculation structure".

In addition, the phosphor moiety (comparative compound (3)) in the compounds (1) and (2) and the comparative compound (1), and that in the comparative compound (2) have substantially the same fluorescence intensity when used alone, and it is thus considered that the difference in structure of the phosphor moiety does not significantly contribute to the evaluation results of the fluorescence intensity of each compound described later.

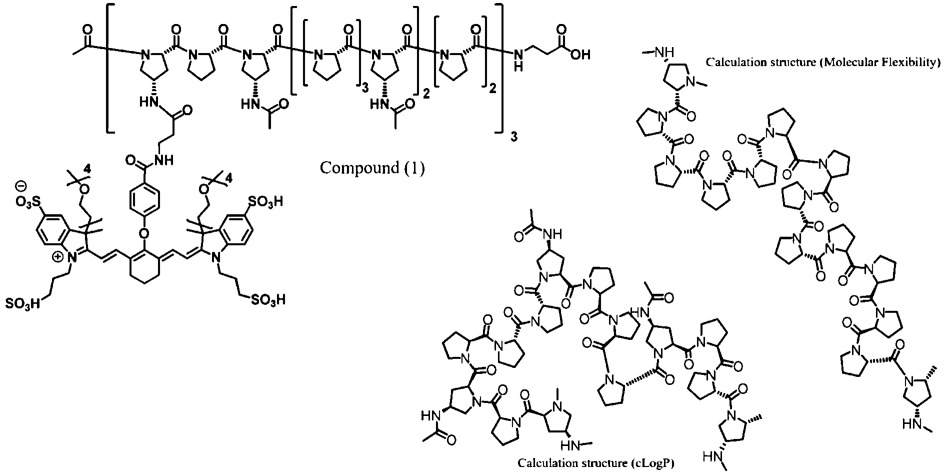

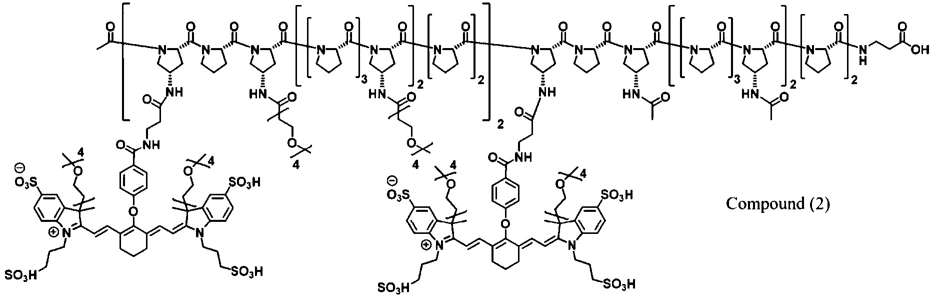

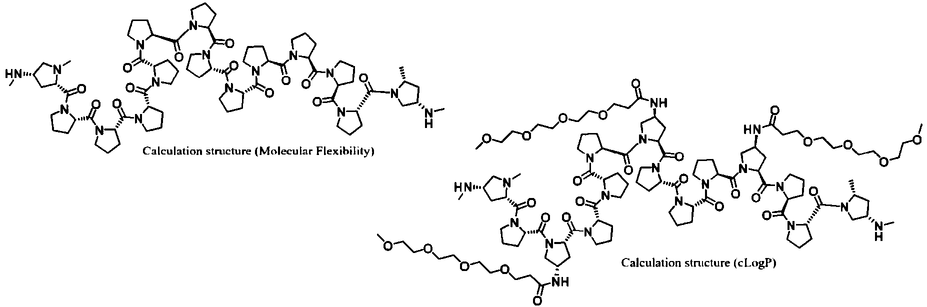

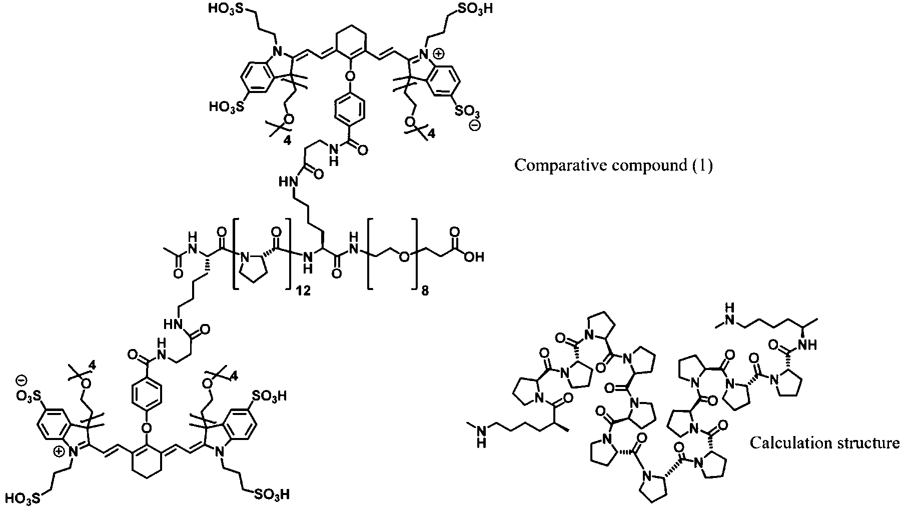

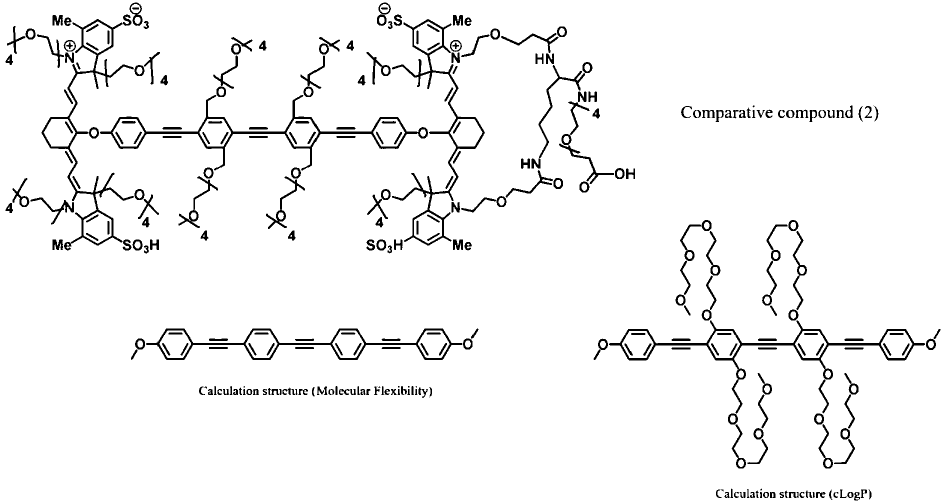

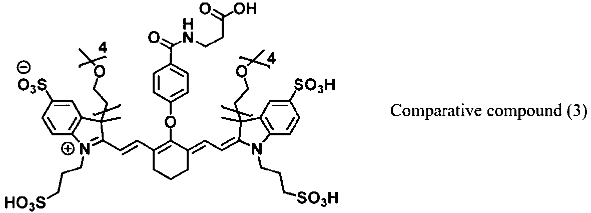

The comparative compound (2) is the compound (4) described in Examples of JP2022-131357A.

The comparative compound (3) is a compound (M2-13) in Synthesis Example of a compound (M2-1) described later.

The method of synthesizing each compound and a labeled antibody will be described in detail below; however, the starting materials, the dye intermediates, and the synthetic routes are not limited thereto.

It is noted that the abbreviations used in the synthesis of the respective compounds described below are as follows.
DBU: 1,8-diazabicyclo[5.4.0]-7-undecene
PyAOP: (7-azabenzotriazole-1-yloxy)tripyrrolidinophosphonium hexafluorophosphate
DMT-MM: 4-(4,6-dimethoxy-1,3,5-triazin-2-yl)-4-methylmorpholinium chloride
DIC: diisopropylcarbodiimide
NHS: N-hydroxysuccinimide
EDCI: 1-ethyl-3-(3-dimethylaminopropyl)carbodiimide hydrochloride
HOBt: 1-hydroxybenzotriazole
NMP: N-methyl-2-pyrrolidone
DMF: dimethylformamide
DMAP: 4-dimethylaminopyridine
DIPEA: N-diisopropylethylamine
TFA: trifluoroacetic acid
TFE: 2,2,2-trifluoroethanol
THF: tetrahydrofuran
Me: methyl group
Ms: mesyl group
Et: ethyl group
tBu: tert-butyl group
Ac: acetyl group
Trt: trityl group (triphenylmethyl group)
Fmoc: 9-fluorenylmethyloxycarbonyl group
Boc: tert-butoxycarbonyl group
Ala: alanine
Lys: lysine
Pro: proline
Resin: resin
PEGₘ or PEGm: poly(ethyleneoxy) group having average repetition number m

In addition, %v/v means a percent by volume, and %w/v means a percent by weight per volume.

Unless otherwise specified, SNAP Ultra C18 (product name, manufactured by Biotage, LLC) or Sfar C18 (product name, manufactured by Biotage, LLC) was used as the carrier in the reverse phase column chromatography, and Hi-Flash Column (product name, manufactured by Yamazen Corporation) was used as a carrier in the normal phase column chromatography.

The mixing ratio in the eluent used in the reverse phase column chromatography or the normal phase column chromatography is in terms of the volume ratio. For example, "acetonitrile:water = from 0:100 to 20:80" means that the eluent of "acetonitrile:water = 0:100" was changed to an eluent of "acetonitrile:water = 20:80".

For the preparative high performance liquid chromatography (HPLC), 2767 (product name, manufactured by Waters Corporation) was used.

The MS spectrum was measured by ACQUITY SQD LC/MS System [product name, manufactured by Waters Corporation, ionization method: electrospray Ionization (ESI)] or LCMS-2010EV [product name, manufactured by Shimadzu Corporation, ionization method: an ionization method simultaneously carrying out ESI and atmospheric pressure chemical ionization (APCI)].

It is noted that in the synthesis of each compound, the synthesis of the peptide chain was carried out according to the general method of the peptide solid phase method described in WO2018/174078A.

### [General method of solid phase peptide synthesis method using automatic peptide synthesizer]

Solid phase peptide synthesis was carried out using an automatic peptide synthesizer (product name: SyroI, manufactured by biotage, LLC). The synthesizer was set with Rink Amide-ChemMatrix (registered trade name, manufactured by Biotage, LLC), an N-methyl-2-pyrrolidone (NMP) solution of Fmoc amino acid (0.5 mol/L), an NMP solution of cyano-hydroxyimino-acetic acid ethyl ester (1.0 mol/L) and diisopropylethylamine (0.1 mol/L), an NMP solution of diisopropylcarbodiimide (1.0 mol/L), an NMP solution of piperidine (20% v/v), and an NMP solution of acetic anhydride (20 %v/v), and synthesis was carried out according to the manual. A cycle consisting of Fmoc deprotection (20 minutes), washing with NMP, condensation of Fmoc amino acids (1 hour), and washing with NMP as one cycle was repeated to elongate the peptide chain.

### [Synthesis of compound (M2-1)]

A compound (M2-1) was synthesized based on the following scheme. The results of the MS measurement of the compound (M2-13) were as follows.
MS (ESI m/z): (M+H⁺)⁺ = 1,384, (M-H⁺)⁻ = 1,382

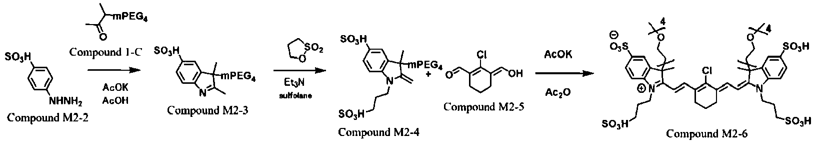

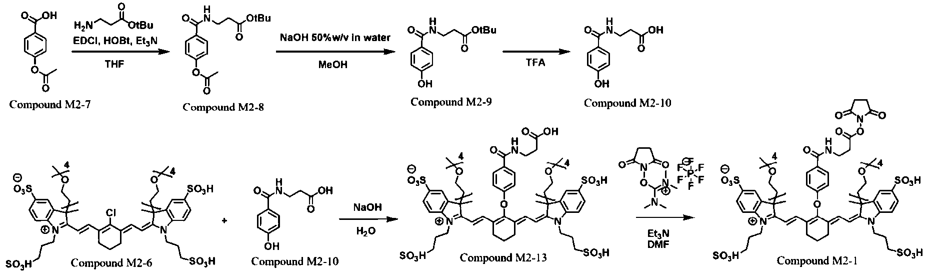

### <Synthesis of compound (1)>

### 1) Synthesis of compound (1-8)

A compound (1-8) was synthesized according to the following scheme.

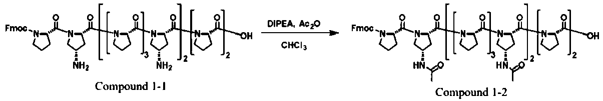

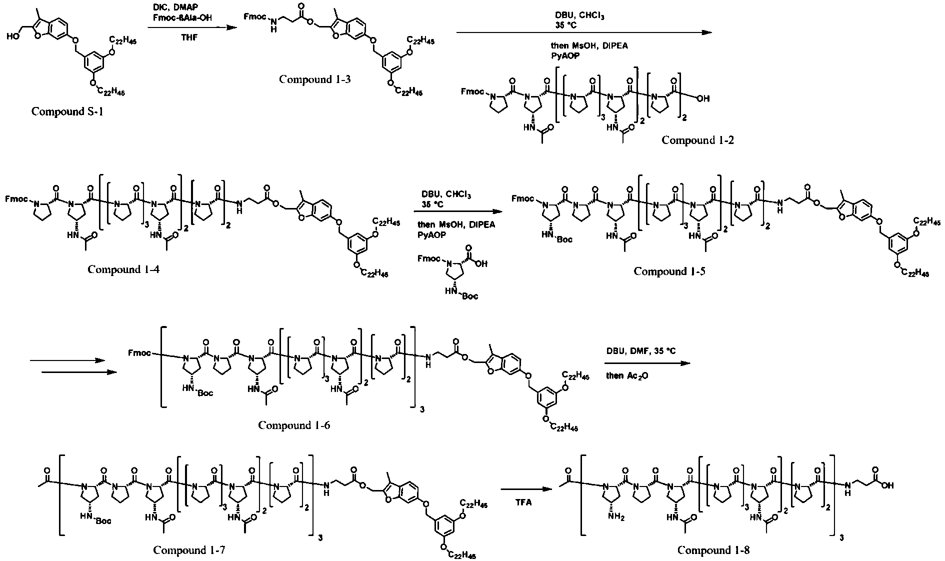

### (i) Synthesis of compound (1-1)

Solid phase peptide synthesis was carried out using H-Pro-Trt(2-Cl)-Resin (manufactured by Watanabe Chemical Industries, Ltd., 0.93 mmol/g, 53.8 mg) as a starting raw material. N-(9-fluorenylmethoxycarbonyl)-L-proline (Fmoc-Pro-OH) and (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH(2S,4S)) were subjected to elongation, and the elongation using N-(9-fluorenylmethoxycarbonyl)-L-proline (Fmoc-Pro-OH) was repeated for 3 cycles. (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH (2S,4S)) was subjected to elongation, and the elongation using N-(9-fluorenylmethoxycarbonyl)-L-proline (Fmoc-Pro-OH) was repeated for 3 cycles. (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH(2S,4S)) and N-(9-fluorenylmethoxycarbonyl)-L-proline (Fmoc-Pro-OH) were subjected to elongation. After completion of the elongation, the resin was washed with dichloromethane, and then the solvent was distilled off under reduced pressure. 2.0 mL of a mixed solution of TFA:triisopropylsilane:water = 95:2.5:2.5 was added thereto, and the peptide was cut out and deprotected. After 2 hours, the resin was filtered out, and methyl-t-butyl ether (12 mL) was added to the filtrate to generate a solid. The solid was precipitated by centrifugation, and then the supernatant was removed. The solid was washed with methyl-t-butyl ether, and then the solvent was distilled off under reduced pressure to obtain 51.2 mg of a white solid compound (1-1).

### (ii) Synthesis of compound (1-2)

352 mg of the compound (1-1), 3.5 mL of chloroform (CHCl₃), 423 µL of N-diisopropylethylamine (DIPEA), and 115 µL of acetic anhydride (Ac₂O) were placed in an eggplant flask having a capacity of 50 mL, and stirring was carried out at room temperature for 1 hour. Thereafter, the reaction solution was concentrated and purified by preparative HPLC, and freeze drying was followed to obtain 314 mg of a compound (1-2).

### (iii) Synthesis of compound (1-3)

500 mg of the compound (4-1) described in WO2020/175473A as the compound (S-1), 5 mL of tetrahydrofuran (THF), 254 mg of N-[(9H-fluoren-9-ylmethoxy)carbonyl]-(3-alanine (Fmoc-βAla-OH), 128 µL of diisopropylcarbodiimide, and 13.3 mg of 4-dimethylaminopyridine were placed in an eggplant flask having a capacity of 50 mL, and stirring was carried out at room temperature for 2 hours. Acetonitrile (50 mL) was added thereto to filter the precipitated solid, which was subsequently subjected to drying under reduced pressure to obtain 593 mg of a compound (1-3).

### (iv) Synthesis of compound (1-4)

108 mg of the compound (1-3), 2.2 mL of chloroform (CHCl₃), and 26.8 µL of 1,8-diazabicyclo[5.4.0]-7-undecene (DBU) were placed in an eggplant flask having a capacity of 50 mL, and stirring was carried out at 35°C for 1 hour. 11.7 µL of methanesulfonic acid (MsOH), 93.3 µL of N,N-diisopropylethylamine (DIPEA), 169 mg of the compound (1-2), and 141 mg of (7-azabenzotriazole-1-yloxy)tripyrrolidinophosphonium hexafluorophosphate (PyAOP) were placed therein, and stirring was carried out at 35°C for 3 hours. Acetonitrile (10 mL) was added thereto to filter the precipitated solid, which was subsequently subjected to drying under reduced pressure to obtain 219 mg of a compound (1-4).

### (v) Synthesis of compound (1-5)

233 mg of the above-described compound (1-5) was synthesized from 219 mg of the compound (1-4) in the same manner, except that in the synthesis of the compound (1-4) described above, (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH(2S,4S)) was used instead of the compound (1-2) and the compound (1-4) was used instead of the compound (1-3).

### (vi) Synthesis of compound (1-6)

The same operation as the above-described synthesis of the compounds (1-4) and (1-5) was repeated twice to synthesize 190 mg of a compound (1-6) from 100 mg of the compound (1-5).

### (vii) Synthesis of compound (1-7)

97.5 mg of the compound (1-6), 975 µL of N,N-dimethylformamide (DMF), and 8.9 µL of 1,8-diazabicyclo[5.4.0]-7-undecene (DBU) were placed in an eggplant flask having a capacity of 10 mL, and stirring was carried out at 35°C for 1 hour. 3.7 µL of acetic anhydride (Ac₂O) was added thereto, and the mixture was stirred at 35°C for 1 hour. A solid precipitated by adding acetonitrile (50 mL) was subjected to filtration and drying under reduced pressure to obtain 74.1 mg of a compound (1-7).

### (viii) Synthesis of compound (1-8)

30.1 mg of the compound (1-7) and 1.0 mL of TFA were added to an eggplant flask having a capacity of 10 mL, and the mixture was stirred at room temperature for 1 hour. 10 mL of methanol (MeOH) was added to the reaction solution to generate a solid to be removed, and then the solid was removed by filtration. The filtrate was concentrated and purified by preparative HPLC, and freeze drying was followed to obtain 10.6 mg of a compound (1-8).

### 2) Synthesis of compound (1)

A compound (1) was synthesized based on the following scheme.

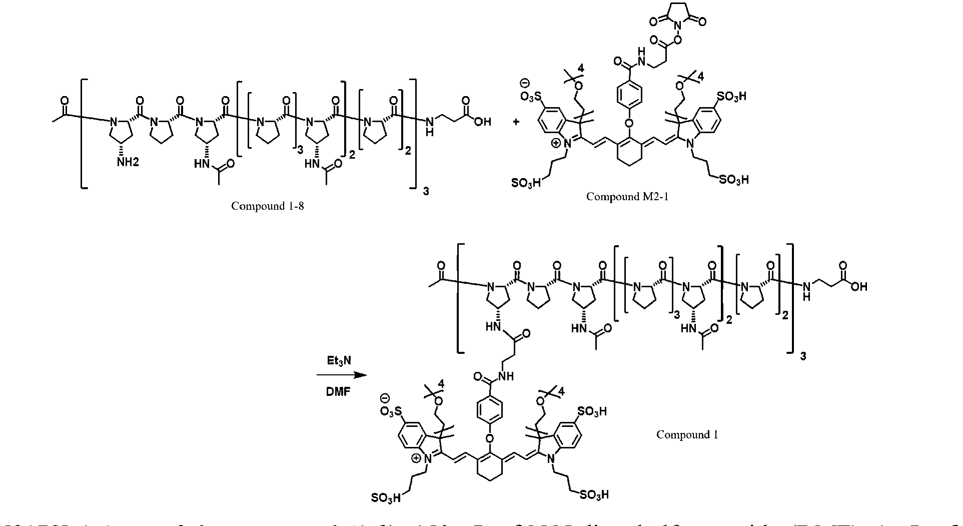

1.5 mg of the compound (1-8), 150 µL of N,N-dimethylformamide (DMF), 1 µL of triethylamine (Et₃N), and 3.75 mg of the compound (M2-1) were placed in an eggplant flask having a capacity of 10 mL, and stirring was carried out at room temperature for 1 hour. Thereafter, the reaction solution was concentrated and purified by preparative HPLC, and freeze drying was followed to obtain 0.85 mg of a compound (1). The results of the MS measurement of the compound (1) were as follows.
MS (ESI m/z): (M+H⁺)⁺ = 8,571, (M-H⁺)⁻ = 8,569

### <Synthesis of compound (2)>

### 1) Synthesis of compound (2-11)

A compound (2-11) was synthesized according to the following scheme.

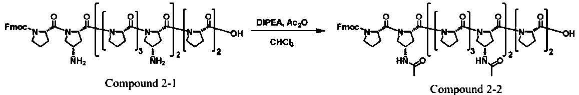

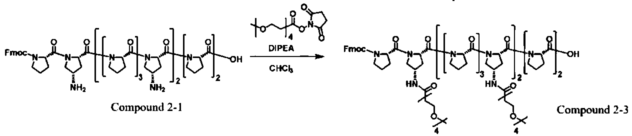

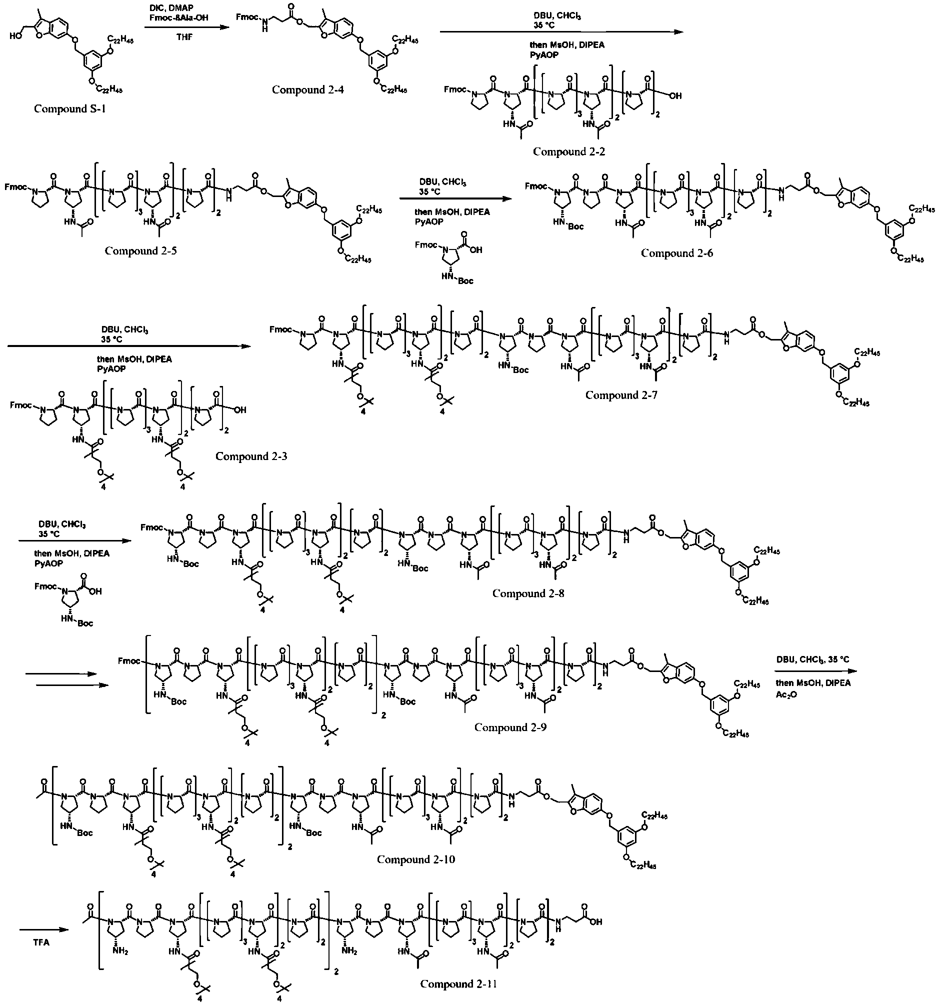

### (i) Synthesis of compound (2-1)

Solid phase peptide synthesis was carried out using H-Pro-Trt(2-Cl)-Resin (manufactured by Watanabe Chemical Industries, Ltd., 0.93 mmol/g, 53.8 mg) as a starting raw material. N-(9-fluorenylmethoxycarbonyl)-L-proline (Fmoc-Pro-OH) and (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH(2S,4S)) were subjected to elongation, and the elongation using N-(9-fluorenylmethoxycarbonyl)-L-proline (Fmoc-Pro-OH) was repeated for 3 cycles. (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH (2S,4S)) was subjected to elongation, and the elongation using N-(9-fluorenylmethoxycarbonyl)-L-proline (Fmoc-Pro-OH) was repeated for 3 cycles. (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH(2S,4S)) and N-(9-fluorenylmethoxycarbonyl)-L-proline (Fmoc-Pro-OH) were subjected to elongation. After completion of the elongation, the resin was washed with dichloromethane, and then the solvent was distilled off under reduced pressure. 2.0 mL of a mixed solution of TFA:triisopropylsilane:water = 95:2.5:2.5 was added thereto, and the peptide was cut out and deprotected. After 2 hours, the resin was filtered out, and methyl-t-butyl ether (12 mL) was added to the filtrate to generate a solid. The solid was precipitated by centrifugation, and then the supernatant was removed. The solid was washed with methyl-t-butyl ether, and then the solvent was distilled off under reduced pressure to obtain 51.2 mg of a white solid compound (2-1).

### (ii) Synthesis of compound (2-2)

352 mg of the compound (2-1), 3.5 mL of chloroform (CHCl₃), 423 µL of N-diisopropylethylamine (DIPEA), and 115 µL of acetic anhydride (Ac₂O) were placed in an eggplant flask having a capacity of 50 mL, and stirring was carried out at room temperature for 1 hour. Thereafter, the reaction solution was concentrated and purified by reverse phase column chromatography, and freeze drying was followed to obtain 314 mg of a compound (2-2).

### (iii) Synthesis of compound (2-3)

372 mg of the compound (2-1), 5.5 mL of chloroform (CHCl₃), 403 µL of N-diisopropylethylamine (DIPEA), and 346 mg of methyl-PEG₄-NHS ester were placed in an eggplant flask having a capacity of 50 mL, and stirring was carried out at room temperature for 1 hour. The reaction solution was concentrated and purified by reverse phase column chromatography, and freeze drying was followed to obtain 499 mg of a compound (2-3).

### (iv) Synthesis of compound (2-4)

500 mg of the compound (4-1) described in WO2020/175473A as the compound (S-1), 3 mL of tetrahydrofuran (THF), 254 mg of N-[(9H-fluoren-9-ylmethoxy)carbonyl]-β-alanine (Fmoc-βAla-OH), 128 µL of diisopropylcarbodiimide, and 13.3 mg of 4-dimethylaminopyridine were placed in an eggplant flask having a capacity of 50 mL, and stirring was carried out at room temperature for 2 hours. Acetonitrile (50 mL) was added thereto to filter the precipitated solid, which was subsequently subjected to drying under reduced pressure to obtain 593 mg of a compound (2-4).

### (v) Synthesis of compound (2-5)

108 mg of the compound (2-4), 2.2 mL of chloroform (CHCl₃), and 26.8 µL of 1,8-diazabicyclo[5.4.0]-7-undecene (DBU) were placed in an eggplant flask having a capacity of 50 mL, and stirring was carried out at 35°C for 1 hour. 11.7 µL of methanesulfonic acid (MsOH), 93.3 µL of N,N-diisopropylethylamine (DIPEA), 169 mg of the compound (2-2), and 141 mg of (7-azabenzotriazole-1-yloxy)tripyrrolidinophosphonium hexafluorophosphate (PyAOP) were placed therein, and stirring was carried out at 35°C for 3 hours. Acetonitrile (10 mL) was added thereto to filter the precipitated solid, which was subsequently subjected to drying under reduced pressure to obtain 219 mg of a compound (2-5).

### (vi) Synthesis of compound (2-6)

233 mg of the above-described compound (2-6) was synthesized from 219 mg of the compound (2-5) in the same manner, except that in the synthesis of the compound (2-5) described above, (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH(2S,4S)) was used instead of the compound (2-2) and the compound (2-5) was used instead of the compound (2-4).

### (vii) Synthesis of compound (2-7)

69.5 mg of the above-described compound (2-7) was synthesized from 38.0 mg of the compound (2-6) in the same manner, except that in the synthesis of the compound (2-5) described above, the compound (2-3) was used instead of the compound (2-2) and the compound (2-6) was used instead of the compound (2-4).

### (viii) Synthesis of compound (2-8)

48.9 mg of the above-described compound (2-8) was synthesized from 69.5 mg of the compound (2-7) in the same manner, except that in the synthesis of the compound (2-5) described above, (2S,4S)-(tert-butoxycarbonyl)-4-amino-1-((9H-fluoren-9-ylmethoxy)carbonyl)-pyrrolidine-2-c arboxylic acid (Fmoc-L-Pro(4-NHBoc)-OH(2S,4S)) was used instead of the compound (2-2) and the compound (2-7) was used instead of the compound (2-4).

### (ix) Synthesis of compound (2-9)

The same operation as the above-described synthesis of the compounds (2-7) and (2-8) was repeated once to synthesize 60.5 mg of a compound (2-9) from 48.9 mg of the compound (2-8).

### (x) Synthesis of compound (2-10)

40.1 mg of the above-described compound (2-10) was synthesized from 60.5 mg of the compound (2-9) in the same manner, except that in the synthesis of the compound (1-7) described above, the compound (2-9) was used instead of the compound (1-6).

### (xi) Synthesis of compound (2-11)

40.1 mg of the compound (2-10) and 1.0 mL of a mixed solution of TFA:triisopropylsilane:water = 95:2.5:2.5 were added to an eggplant flask having a capacity of 10 mL, and stirring was carried out at room temperature for 1 hour. 10 mL of methanol (MeOH) was added to the reaction solution to generate a solid to be removed, and then the solid was removed by filtration. The filtrate was concentrated and purified by preparative HPLC, and freeze drying was followed to obtain 16.8 mg of a compound (2-11).

### 2) Synthesis of compound (2)

0.75 mg of the above-described compound (2) was synthesized from 2.0 mg of the compound (2-11) in the same manner, except that in the synthesis of the compound (1) described above, the compound (2-11) was used instead of the compound (1-8). The results of the MS measurement of the compound (2) were as follows.
MS (ESI m/z): (M+H⁺)⁺ = 9,633, (M-H⁺)⁻ = 9,631

### <Synthesis of comparative compound (1)>

### 1) Synthesis of compound (c1-7)

A compound (c1-7) was synthesized according to the following scheme.

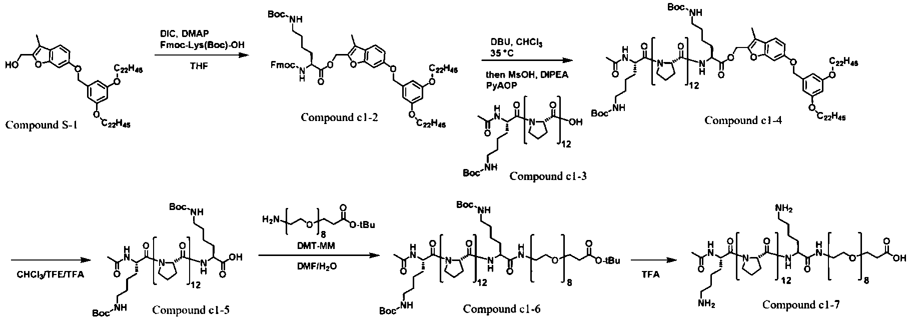

### (i) Synthesis of compound (c1-2)

300 mg of the compound (4-1) described in WO2020/175473A as the compound (S-1), 3 mL of tetrahydrofuran (THF), 229.8 mg of Nε-(tert-butoxycarbonyl)-Nα-[(9H-fluoren-9-ylmethoxy)carbonyl]-L-lysine (Fmoc-Lys(Boc)-OH), 76.8 µL of diisopropylcarbodiimide, and 8.0 mg of 4-dimethylaminopyridine were placed in an eggplant flask having a capacity of 50 mL, and stirring was carried out at room temperature for 2 hours. Acetonitrile (30 mL) was added thereto to filter the precipitated solid, which was subsequently subjected to drying under reduced pressure to obtain 350 mg of a compound (cl-2).

### (ii) Synthesis of compound (c1-3)

Solid phase peptide synthesis was carried out using H-Pro-Trt(2-Cl)-Resin (manufactured by Watanabe Chemical Industries, Ltd., 0.94 mmol/g, 53.2 mg) as a starting raw material. The elongation that was carried out by using N-(9-fluorenylmethoxycarbonyl)-L-proline (Fmoc-Pro-OH) was repeated for 11 cycles to elongate Nε-(tert-butoxycarbonyl)-Nα-[(9H-fluoren-9-ylmethoxy)carbonyl]-L-lysine (Fmoc-Lys(Boc)-OH). Subsequently, an NMP solution of piperidine (20% v/v) was added thereto to carry out a reaction for 20 minutes, thereby carrying out the deprotection of the Fmoc group, and then, an NMP solution of acetic anhydride (20% v/v) was added thereto to carry out a reaction for 10 minutes, thereby subjecting the N-terminal amino group to acetylation. After completion of the elongation, the resin was washed with dichloromethane, and then the solvent was distilled off under reduced pressure. 2.0 mL of a mixed solution of hexafluoro-2-propanol (HFIP):dichloromethane (CH₂Cl₂) = 1:4 was added thereto, and the peptide was cut out. After 30 minutes, the resin was filtered out to concentrate the filtrate, which was subsequently purified by reverse phase column chromatography to obtain 59.3 mg of a white solid compound (c1-3).

### (iii) Synthesis of compound (c1-4)

20 mg of the compound (c1-2), 400 µL of chloroform (CHCl₃), and 4.4 µL of 1,8-diazabicyclo[5.4.0]-7-undecene (DBU) were placed in an eggplant flask having a capacity of 10 mL, and stirring was carried out at 35°C for 1 hour. 1.9 µL of methanesulfonic acid (MsOH), 15.3 µL of N,N-diisopropylethylamine (DIPEA), 25.5 mg of the compound (c1-3), and 23.1 mg of (7-azabenzotriazole-1-yloxy)tripyrrolidinophosphonium hexafluorophosphate (PyAOP) were placed therein, and stirring was carried out at 35°C for 1 hour. Acetonitrile (4 mL) was added thereto to filter the precipitated solid, which was subsequently subjected to drying under reduced pressure to obtain 34.0 mg of a compound (c1-4).

### (iv) Synthesis of compound (c1-5)

30.0 mg of the compound (c1-4), 600 µL of chloroform (CHCl₃), 60 µL of 2,2,2-trifluoroethanol (TFE), and 6 µL of trifluoroacetic acid (TFA) were placed in an eggplant flask having a capacity of 10 mL, and stirring was carried out at room temperature for 4 hours. Thereafter, the reaction solution was filtered, and 6 mL of methanol (MeOH) was added to the filtrate to generate a precipitate, which was subsequently centrifuged. The recovered compound was purified by preparative HPLC and subjected to freeze drying to obtain 16.5 mg of a compound (c1-5).

### (v) Synthesis of compound (c1-6)

10 mg of the compound (c1-5), 200 µL of water, 200 µL of N,N-dimethylformamide (DMF),3.9 mg of tert-butyl amino-PEG8-ate, and 6.7 mg of 4-(4,6-dimethoxy-1,3,5-triazin-2-yl)-4-methylmorpholinium chloride (DMT-MM) were placed in an eggplant flask having a capacity of 10 mL, and stirring was carried out at room temperature for 2 hours. Thereafter, the reaction solution was concentrated and purified by preparative HPLC, and freeze drying was followed to obtain 8.9 mg of a compound (c1-6).

### (vi) Synthesis of compound (cl-7)

7.0 mg of the compound (c1-6) and 200 µL of trifluoroacetic acid (TFA) were placed in an eggplant flask having a capacity of 10 mL, and stirring was carried out at room temperature for 1 hour. Thereafter, the reaction solution was concentrated and purified by preparative HPLC, and freeze drying was followed to obtain 3.5 mg of a compound (c1-7).

### 2) Synthesis of comparative compound (1)

0.96 mg of the above-described comparative compound (1) was synthesized from 2.0 mg of the compound (c1-7) in the same manner, except that in the synthesis of the compound (1) described above, the compound (c1-7) was used instead of the compound (1-1). The results of the MS measurement of the comparative compound (1) were as follows.
MS (ESI m/z): (M+H⁺)⁺ = 4,636, (M-H⁺)⁻ = 4,634

### <Example 1>

A solution of each of the coloring agents produced above in PBS(-) (product name, manufactured by FUJIFILM Wako Pure Chemical Corporation) as a solvent was prepared such that the absorbance at a wavelength showing a maximum molar absorption coefficient (εₘₐₓ) in the vicinity of 785 nm, which was measured using an ultraviolet-visible spectrophotometer (product name: UV-2550, manufactured by Shimadzu Corporation), was in a range of 0.1 to 1.5.

Using an ultraviolet-visible spectrophotometer (product name: UV-2550, manufactured by Shimadzu Corporation), the absorbance of the prepared solution of each of the coloring agents in a wavelength range of 250 to 900 nm was measured. A normalized absorbance at 716 nm was calculated in a case where the absorbance at the wavelength exhibiting εₘₐₓ in the vicinity of 785 nm was normalized to 1. The normalized absorbance of the comparative compound (3) at 716 nm was set as a reference value (1.0), and a ratio of the normalized absorbance of the coloring agent at 716 nm to the reference value (normalized absorbance of coloring agent at 716 nm/normalized absorbance of comparative compound (3) at 716 nm) was calculated and evaluated based on the following evaluation standard. Table 1 collectively shows the results. It is noted that in the table, "normalized absorbance at 716 nm" is described as "716 nm normalized absorbance".

The comparative compound (3) which is a dye monomer has a wavelength at which εₘₐₓ is exhibited in the vicinity of 785 nm, and has a weak absorption due to the association of the coloring agent as a shoulder peak in the vicinity of 716 nm. As the association of the coloring agent proceeds in a dye multimer having a plurality of structures of the comparative compound (3), and the like, the peak intensity in which εₘₐₓ is exhibited in the vicinity of 785 nm decreases, while the peak intensity in the vicinity of 716 nm increases. Therefore, the degree of self-association can be evaluated by comparing the ratio of the normalized absorbance at 716 nm in a case where the absorbance at the wavelength exhibiting εₘₐₓ in the vicinity of 785 nm is normalized to 1, between the multimeric coloring agents. The smaller the ratio of the normalized absorbance to the reference value, the less self-association occurs.

### - Evaluation standard for normalized absorbance at 716 nm -

A: The ratio of the normalized absorbance to the reference value is 1.00 times or more and less than 1.03 times.
B: The ratio of the standardized absorbance to the reference value is 1.03 times or more and less than 1.06 times.
C: The ratio of the normalized absorbance to the reference value is 1.06 times or more and less than 1.09 times.
D: The ratio of the normalized absorbance to the reference value is 1.09 times or more and less than 1.12 times.
E: The ratio of the standardized absorbance to the reference value is 1.12 times or more and less than 1.15 times.
F: The ratio of the standardized absorbance to the reference value is 1.15 times or more and less than 1.18 times.

**[Table 1]**

| No. | Coloring agent | Molecular Flexibility | cLogP | 716 nm normalized absorbance |
|---|---|---|---|---|
| 101 | Compound (1) | 0.500 | -3.2 | A |
| 102 | Compound (2) | 0.500 | -4.1 | A |
| c11 | Comparative compound (1) | 0.529 | 0.7 | C |
| c12 | Comparative compound (2) | 0.330 | 6.3 | F |
| cl3 | Comparative compound (3) | - | - | Reference (1.0) |

From the results in Table 1 above, the following facts can be seen.

The comparative compound (1) is not the compound defined in the present invention in that the Molecular Flexibility of the linking group that links two phosphor moieties consisting of a cyanine dye (comparative compound (3)) is 0.529, and the two phosphor moieties are not linked by the linking group defined in the present invention. As shown in No. c11, the normalized absorbance of the solution of the comparative compound (1) at 716 nm was 1.06 times or more and less than 1.09 times the normalized absorbance of the solution of the comparative compound (3) as a dye monomer at 716 nm, and self-association occurred as a result of forming the dye multimer. In addition, the comparative compound (2) is not the compound defined in the present invention in that the cLogP of the linking group that links two phosphor moieties consisting of a cyanine dye (a cyanine dye having the same conjugated structure as that of the comparative compound (3)) is 6.3, and the two phosphor moieties is not linked by the linking group defined in the present invention. As shown in No. c12, the normalized absorbance of the solution of the comparative compound (2) at 716 nm was 1.15 times or more and less than 1.18 times the normalized absorbance of the solution of the comparative compound (3) as a dye monomer at 716 nm, and self-association occurred as a result of forming the dye multimer.

On the other hand, in the compounds (1) and (2), each linking group that links the three phosphor moieties consisting of the cyanine dye (comparative compound (3)) is a linking group having a Molecular Flexibility of 0.510 or less and a cLogP of 6.0 or less, and is a compound defined in the present invention. As shown in Nos. 101 and 102, the normalized absorbance of the solutions of the compounds (1) and (2) at 716 nm was 1.00 times or more and less than 1.03 times the normalized absorbance of the solution of the comparative compound (3) as a dye monomer at 716 nm.

In general, the degree of self-association of a dye multimer increases as the number of dye structures (phosphor moieties) in a compound increases. However, it is found that, in the compounds (1) and (2) according to the embodiment of the present invention, which have three phosphor moieties in the compound, the ratio of the normalized absorbance at 716 nm is smaller than that of the comparative compound (1) having two phosphor moieties in the compound, and an excellent effect of suppressing the self-association is obtained, and the decrease in absorbance (association quenching) at a wavelength at which εₘₐₓ is exhibited near 785 nm is effectively suppressed. It is noted that the compounds (1) and (2) according to the embodiment of the present invention and the comparative compound (1) have cLogP in a range of -4.13 to 0.70, and in the comparison of the Molecular Flexibility, the compounds are compared in a state of being sufficiently close to each other in hydrophilicity to the extent that the difference in hydrophilicity does not affect the comparison.

As described above, the compound according to the embodiment of the present invention is a compound having two or more phosphor moieties, in which phosphor moieties adjacent to each other are linked by a linking group having a Molecular Flexibility of 0.510 or less and a cLogP of 6.0 or less. Therefore, self-association and association quenching of the compound according to the embodiment of the present invention in a solution state can be effectively suppressed.

Although the present invention has been described with reference to the embodiments, it is the intention of the inventors of the present invention that the present invention should not be limited by any of the details of the description unless otherwise specified and rather be construed broadly within the spirit and scope of the invention appended in WHAT IS CLAIMED IS.

The present application claims the priority of JP2023-028797A filed in Japan on February 27, 2023, the contents of which are incorporated herein by reference, as a part of the description of the present specification.

## Claims

1. A compound comprising:
two or more phosphor moieties,
wherein each of the phosphor moieties adjacent to each other is linked by a linking group having a Molecular Flexibility of 0.510 or less and a cLogP of 6.0 or less.

2. The compound according to claim 1,
wherein the linking group is a group containing at least one of an alkynyl group, an aliphatic hydrocarbon ring, a heterocyclic ring, or a carbonyl group.

3. The compound according to claim 1,
wherein the linking group is a group containing a structure represented by General Formula (I), 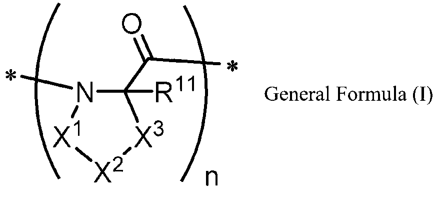 in the formula, X¹ to X³ represent -O-, -S-, >NR¹, or >CR²R³,
R¹ to R³ and R¹¹ represent a hydrogen atom, an alkyl group, an alkenyl group, an alkynyl group, an aryl group, a heteroaryl group, an acyl group, -NR⁸R⁹, -OR¹⁰, an anionic group, a betaine residue, a polyol residue, a sugar residue, a polyamino acid residue, -(O-L)_{g}R^{E}, or -(L-O)_{g}R^{E}
R⁸ to R¹⁰ represent a hydrogen atom, an alkyl group, an alkenyl group, an alkynyl group, an acyl group, an aryl group, a heteroaryl group, an anionic group, a betaine residue, a polyol residue, a sugar residue, a polyamino acid residue, -(O-L)_{g}R^{E}, or -(L-O)_{g}R^{E},
L represents an alkylene group, R^{E} has the same meaning as R⁸ to R¹⁰ described above and g represents 2 to 50, provided that R^{E} represents neither -(O-L)_{g}R^{E} nor -(L-O)_{g}R^{E},
n represents an integer of 2 or more, and
* represents a bonding site.

4. The compound according to claim 3,
wherein n represents an integer of 3 or more.

5. The compound according to claim 3,
wherein the compound is represented by General Formula (II), 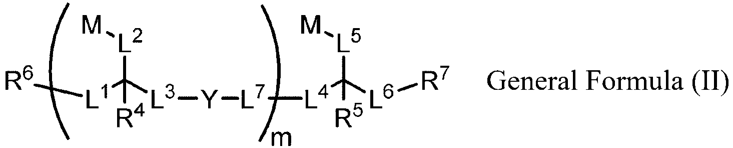
in the formula, R⁴ and R⁵ represent a hydrogen atom, an alkyl group, an alkenyl group, an alkynyl group, an acyl group, an amino group, a hydroxy group, an alkoxy group, a sulfanyl group, an aryl group, or a heteroaryl group,
R⁶ and R⁷ represent a hydrogen atom, a hydroxy group, a sulfanyl group, an alkyl group, an alkenyl group, an alkynyl group, an aryl group, an alkoxy group, an amino group, an acyl group, a heteroaryl group, or Q,
Q represents an anionic group, a cationic group, a betaine residue, a polyol residue, a sugar residue, a polyamino acid residue, -(O-L)_{g}R^{E}, -(L-O)_{g}R^{E}, a substituent allowing binding to a biological substance, or a substituent allowing binding to a solid support,
L¹ to L⁷ represent a single bond or a divalent linking group,
M represents a phosphor moiety, a physiologically active substance moiety, a prodrug moiety, or a radioactive isotope-containing moiety,
Y represents the structure represented by General Formula (I),
m is an integer of 1 or more, and
L, R^{E}, and g have the same meanings as L, R^{E}, and g, all of which are described above,
provided that at least two of M's represent the phosphor moieties described above.

6. The compound according to claim 5,
wherein the compound is represented by General Formula (III), 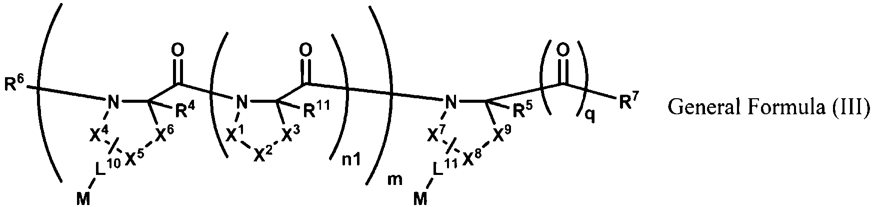 in the formula, X⁴ to X⁹ represent -O-, -S-, >NR¹⁰¹, or >CR¹⁰²R¹⁰³,
provided that at least one of X⁴, X⁵, or X⁶ represents >N-L¹⁰-M or >C(R¹⁰²)-L¹⁰-M, and at least one of X⁷, X⁸, or X⁹ is >N-L¹¹-M or >C(R¹⁰²)-L¹¹-M,
R¹⁰¹ to R¹⁰³ which are neither -L¹⁰-M nor -L¹¹-M represent a hydrogen atom, an alkyl group, an alkenyl group, an alkynyl group, an aryl group, a heteroaryl group, an acyl group, -NR⁸R⁹, -OR¹⁰, an anionic group, a betaine residue, a polyol residue, a sugar residue, a polyamino acid residue, -(O-L)_{g}R^{E}, or -(L-O)_{g}R^{E},
L¹⁰ and L¹¹ represent a single bond or a divalent linking group,
n1 represents an integer of 2 or more, and q represents an integer of 0 or 1, and
R⁴ to R¹¹, X¹ to X³, M, L, R^{E}, g, and m have the same meanings as R⁴ to R¹¹, X¹ to X³, M, L, R^{E}, g, and m, all of which are described above.

7. The compound according to claim 6,
wherein the compound is represented by General Formula (IV), 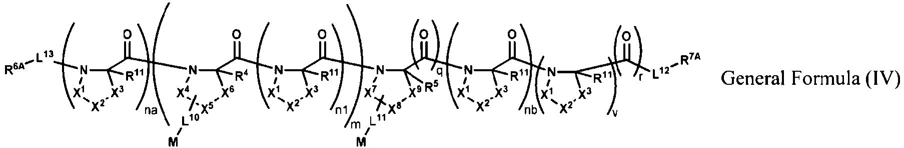
in the formula, R^{6A} and R^{7A} represent a hydrogen atom, a hydroxy group, a sulfanyl group, an alkyl group, an alkenyl group, an alkynyl group, an aryl group, an alkoxy group, a heteroaryl group, an amino group, an acyl group, or Q, provided that at least one of R^{6A} or R^{7A} represents a carboxy group, a substituent allowing binding to a biological substance, or a substituent allowing binding to a solid support,
L¹² and L¹³ represent a single bond or a linking group,
na and nb represent an integer of 0 or more, and r and v represent an integer of 0 or 1, and
R⁴, R⁵, R¹¹, L¹⁰, L¹¹, X¹ to X⁹, M, Q, q, n1, and m have the same meanings as R⁴, R⁵, R¹¹, L¹⁰, L¹¹, X¹ to X⁹, M, Q, q, n1, and m, all of which are described above.

8. The compound according to claim 7,
wherein (A) na represents an integer of 1 or more, and R^{6A} represents the carboxy group, the substituent allowing binding to a biological substance, or the substituent allowing binding to a solid support, and/or (B) a sum of nb and v, both of which are described above, represents an integer of 1 or more, and R^{7A} represents the carboxy group, the substituent allowing binding to a biological substance, or the substituent allowing binding to a solid support,
provided that the number of shortest linking atoms of L¹³ is 7 or less in a case of the (A), and the number of shortest linking atoms of L¹² is 7 or less in a case of the (B).

9. The compound according to claim 3,
wherein the structure represented by General Formula (I) includes a structure in which X¹ to X³ are >CR²R³.

10. The compound according to claim 9,
wherein at least one R² in the structure in which X¹ to X³ are >CR²R³ included in the structure represented by General Formula (I) is -NR⁸R⁹, -OR¹⁰, an anionic group, a betaine residue, a polyol residue, a sugar residue, a polyamino acid residue, -(O-L)_{g}R^{E}, or -(L-O)_{g}R^{E}.

11. The compound according to claim 1,
wherein at least two of the phosphor moieties are phosphor moieties having light absorption characteristics that are equivalent to each other.

12. The compound according to claim 1,
wherein at least two of the phosphor moieties are phosphor moieties having light absorption characteristics that are non-equivalent to each other.

13. The compound according to claim 6,
wherein m in General Formula (III) is an integer of 3 or less.

14. A labeled biological substance that is obtained by bonding the compound according to any one of claims 1 to 13 to a biological substance.

15. The labeled biological substance according to claim 14,
wherein the biological substance is any one of a protein, an amino acid, a nucleic acid, a nucleotide, a sugar chain, or a phospholipid.
